# FASCICULE DE BREVET EUROPEEN

(11) **EP 1 206 452 B1**
(45) Date de publication et mention de la délivrance du brevet: **03.11.2004**
(21) Numéro de dépôt: 00953267.2
(22) Date de dépôt: 20.07.2000
(51) Int. Cl.: C07D 209/72

(54) **PROCEDE DE PREPARATION DE COMPOSES BENZOPERHYDROISOINDOLE**
VERFAHREN ZUR HERSTELLUNG VON BENZOPERHYDROISOINDOLEN
NOVEL METHOD FOR PREPARING BENZOPERHYDROISOINDOLE COMPOUNDS

(30) Priorité: 22.07.1999 FR 9909508
(43) Date de publication de la demande: 22.05.2002
(73) Titulaire: Aventis Pharma S.A., 92160 Antony (FR)
(72) Inventeur: MAILLIET, Patrick, F-94120 Fontenay Sous Bois (FR); SALAGNAD, Christophe, F-94490 Ormesson sur Marne (FR)
(74) Mandataire: Le Pennec, Magali
(86) Numéro de dépôt international: PCT/FR2000/002082
(87) Numéro de publication internationale: WO 2001/007408

(56) Documents cités:
- EP-A- 0 407 909
- WO-A-98/29390
- WO-A-99/33834

## Description

La présente invention concerne un nouveau procédé de préparation de composés de type benzoperhydroisindole de formule générale (I) :

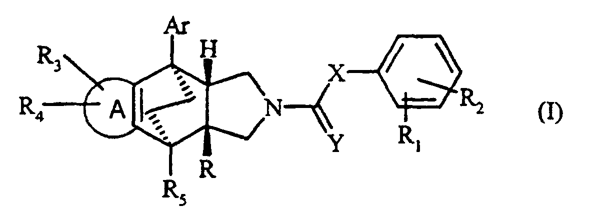

Les composés de formule générale et leur activité en tant qu'inhibiteurs de farnésyle transférase sont décrits dans les demandes WO 98/29390, FR 2772764.
La protéine famésyle transférase est une enzyme qui catalyse le transfert du groupement famésyle du pyrophosphate de famésyle (FPP) au résidu cystéine terminal de la séquence tétrapeptidique CAAX d'un certain nombre de protéines et en particulier de la protéine p21Ras, exprimant l'oncogène *ras*.
L'oncogène *ras* (H-, N- ou K-*ras*) est connu pour jouer un rôle clé dans les voies de signalisation cellulaire et les processus de division cellulaire. La mutation de l'oncogène *ras* ou sa surexpression est souvent associée au cancer humain: la protéine p21Ras mutée se retrouve dans de nombreux cancers humains et notamment dans plus de 50% des cancers du colon et 90% des cancers du pancréas (Kohl et al., Science, 260, 1834-1837, 1993).
L'inhibition de la farnésyle transférase et par conséquent de la famésylation de la protéine p21Ras bloque la capacité de la protéine p21Ras mutée à induire une prolifération cellulaire et à transformer les cellules normales en cellules cancéreuses.
D'autre part, il a été démontré que les inhibiteurs de farnésyle transférase sont également actifs sur des lignées cellulaires tumorales n'exprimant pas de *ras* muté ou surexprimé, mais présentant la mutation d'un oncogène ou la surexpression d'une oncoprotéine dont la voie de signalisation utilise la famésylation d'une protéine, telle qu'un *ras* normal (Nagasu et al., Cancer Research 55, 5310-5314, 1995 ; Sepp-Lorenzino et al., Cancer Research 55, 5302-5309, 1995) ou telle que *rhoB* (Wei et al, Molecular and Cellular Biology, 19, 1831-1840, 1999)..
Les inhibiteurs de la Farnésyle transférase sont des inhibiteurs de la prolifération cellulaire et par conséquent des agents anti-tumoraux et anti-leucémiques.

Dans les demandes WO 98/29390, FR 2772764 est également décrit le procédé de préparation des composés de formule générale (I) : Selon ce procéde, les composés finaux sont obtenus sous forme de mélange racémique ce qui nécessite une séparation ultérieure des énantiomères constituant ce mélange. Il s'ensuit donc une étape supplémentaire délicate, ce qui a pour conséquence de diminuer le rendement global par rapport au produit de départ et à chacun des réactifs.

Selon la présente invention, il a été découvert un procédé permettant d'obtenir directement les produits de formule générale (I) sous forme d'isomères optiques, sans mettre en oeuvre de séparation. Ce procédé comprend une étape enzymatique permettant la résolution énantiomèrique des composés de formule générale (I).

La présente invention concerne donc un procédé de préparation des isomères optiques des composés décrits dans les demandes WO 98/29390, FR 2772764 incorporés ici par référence.

Les composés susceptibles d'être préparés par le procédé selon la présente invention sont les composés de formule générale (I) : 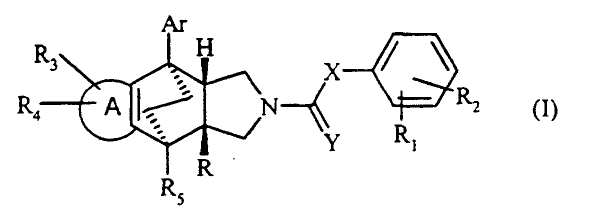 dans laquelle:
- A représente :
   - soit un radical phényle fusionné avec le noyau isoindole
      et dans ce cas :
      - R₃ et R₄, identiques ou différents, représentent un atome d'hydrogène, d'halogène ou un radical alcoyle, hydroxy, alcoyloxy, alcoylcarbonyloxy, mercapto, alcoylthio, alcoylsulfonyle ou alcoylsulfinyle, amino, alcoylamino ou dialcoylamino, alcoyloxycarbonylamino, carboxy, alcoyloxycarbonyle, carbamoyle alcoylcarbamoyle ou dialcoylcarbamoyle, formyl, alcoylcarbonyle, cyano ou trifluorométhyle,
      préférentiellement, ou bien R₃ et R₄ représentent chacun un atome d'hydrogène, ou bien l'un des symboles R₃ ou R₄ représente un atome d'hydrogène et l'autre des symboles R₃ ou R₄ représente un radical méthoxy, et plus avantageusement en position 5 du noyau benzoperhydroisoindole;
      très avantageusement, R₃ et R₄ représentent chacun un atome d'hydrogène; avec pour l'ensemble des radicaux possédant un groupement alcoyle, proposés dans la définition de R₃ et R₄, alcoyle contenant 1 à 4 atomes de carbone.
   - soit A représente un système monocyclique ou, bi-, ou tri-cyclique condensé, pour lequel chaque cycle, saturé ou non, contient de 4 à 7 chaînons, et pour lequel au moins un des cycles contient de 1 à 4 hétéroatomes, identiques ou différents, choisis indépendamment parmi les atomes d'azote, d'oxygène et de soufre ; ce système hétérocyclique, éventuellement substitué peut être condensé au noyau isoindole par 2 quelconques de ses atomes adjacents; A représente préférentiellement un système mono- ou bicyclique aromatique de 5 à 9 chainons contenant un atome d'azote ou de soufre; A pouvant être également choisi de façon non limitative parmi les radicaux suivants : thiényle, furyle, pyrrolyle, pyrazolyle, imidazolyle, pyridyle, pyrazinyle, pyrimidinyle, pyridazinyle, benzofuranyle, benzothiényle, chromènyle, indolyle, ou quinolyle, ainsi que leurs isomères *iso*;
   préférentiellement, A représente un radical thiényle, indolyle;
   avantageusement selon l'invention, A représente thiényle;
   et dans ce cas R₃ et R₄ représentent un atome d'hydrogène.
- Ar représente
   - un radical phényle éventuellement substitué par un ou plusieurs atomes ou radicaux, identiques ou différents, choisis parmi les atomes d'halogène et les radicaux alcoyles contenant 1 à 4 atomes de carbone, tel que méthyle, alcényles contenant 2 à 4 atomes de carbone, hydroxy, mercapto, alcoylthio, alcoylsulfonyle ou alcoylsulfinyle, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle, alcoxy contenant 1 à 4 atomes de carbone, tel que méthoxy, dont la portion alcoyle est éventuellement perhalogénée, tel que trifluorométhoxy, ou
   - un radical phényle condensé à un hétérocycle de 4 à 7 chaînons contenant un ou plusieurs hétéroatomes choisis parmi les atomes d'oxygène, d'azote et de soufre, le système bicyclique ainsi formé pouvant être notamment choisi parmi les radicaux 2,3-dihydro-1,4-benzodioxin-6-yle ou 2,3-dihydrobenzofuran-5-yl ou le 2,3-dihydrobenzopyran-6-yle ou benzothiényle ou
   - un radical polycyclique aromatique ou non aromatique, tel que le 1- ou 2-naphtyle ou le 5-indanyle, ou le 1,2,3,4-tétrahydronapht-6-yle
   - un radical hétérocyclique aromatique ou non aromatique de 5 à 12 chaînons incorporant un ou plusieurs hétéroatomes choisis parmi les atomes d'oxygène, d'azote et de soufre, lié au cycle condensé par une liaison carbone-carbone, ledit radical étant substitué le cas échéant par un ou plusieurs atomes ou radicaux, identiques ou différents, choisis parmi les atomes d'halogène et les radicaux alcoyles, alcényles contenant 2 à 4 atomes de carbone, hydroxy, alcoxy contenant 1 à 4 atomes de carbone, mercapto, alcoylthio, alcoylsulfonyle ou alcoylsulfinyl, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle,
   préférentiellement, Ar représente un radical 2,3-dihydro-1,4-benzodioxin-6-yle ou 2,3-dihydrobenzofuran-5-yl, ou benzothiényle ou un radical phényle éventuellement substitué en position 2 et/ou 4, de préférence par un atome d'halogène ou un radical méthyle, trifluorométhyle ou méthoxy; en particulier, le radical 2,3-dihydro-1,4-benzodioxin-6-yle, benzothiényle; très avantageusement, Ar représente un radical phényle éventuellement substitué en position 4 par un radical méthyle, ou en position 2,4 par un atome d'halogène.
   avec pour l'ensemble de ces radicaux, alcoyl contenant 1 à 4 atomes de carbone,
- R représente
   un radical de formule générale

   - (CH₂)ₘ-X₁-(CH₂)ₙ-Z

   dans laquelle
   - X₁ représente une simple liaison ou un atome d'oxygène ou de soufre,
   - m représente un nombre entier égal à 0 ou 1,
   - n représente un nombre entier égal à 0, 1 ou 2,
   - un ou plusieurs radicaux méthylènes peuvent être substitués par un radical carboxy, alcoxycarbonyle, carbamoyle, alcoylcarbamoyle, dialcoylcarbamoyle, amino, alcoylamino, dialcoylamino avec pour l'ensemble de ces radicaux, alcoyl contenant 1 à 4 atomes de carbone,
   - Z représente
      - un radical carboxy,
      - un radical COOR₆, dans lequel R₆ représente un radical alcoyle droit ou ramifié contenant 1 à 3 atomes de carbone, tel que le méthyle,ou
      - un radical de formule CON(R₇)(R₈) dans lequel
         - R₇ représente un atome d'hydrogène ou un radical alcoyle droit ou ramifié contenant 1 à 6 atomes de carbone et
         - R₈ représente
            - un atome d'hydrogène,
            - un radical hydroxy,
            - un radical arylsulfonyle, tel que phénylsulfonyle, éventuellement substitué par un ou plusieurs atomes ou radicaux, identiques ou différents, choisis parmi les atomes d'halogène et les radicaux alcoyles, alcoyloxy, avec, pour ces radicaux alcoyl contenant 1 à 4 atomes de carbone,
            - un hétérocycle de 5 à 7 chaînons incorporant un ou plusieurs hétéroatomes choisis parmi les atomes d'azote, d'oxygène ou de soufre, ledit hétérocycle pouvant être lié par un hétéroatome,
            - un radical amino éventuellement substitué par un ou deux radicaux, identiques ou différents choisis parmi les radicaux
               - alcoyle contenant 1 à 4 atomes de carbone,
               - aryle, tel que phényle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
               - hétérocyclyle de 5 à 7 chaînons et contenant un ou plusieurs hétéroatomes choisis parmi les atomes d'azote, d'oxygène et de soufre,
               - arylcarbonyle, tel que benzoyle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
            - un radical alcoyloxy contenant 1 à 6 atomes de carbone en chaîne droite ou ramifiée éventuellement substitué par un radical phényle,
            - un radical alcoyle droit ou ramifié contenant 1 à 6 atomes de carbone, tel que méthyle, éventuellement substitué par un radical amino, alcoylamino, dialcoylamino, hydroxy, alcoxy contenant 1 à 4 atomes de carbone, mercapto, alcoylthio, alcoyloxycarbonyle, carboxy, cyano, un radical aromatique mono- ou polycyclique et ayant de 5 à 12 chaînons, incorporant ou non, un ou plusieurs hétéroatomes choisis parmi les atomes d'oxygène, d'azote et de soufre éventuellement substitué, ledit radical aromatique pouvant être notamment le radical 2-, ou 3-, ou 4- pyridyle, préférentiellement le 3-pyridyle ou le 4-pyridyle, ou le N-oxyde de pyridine, ou pouvant être également un radical phényle éventuellement substitué par un ou plusieurs atomes d'halogène ou par un ou plusieurs groupements hydroxy, amino, ou trifluorométhyle, ou par un ou plusieurs radicaux alcoyle ou alcényle, alcoxy, alcoylthio, alcoylamino, alcoylcarbonyle ou alcoxycarbonyle en C2 à C4, carbamoyle, alcoylcarbamoyle ou dialcoylcarbamoyle dont la partie alcoyle est en C1 à C8 , formyle ou encore un radical naphtyle-1 ou -2 ou,
      préférentiellement, R représente un radical carboxy, ou un radical -COOMe, ou encore un radical -CON(R₇)(R₈) pour lequel lorsque R₇ représente un atome d'hydrogène, R₈ représente un radical méthyle substitué par le radical 3-pyridyle ;
      très avantageusement, R représente un radical carboxy ;
      d'un intérêt tout particulier, R représente un radical -CON(R₇)(R₈) pour lequel lorsque R₇ représente un atome d'hydrogène, R₈ représente un radical méthyle substitué par le radical 3-pyridyle;
   - Z représente
      - un radical PO(OR₉)₂ dans lequel R₉ représente un atome d'hydrogène ou un radical alcoyle droit ou ramifié contenant 1 à 6 atomes de carbone, ou
      - un radical -NH-CO-T dans lequel T représente un atome d'hydrogène ou un radical alcoyle droit ou ramifié contenant 1 à 6 atomes de carbone éventuellement substitué par un radical amino, carboxy, alcoyloxycarbonyle, hydroxy, alcoyloxy, mercapto ou alcoylthio, ou bien
      - un radical 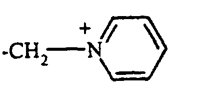 ayant pour contre-ion un anion, tel que le trifluorométhanesulfonate,
   avec pour l'ensemble des radicaux possédant un groupement alcoyle, proposés dans la définition de Z, alcoyl contenant 1 à 4 atomes de carbone.
- R₁ et R₂, identiques ou différents, représentent un atome d'hydrogène, un atome d'halogène ou un radical alcoyle, un radical alcoyloxy, tel que méthoxy, chacun éventuellement substitué par un radical dialcoylamino dont chaque partie alcoyle contient 1 à 4 atomes de carbone ou forme avec l'atome d'azote un hétérocycle saturé contenant 5 ou 6 chaînons, un radical alcoylthio, un radical alcoyloxycarbonyle, ou bien
   situés en ortho l'un par rapport à l'autre, R₁ et R₂ forment un hétérocycle saturé ou non saturé contenant 1 ou 2 hétéroatomes choisis parmi l'azote et l'oxygène, éventuellement substitué par un atome d'halogène ou par un radical alcoyle ou alcoyloxy,
   préférentiellement, l'un des symboles R₁ ou R₂ représente un atome d'hydrogène et l'autre des symboles représente un radical méthoxy, et plus avantageusement fixé en position *ortho* du cycle phénylique,
   avec pour l'ensemble des radicaux possédant un groupement alcoyle, proposés dans la définition de R₁ et R₂, alcoyle contenant 1 à 4 atomes de carbone.
- R₅ représente un atome d'hydrogène ou un radical alcoyle, un radical alcoylthio, avec pour la définition de R₅, alcoyle contenant 1 à 4 atomes de carbone;
   préférentiellement, R₅ représente un atome d'hydrogène ou un radical méthyle;
   très avantageusement, R₅ représente un atome d'hydrogène;
- X représente un atome d'oxygène ou de soufre ou un groupement -NH-, -CO-, méthylène, alcèn-1,1-diyle tel que vinyldiyle ou cycloalcan-1,1-diyle contenant 3 à 6 atomes de carbone,
   préférentiellement, X représente un groupement méthylène ou vinyldiyle,
   de façon particulièrement avantageuse, X représente le groupement vinyldiyle,
   et
- Y représente un atome d'oxygène ou de soufre, préférentiellement, Y représente un atome d'oxygène,
leurs isomères optiques ainsi que les sels du produit de formule générale (I).

Dans les définitions qui précédent et celles qui suivent,
- les radicaux ou portions "alcoyle contenant 1 à 8 atomes de carbone", ou "alcoyle en C1 à C8" définit les radicaux méthyle, éthyle, propyle, butyle, pentyle, hexyle, heptyle, octyle et les isomères *iso, sec ter* correspondants,
   les radicaux ou portions "alcoyle contenant 1 à 6 atomes de carbone", ou "alcoyle en C1 à C6" définit les radicaux méthyle, éthyle, propyle, butyle, pentyle, hexyle et les isomères *iso, sec, tert* correspondants,
   les radicaux ou portions "alcoyle contenant 1 à 4 atomes de carbone", ou "alcoyle en C1 à C4" définit les radicaux méthyle, éthyle, propyle, butyle, et les isomères *iso, sec, tert* correspondants
- "alcényles contenant 2 à 4 atomes de carbone" définit les radicaux vinyle, allyle, propène-2 yle, butène-1 ou -2 ou-3 yle, et les isomères iso correspondants
- "alcoxy contenant 1 à 4 atomes de carbone" définit les radicaux méthoxy, éthoxy, propoxy, butoxy, et les isomères *iso, sec, tert* correspondants
- "alcoxycarbonyle en C2 à C4" définit les radicaux métoxycarbonyle, éthoxycarbonyle, propoxycarbonyle, et les isomères *iso, sec, tert* correspondants,

Préférentiellement, les composés susceptibles d'être préparés par le procédé selon l'invention présentent une formule générale (I) pour laquelle :
A représente
   - soit un radical phényle fusionné au cycle isoindole et R₃ et R₄ représentent un atome d'hydrogène,
   - soit un radical thiényle et R₃ et R₄ représentent un atome d'hydrogène,
Ar représente un radical 2,3-dihydro-1,4-benzodioxin-6-yle ou 2,3-dihydrobenzofuran-5-yl, benzothièn-2-yle ou un radical phényle éventuellement substitué, en position 4 de préférence, par un radical méthyle, trifluorométhyle, méthoxy, ou par un atome de chlore ou de brome, ou en position 3-, 4- ou 2-, 4- par un atome de chlore ;
R représente un radical carboxy, ou un radical -COOMe, ou encore un radical - CON(R₇)(R₈) pour lequel lorsque R₇ représente un atome d'hydrogène, R₈ représente un radical méthyle substitué par le radical 3-pyridyle,
l'un des symboles R₁ ou R₂ représente un atome d'hydrogène et l'autre des symboles représente un radical méthoxy, et plus avantageusement fixé en position *ortho* du cycle phénylique,
R₅ représente un atome d'hydrogène ou un radical méthyle,
X représente un groupement méthylène ou vinyldiyle,
Y représente un atome d'oxygène;
sous forme de leurs isomères optiques ainsi que leurs sels.

Selon l'invention, les composés de formule générale (I) se présentent de préférence sous forme dextrogyre.

On peut citer selon l'invention, tout composé de formule générale (I) sélectionné individuellement parmi :
4,9-éthano-2-[2-(2-méthoxyphényl)propènoyl]-9-(4-méthylphényl)-2,3,3a,4,9,9a-hexahydro- 1H-benzo[f]isoindole-3a-carboxylate de méthyle-(3aRS,4SR,9SR,9aRS)
acide 4,9-éthano-2-(2-méthoxyphényl)propènoyl-9-(4-méthylphényl)-2,3,3a,4,9,9a-hexahydro-1H-benzo[f]isoindole-3a-carboxylique-(3aRS,4SR,9SR,9aRS)
acide 4,9-éthano-2-[(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS),
acide 4,9-éthano-9-(4-méthoxyphényl)-2-[(2-méthoxyphényl)acétyl]-2,3,3a,4,9,9a-hexahydro-1H-benzo[f]isoindole-3a-carboxylique (3aRS,4SR,9SR,9aRS),
acide 4,9-éthano-2-[(2-méthoxyphényl)acétyl]-9-(4-méthylsulfanylphényl)-2,3,3a,4, 9,9a-hexahydro-1H-benzo[f]isoindole-3a-carboxylique (3aRS,4SR,9SR,9aRS),
acide 4,9-éthano-9-(4-fluorophényl)-2-[(2-méthoxyphényl)acétyl]-2,3,3a,4,9,9a-hexahydro- 1H-benzo[f]isoindole-3a-carboxylique-(3aRS,4SR,9SR,9aRS),
acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS),
acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS),
9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS),
acide 9-(3,4-diméthylphényl)-4,9-éthano-2-[(2-méthoxyphényl)acétyl]-2,3,3a,4,9,9a-hexahydro-1H-benzo[f]isoindole-3a-carboxylique-(3aRS,4SR,9SR,9aRS),
3a-N-benzylcarbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),
3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),
4,9-éthano-2-[2-(2-méthoxyphényl)propènoyl]-9-(4-méthylphényl)-2,3,3a,4,9,9a-hexahydro-1H-benzo[f]isoindole-3a-hydroxamate de benzyle-(3aRS,4SR,9SR,9aRS),
acide 4,9-éthano-2-[2-(2-méthoxyphényl)propènoyl]-9-(4-méthylphényl)-,3,3a,4,9,9a-hexahydro-1H-benzo[f]isoindole-3a-hydroxamique-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR, 9SR,9aRS)
4,9-éthano-2-[2-(2-méthoxyphényl)propènoyl]-9-(4-méthylphényl)-2,3,3a,4,9,9a-hexahydro- 1H-benzo[f]isoindole-3a-N-(4-pyridylméthyl)-carboxamide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-hydroxamate de méthyle-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N',N'-diméthylcarbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-phénylcarbohydrazide-(3aRS,4SR, 9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N',N'-pentaméthylènecarbohydrazide-(3aRS,4SR,9SR,9aRS)
4,9-éthano-2-[2-(méthoxyphényl)propènoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N-(N-oxo-3pyridyl)méthyl-carboxamide-(3aRS,4SR,9SR,9aRS)
4,9-éthano-2-[2-(2-méthoxyphényl)propènoyl]-9-(4-méthylphényl)-2,3,3a,4,9,9a-hexahydro- 1H-benzo[f]isoindole-3a-N'-(4-méthoxyphényl)carbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro- 1H-benzo[f]isoindole-3a-N'-méthyl,N'-phénylcarbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-(2-méthylphényl)carbohydrazide-(3aRS,4SR,9SR,9aRS)
9-(2,3-dihydro-1,4-benzodioxin-6-yl)-4,9-éthano-2-[2-(2-méthoxyphényl)propènoyl]-2,3,3a,4,9,4a-hexahydro-1H-benzo[f]isoindole-3a-carboxylate de méthyle-(3aRS,4SR,9SR,9aRS)
acide 9-(2,3-dihydro-1,4-benzodioxin-6-yl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)
9-(2,3-dihydro-1,4-benzodioxin-6-yl)-4,9-éthano-2-[2-(2-méthoxy phényl)propènoyl]-2,3,3a,4,9,9a-hexahydro-1H-benzo[f] isoindole-3a-[N-(3-pyridylméthyl)carboxamide-(3aRS,4SR,9SR,9aRS)
9-(2,3-dihydro-1,4-benzodioxin-6-yl)-4,9-éthano-2-[2-(2-méthoxyphényl)propènoyl]-2,3,3 a,4,9,9a-hexahydro-1H-benzo[f]isoindole -3a-N-(2-thiénylméthyl)carboxamide-(3aRS,4SR,9SR,9aRS)
acide 4,9-éthano-2-[(2-méthoxyphényl)acétyl)-9-(3,4,5-triméthylphényl)-2,3,3a,4,9,9a-hexahydro-1H-benzo[f]isoindole-3a-carboxylique-(3aRS,4SR,9SR,9aRS)
acide 4,9-éthano-2-[2-(2-méthoxyphényl)propènoyl]-9-(2-méthylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-trifluoromethylphényl)-2,3,3a,4,9,9a-hexahydro-1H-benzo[f]isoindole-3a-N-(4-pyridylméthyl)-carboxamide-(3aRS,4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-N'-benzoyl-carbohydrazide-(3aRS,4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-N'-phényl-carbohydrazide-(3aRS,4SR,9SR,9aRS)
4,9-éthano-2-[2-(2-méthoxyphényl)propènoyl]-9-(2-naphtyl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR, 9aRS)
4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR, 9SR,9aRS)
9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)
acide 9-(4-bromophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR, 9aRS)
acide 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-chlorophényl)-2,3,3a,4,9,9a-hexahydro-1H-benzo[f]isoindole-3a-carboxylate de méthyle-(3aRS, 4SR,9SR,9aRS)
acide 9-(4-chlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(3-indolyl)-2,3,3a,4,9,9a-hexahydro-1H-benzo[f]isoindole-3a-carboxylate de méthyle-(3aRS, 4SR,9SR,9aRS)
acide 4,9-éthano-9-(4-isopropylphényl)-2-[(2-méthoxyphényl)acétyl]-2,3,3a,4,9,9a-hexahydro-1H-benzo[f]isoindole-3a-carboxylique-(3aRS,4SR,9SR, 9aRS)
acide 4,9-éthano-9-(4-isopropylphényl)-2-[2-(2-méthoxyphényl)propènoyl]-2,3,3a,4,9,9a-hexahydro-1H-benzo[f]isoindole-3a-carboxylique-(3aRS, 4SR,9SR, 9aRS)
4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS, 4SR, 9SR,9aRS)
4,9-éthano-9-(4-éthylphényl)-2-[2-(2-méthoxyphényl)propènoyl]- 2,3,3a,4,9,9a-hexahydro-1H-benzo[f]isoindole-3a-carboxylate de méthyle-(3aRS, 4SR, 9SR,9aRS)
acide 4,9-éthano-9-(4-éthylphényl)-2-[2-(2-méthoxyphényl)propènoyl]-2,3,3a,4,9,9a-hexahydro-1H-benzo[f]isoindole-3a-carboxylique-(3aRS,4SR,9SR, 9aRS)
9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)
9-(2,3-dihydrobenzofuran-5-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS)
4,9-éthano-9-(4-fluorophényl)-2-[2-(2-méthoxyphényl)propènoyl]- 2,3,3a,4,9,9a-hexahydro-1H-benzo[f]isoindole-3a-carboxylate de méthyle-(3aRS, 4SR,9SR,9aRS) acide 4,9-éthano-9-(4-fluorophényl)-2-[2-(2-méthoxyphényl)propènoyl]-2,3,3a,4,9,9a-hexahydro-1H-benzo[f]isoindole-3a-carboxylique-(3aRS, 4SR,9SR,9aRS)
9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(3-méthylphényl)-2,3,3a,4,9,9a-hexahydro-1H-benzo[f]isoindole-3a-carhoxylate de méthyle-(3aRS, 4SR,9SR,9aRS)
acide 4,9-éthano-2-[2-(2-méthoxyphényl)propènoyl]-9-(3-méthylphényl)-2,3,3a,4,9,9a-hexahydro-1H-benzo[f]isoindole-3a-carboxylique-(3aRS, 4SR,9SR,9aRS)
9-(1,3-benzodioxol-5-yl)-4,9-éthano-2-[2-(2-méthoxyphényl) propènoyl]-2,3,3a,4,9,9a-hexahydro-1H-benzo[f]isoindole-3a-carboxylate de méthyle-(3aRS, 4SR,9SR,9aRS)
9-(3,4-diméthylphényl)-4,9-éthano-2[2-(2-méthoxy phényl)propènoyl]-2,3,3a,4,9,9a-hexahydro-1H-benzo[f]isoindole-3a-carboxylate de méthyle-(3aRS,4SR,9SR,9aRS) acide-9-(3,4-diméthylphényl)-4,9-éthano-2[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-9-(4-méthoxyphényl)-2-[2-(2-méthoxyphényl)propènoyl]- 2,3,3a,4,9,9a-hexahydro-1H-benzo[f]isoindole-3a-carboxylate de méthyle-(3aRS, 4SR,9SR,9aRS)
acide 4,9-éthano-9-(4-méthoxyphényl)-2-[2-(2-méthoxyphényl) propènoyl]-2,3,3a,4,9,9a-hexahydro-1H-benzo[f]isoindole-3a-carboxylique-(3aRS, 4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N-(3-pyridylméthyl)-carboxamide-(3aRS,4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N'-phénylcarbohydrazide-(3aRS,4SR,9SR,9aRS)
acide 4,9-éthano-9-(4-méthoxyphényl)-2-[2-(2-méthoxyphényl)propènoyl]-2,3,3a,4,9,9a-hexahydro-1H-benzo[f]isoindole-3a-hydroxamique-(3aRS,4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N'-(3-pyridyl)carbohydrazide-(3aRS,4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N-(3-thiénylméthyl)-carboxamide-(3aRS,4SR,9SR,9aRS)
acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétiques-(RS) et (SR)
acide 4,9-éthano-2-[2-(2-méthoxyphényl)propènoyl]-9-(4-trifluorométhoxyphényl)-2,3,3a,4,9,9,9a-hexahydro-1H-benzo[f]isoindole-3a-carboxylique-(3aRS, 4SR, 9SR,9aRS)
9-(3-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)
acide 9-(3-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS)
4,9-éthano-9-(3-fluorophényl-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 4,9-éthano-9-(3-fluorophényl)-2-[2-(2-méthoxyphényl)propènoyl])-2,3,3a,4,9,9a-hexahydro-1H-benzo[f]-isoindole-3a-carboxylique-(3aRS, 4SR, 9SR, 9aRS)
4,9-éthano-9-(3-fluorophényl)-2-[2-(2-méthoxyphényl)propènoyl)]-2,3,3a,4,9,9a-hexahydro-1H-benzo[f]isoindole-3a-[N-(3-pyridylméthyl)carboxamide-(3aRS,4SR,9SR,9aRS)
9-(3-chlorophényl)-4,9-éthano-2-[2-(2-méthoxyphényl)propènoyl]-2,3,3a,4,9,9a-hexahydro- 1H-benzo[f]isoindole-3a-carboxylate de méthyle-(3aRS, 4SR,9SR,9aRS)
acide 9-(3-chlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
9-(3-chlorophényl)-4,9-éthano-2-[2-(2-méthoxyphényl)propènoyl)]-2,3,3a,4,9,9a-hexahydro-1H-benzo[f]isoindole 3a-[N-(3-pyridylméthyl)carboxamide-(3aRS,4SR,9SR,9aRS)
9-(3-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
acide 9-(3-N,N-diméthylaminophényl)-4,9-éthano-2-[2-(2-méthoxyphényl) propènoyl]-2,3,3a,4,9,9,9a-hexahydro-1H-benzo[f]isoindole-3a-carboxylique-(3aRS,4SR,9SR, 9aRS)
chlorhydrate de 9-(3-aminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
9-(4-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
acide 9-(4-cyanophényl)-4,9-éthano-2-[2-(2-méthoxyphényl)propènoyl]-2,3,3a,4,9,9,9a-hexahydro-1H-benzo[f]isoindole-3a-carboxylique-(3aRS,4SR,9SR, 9aRS)
9-(3-cyanophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
acide 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)
4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylate de méthyle-(3aRS,4SR, 9SR,9aRS)
acide 4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylique-(3aRS,4SR, 9SR,9aRS)
acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)
acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 4,8-éthano-2-[2-(2-méthoxyphényl)propènoyl]-8-phényl-2,3,3a,4,8,8a-hexahydro-1H-thiéno[2,3-f]isoindole-3a-carboxylique-(3aRS,4SR,8SR,8aRS)
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)
acide 4,8-éthano-2-[2-(2-méthoxyphényl)propénoyl]-8-(4-trifluorométhylphényl)-2,3,3a,4,8,8a-hexahydro-1H-thiéno[2,3-f]isoindole-3a-carboxylique-(3aRS,4SR,8SR,8aRS)
N-benzyl-4,8-éthano-2-[2-(2-méthoxyphényl)propènoyl]-8-(4-trifluorométhylphényl)-2,3,3a,4,8,8a-hexahydro-1H-thiéno[2,3-f]isoindole-3a-carboxamide-(3aRS,4SR,8SR,8aRS)
acide 4,9-éthano-2-[2-(2-méthoxyphényl)propènoyl]-10-(4-méthylphényl)-2,3,3a,4,10,10a-hexahydro-1H-indolo[2,3-f]isoindole-3a-carboxylique-(3aRS,4SR,10SR,10aRS)
N-(3-pyridyl)méthyl-4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxamide-(3aRS,4SR,8SR,8aRS).
4,8-éthano-8-(4-méthoxyphényl)-2-[2-(2-méthoxyphényl)propènoyl]-2,3,3a,4,8,8a-hexahydro-1H-thiéno[2,3-f]isoindole-3a-carboxylate de méthyle-(3aRS,4SR,8SR,8aRS).
acide 4,8-éthano-8-(4-méthoxyphényl)-2-[2-(2-méthoxyphényl)propènoyl]-2,3,3a,4,8,8a-hexahydro-1H-thiéno[2,3-f]isoindole-3a-carboxylique-(3aRS,4SR,8SR,8aRS).
N-(3-pyridyl)méthyl-8-(benzo-1,4-dioxan-6-yl)-4,8-éthano-2-[2-(2-méthoxyphényl)propènoyl]-2,3,3a,4,8,8a-hexahydro-1H-thiéno[2,3-f]isoindole-3a-carboxamide-(3aRS,4SR,8SR,8aRS).
sous forme racémique ou leurs isomères optiques ainsi que leurs sels.

Dans ce qui précède et ce qui suit, l'expression "isomère optique" ou forme optiquement active définit la forme pure dudit isomère optique ou éventuellement le mélange des isomères optiques "enrichi", c'est-à-dire contenant majoritairement ledit isomère optique ou ladite forme.

Plus préférentiellement, on peut encore citer selon l'invention, tout composé de formule générale (I) sélectionné individuellement parmi :
acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)
acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS
acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 9-(3,4-dichlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)
acide 9-(4-bromophényl)-4,9-éthano-2-[2-(2-méthoxyphényl)propénoyl]-2,3,3a,4,9,9a-hexahydro-1H-benzo[f]isoindole-3a-carboxylique -(3aRS, 4SR, 9SR,9aRS)
acide 4,9-éthano-2-[2-(2-méthoxyphényl)propènoyl]-9-(4-chlorophényl)-2,3,3a,4,9,9a-hexahydro-1H-benzo[f]isoindole-3a-carboxylique-(3aRS,4SR,9SR,9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)
sous forme de leurs isomères optiques ainsi que leurs sels.

A titre d'isomère optique tout particulièrement avantageux des composés de formule générale (I) selon l'invention, on peut citer tout composé choisi isolément parmi :
acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)
acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS
acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 9-(3,4-dichlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)
acide 9-(4-bromophényl)-4,9-éthano-2-[2-(2-méthoxyphényl)propènoyl]-2,3,3a,4,9,9a-hexahydro-1H-benzo[f]isoindole-3a-carboxylique -(3aRS, 4SR, 9SR,9aRS)
acide 4,9-éthano-2-[2-(2-méthoxyphényl)propènoyl]-9-(4-chlorophényl)-2,3,3a,4,9,9a-hexahydro-1H-benzo[f]isoindole-3a-carboxylique-(3aRS,4SR,9SR,9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)
ou leurs sels.

Selon les demandes WO 98/29390 et FR2772764, le procédé de préparation des produits de formule générale (I) met en oeuvre l'intermédiaire de formule générale (X) : 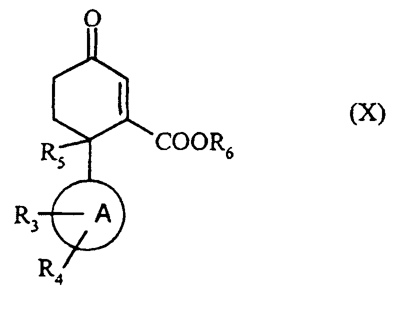 dans laquelle A, R₃, R₄, R₅, R₆ sont tels que définis en formule générale (I), sous forme racémique, conduisant à des produits ultérieurs également sous forme racémique.

Selon la présente invention, la stéréochimie des produits de formule générale (X) sous forme racémique peut être résolue, en formant les isomères optiques (X') des produits de formule générale (X).

Cette étape permet de mettre en oeuvre les étapes ultérieures du procédé de préparation à partir des isomères optiques de formule (X') des produits de formule générale (X). 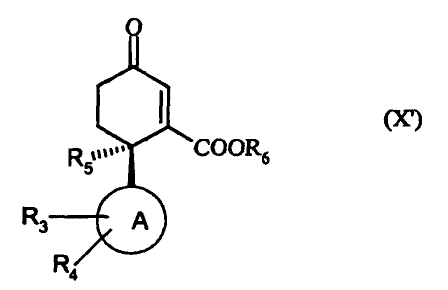 dans laquelle A, R₃ , R₄, R₅ et R₆ sont définis comme précédemment.

Il est entendu que les autres étapes ultérieures du procédé décrites ci-avant et ci-après restent inchangées et peuvent être conduites de la même façon sur le mélange racémique (X) ou les isomères optiques des composés de formule générale (X').
Le procédé de préparation mettant en oeuvre la résolution énantiomérique du composé (X') est plus simple et plus performant que celui mettant en oeuvre le mélange racémique du composé (X) : en effet, cette voie permet d'éviter l'étape finale de séparation des énantiomères du mélange racémique et permet également d'augmenter le rendement global tout en économisant la quantité de réactifs mis en oeuvre dans les étapes ultérieures à l'étape enzymatique.
Ce procédé fait également partie de la présente invention.

Les isomères optiques de formule générale (X') peuvent être obtenus à partir des composés de formule générale (X) de la façon suivante :
La séparation des isomères optiques de formule générale (X') des composés de formule générale (X) peut être effectuée à partir des composés de formule générale (X) sous forme de mélange racémique, de façon avantageuse par dédoublement enzymatique.
Très avantageusement, il a été découvert selon l'invention que la résolution énantiomérique pouvait être obtenue en effectuant une réaction enzymatique énantiospécifique.

Comme enzyme peut être utilisée de préférence une enzyme permettant de séparer les énantiomères de formule générale (X') de stéréochimie désirée, tels que lévogyres des autres énantiomères, tels que dextrogyres ou leurs dérivés.

Selon l'invention, l'enzyme mise en oeuvre est choisie parmi les lipases, estérases ou protéases.
Préférentiellement, on utilise une lipase ou une estérase pour préparer le produit de formule générale (X') sous forme lévogyre.
Préférentiellement, on utilise une protéase ou une lipase pour préparer le produit de formule générale (X') sous forme dextrogyre.

Les lipases, estérases et protéases et leurs utilisations sont notamment décrites dans Enzyme catalysis in organic synthesis, K. Drauz *et al*., VCH.

Parmi les lipases et estérases permettant d'obtenir le produit de formule générale X' sous forme levogyre, on préfère :
Chirazyme® L2 (commercialisée par Boehringer Mannheim), Lipase PS C (commercialisée par Amano Enzymes), ESL 01 (commercialisée par Recombinant Biocatalyst), SP 525 (commercialisée par Novo Nordisk), Chirazyme® L6 (commercialisée par Boehringer Mannheim), Estérase 30000 (commercialisée par Gist Brocades)

Parmi les protéases et lipases permettant d'obtenir le produit de formule générale X' sous forme dextrogyre, on préfère:
Pronase® (Boehringer Mannheim.), Subtilisin (Novo Nordisk), Alacalase 2,4 L (Novo Nordisk), Protéase Nagarse (Sigma), EH 16 (Altus Biologics), EH 15 (Altus Biologies), EH 11 (Altus Biol.), Lipase M (Amano Enzymes)

Les enzymes utilisées peuvent être disponibles commercialement, ou dérivées de microorganismes convenables, pouvant être choisis par exemple dans le Catalogue de l'American Type Culture Collection (ATCC), éventuellement génétiquement modifiés, selon les méthodes classiques connues de l'homme de l'art, incluant notamment l'utilisation des enzymes isolées ou d'extraits cellulaires.
Préférentiellement, on utilise une protéase pour préparer le produit de formule générale (X') sous forme dextrogyre.

Les lipases, estérases et protéases et leurs utilisations sont notamment décrites dans Enzyme catalysis in organic synthesis, K. Drauz *et al*., VCH.

Généralement, la réaction est effectuée à partir d'un mélange d'une solution contenant le produit de formule générale (X) sous forme racémique, dans un solvant organique hydrocarboné tel que le cyclohexane, et d'une solution aqueuse d'hydrogenophosphate de potassium, de préférence ajustée à pH compris entre 6 et 8, par exemple à l'aide d'acide phosphorique, auquel est ajoutée une solution aqueuse de l'enzyme appropriée.
Généralement, la concentration du produit de formule générale (X) dans le mélange réctionnel est comprise entre 5 et 50 g/l.
De préférence, le volume de la solution organique représente 10 à 30 % du volume total de la solution aqueuse.
De préférence, la quantité d'enzyme mise en oeuvre dans le mélange réactionnel est comprise entre 0.4 et 4 MU/l ( 1 MU : unité d'activité enzymatique).
Généralement, la réaction est mise en oeuvre à température comprise entre 20 et 40°C, sous agitation et son avancement peut être suivi par HPLC chirale. La phase organique peut ensuite éventuellement être séparée de la phase aqueuse puis clarifiée par addition d'un solvant ou un mélange de solvants appropriés, comme par exemple les alcools aliphatiques tel que l'éthanol.

Très avantageuseument, il a été découvert selon l'invention, que la lipase de Candida Antartica " fraction B ", comme par exemple l'enzyme Chirazyme® L2 commercialisée par Boerhringer Mannheim, peut effectuer la réaction conduisant au composé de formule générale (X') avec un excès énantiomérique supérieur à 90 %.

Très avantageusement, il a été découvert selon la présente invention que les composés de formule générale (X') sous forme lévogyre permettaient d'aboutir directement aux composés de formule générale (I), sous forme dextrogyre, décrits comme particulièrement avantageux dans les demandes WO 98/29390 et FR2772764.

La présente invention concerne également l'utilisation du composé de formule générale (X') pour la préparation des composés de formule générale (I) sous forme d'isomères optiques :
Les étapes ultérieures du procédé sont ensuite effectuées sur l'isomère optique de formule générale (X') de la même façon que pour le procédé décrit dans les demandes WO 98/29390 et FR2772764 mettant en oeuvre le mélange racémique de formule générale (X).

### A savoir :

Le produit de formule générale (X') est transformé en produit de formule générale (IX), sous forme d'isomères optiques, de préférence l'isomère lévogyre : 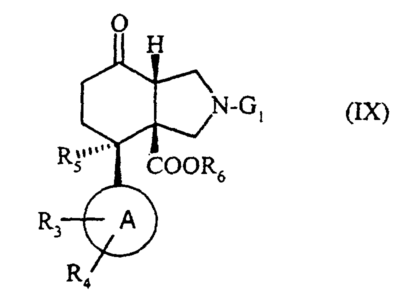 dans laquelle A, R₃, R_{4,} R₅, R₆ sont tels que définis en formule générale I, G₁ représente un groupement protecteur de la fonction amine, tel que benzyle, par action d'une N-trialcoylsilylméthyl-N-alcoxyméthyl-amine portant un groupement protecteur de la fonction amine tel qu'un radical benzyle, comme la N-triméthylsilylméthyl-N-n.butoxyméthyl-benzylamine qui peut être préparée dans les conditions décrites dans Chem. Pharm. Bull., 276 (1985).

Généralement, la réaction est effectuée dans un solvant organique tel qu'un hydrocarbure aliphatique halogéné comme le dichlorométhane en présence d'un acide fort tel que l'acide trifluoroacétique à une température comprise entre 0°C et le reflux du mélange réactionnel.

Les produits de formule générale (IX) sous forme d'isomères optiques sont ensuite mis en oeuvre selon le procédé décrit dans les demandes WO 98/29390 et FR2772764 selon les voies possibles conduisant aux produits de formule générale (I) sous forme d'isomères optiques.

1. Selon une première voie, les produits de formule générale (I) sous forme d'isomères optiques, peuvent être obtenus à partir des produits de formule générale (IX), par cyclisation du noyau cycloperhydroisoindole en passant par les composés de formule générale (III) 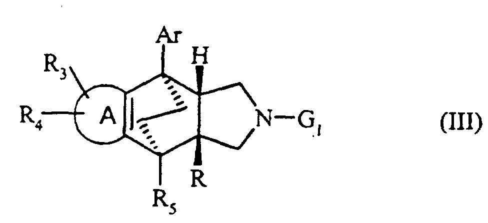 sous forme d'isomères optiques, de préférence l'isomère dextrogyre,
dans laquelle:
- A, Ar, R, R₃, R₄ et R₅sont définis selon la formule générale I et
- G₁ représente un atome d'hydrogène,
puis indifféremment par couplage avec la chaîne latérale, et éventuellement modification du substituant R.

Les produits de formule générale (I) pour lesquels :
- Y représente un atome d'oxygène ou de soufre et
- X représente un groupement -CO-, méthylène, alcèn-1,1-diyle tel que vinyldiyle ou cycloalcan-1,1-diyle contenant 3 à 6 atomes de carbone,
peuvent être obtenus par action d'un acide de formule générale (II) : 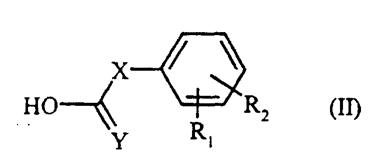 dans laquelle :
- R₁, R₂ sont définis selon la formule générale I et X défini comme précédemment et
- Y représente un atome d'oxygène ou de soufre,
de son ester méthylique ou d'un dérivé de cet acide tel qu'un halogénure ou l'anhydride, sur un produit de formule générale (III) : 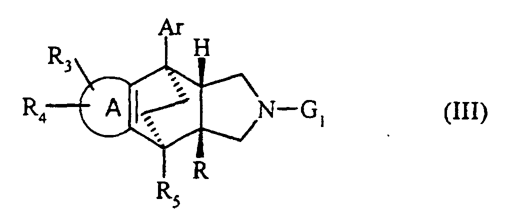 dans laquelle:
- A, Ar, R, R_{3,} R₄ et R₅sont définis selon la formule générale I et
- G₁ représente un atome d'hydrogène (qui peut être obtenu à partir d'un produit de formule générale (III) dans laquelle G₁ représente un groupement protecteur d'une fonction amino tel qu'un radical benzyle, benzyloxycarbonyle, *tert*-butoxycarbonyle ou vinyloxycarbonyle par hydrogénolyse en présence d'un catalyseur tel que le palladium sur charbon, lorsque G₁ représente un radical benzyle ou benzyloxycarbonyle, ou par hydrolyse en milieu acide, lorsque G₁ représente un radical *tert*-butoxycarbonyle, vinyloxycarbonyle ou benzyloxycarbonyle).

Dans ce qui suit, la définition pour laquelle G₁ représente un radical benzyle n'est donnée qu'à titre illustratif, et il est évident à l'homme de l'art d'adapter les divers protocoles aux autres groupes protecteurs d'une fonction amino tel qu'un radical benzyle, benzyloxycarbonyle, *tert*-butoxycarbonyle ou vinyloxycarbonyle

Généralement, la réaction du produit de formule générale (II) sous forme acide sur le produit de formule générale (III) est effectuée en opérant dans un solvant organique tel qu'un hydrocarbure aliphatique halogéné tel que le dichlorométhane en présence d'un agent de condensation tel que le chlorhydrate de 1-éthyl-3-[3-(diméthylamino)propyl]carbodiimide, le 1,3-dicyclohexylcarbodiimide ou l'hexafluorophosphate de benzotriazol-1-yloxytris(diméthylamino)phosphonium et éventuellement d'un agent d'activation tel que l'hydroxybenzotriazole à une température comprise entre 0°C et la température de reflux du mélange réactionnel.

Généralement, la réaction du produit de formule générale (II) sous forme d'ester méthylique sur un produit de formule générale (III) est effectuée en opérant dans un solvant organique tel que le dioxane ou un hydrocarbure aliphatique halogéné tel que le dichlorométhane à une température comprise entre 0°C et la température de reflux du mélange réactionnel.

Généralement, la réaction du produit de formule générale (II) sous forme d'halogénure sur le produit de formule générale (III) est effectuée en opérant dans un solvant organique tel qu'un hydrocarbure aliphatique halogéné tel que le dichlorométhane en présence d'une base (amine aliphatique tertiaire) à une température comprise entre 0°C et la température de reflux du mélange réactionnel.

Généralement, la réaction du produit de formule générale (II) sous forme d'anhydride sur le produit de formule générale (III) est effectuée en opérant dans un solvant organique tel qu'un hydrocarbure aliphatique halogéné comme le dichlorométhane en présence d'une base (amine aliphatique tertiaire, pyridine ou 4-diméthylaminopyridine) à une température comprise entre 0 et 50°C.

En outre, à l'issue de la réaction de II sur III, on peut éventuellement, lorsque R représente ou contient un radical -COOR₆ ou -PO(OR₉)₂ avec R₆ et R₉ représentant un radical alcoyle,
- procéder à une saponification du produit obtenu pour obtenir un produit de formule générale (I) dans laquelle R représente ou contient un radical carboxy ou
- procéder à la transformation au moyen d'agent nucléophile du produit obtenu pour obtenir un produit de formule générale (I) dans laquelle R représente ou contient un radical -PO₃H₂.

Généralement la saponification d'un produit de formule générale (I) dans laquelle R représente ou contient un ester de formule générale -COOR₆ en produit de formule générale (I) dans laquelle R représente ou contient un radical carboxy est effectuée au moyen d'une base minérale telle que la soude ou la potasse ou le carbonate de sodium dans un solvant organique tel qu'un alcool comme le méthanol ou l'éthanol ou tel qu'un éther comme le dioxane, à une température comprise entre 20°C et le reflux du solvant.

Généralement la transformation d'un produit de formule générale (I) dans laquelle R représente ou contient un radical PO(OR₉)₂ en produit de formule générale (I) dans laquelle R représente ou contient un radical PO₃H₂ s'effectue par action d'un agent nucléophile tel qu'un halogénure (iodure) de trialcoylsilyle (triméthylsilyle) ou d'un halogénure de sodium ou de lithium (iodure de sodium) en présence d'un trialcoylhalogénosilane (triméthylchlorosilane, triméthylbromosilane) en opérant dans un solvant tel que le tétrachlorure de carbone ou l'acétonitrile à une température comprise entre 0 et 50°C ou par chauffage avec un halogénure de métal alcalin (iodure de sodium), suivi d'une hydrolyse.
La présente invention concerne également l'utilisation des produits de formule générale (III) dans le procédé revendiqué : 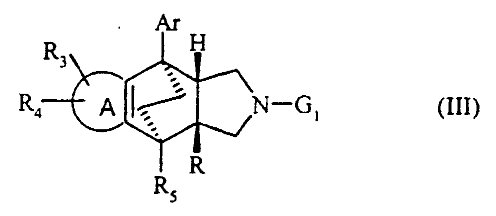 sous forme d'isomères optiques, de préférence l'isomère dextrogyre :
dans laquelle:
- Ar, R, R₃, R₄ et R₅ sont définis selon la formule générale I et
- G₁ représente un atome d'hydrogène ou un groupe protecteur d'une fonction amino, tel que benzyl.

Les produits de formule générale (I) dans laquelle:
- Y représente un atome d'oxygène ou de soufre et
- X représente un atome d'oxygène
peuvent être obtenus par action d'un halogénoformiate ou d'un halogénothioformiate de formule générale (IV) : 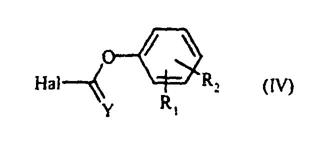 dans laquelle :
- Y représente un atome d'oxygène ou de soufre,
- R₁ et R₂ sont définis comme précédemment et
- Hal représente un atome d'halogène,
sur un produit de formule générale (III).

Généralement, la réaction de l'halogénure de formule générale (IV) sur le produit de formule générale (III) est effectuée en milieu organique ou hydro-organique tel qu'un mélange dioxane-eau en présence d'une base minérale (soude) ou organique (triéthylamine) à une température comprise entre 0 et 50°C.

De la même façon que précédemment, lorsque R représente ou contient un radical -COOR₆ ou -PO(OR₉)₂, il est possible de saponifier le produit obtenu pour obtenir un produit de formule générale (I) dans laquelle R représente ou contient un radical carboxy ou de transformer au moyen d'agent nucléophile le produit obtenu en un produit de formule générale (I) dans laquelle R représente ou contient un radical -PO₃H₂.

Les produits de formule générale (I) dans laquelle :
- Y représente un atome d'oxygène ou de soufre et
- X représente un groupement NH
peuvent être obtenus par action d'un isocyanate ou d'un isothiocyanate de formule générale (V) : 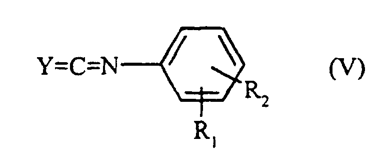 dans laquelle :
Y représente un atome d'oxygène ou de soufre et
R₁ et R₂ sont définis selon la formule générale I
sur un produit de formule générale (III) tel que défini précédemment.

Généralement, la réaction du produit de formule générale (V) sur le produit de formule générale (III) est effectuée dans un solvant organique inerte tel que le tétrahydrofurane ou le toluène en présence d'un agent d'activation tel que la 4-diméthylamino-pyridine ou la 4-pyrrolidino-pyridine à une température comprise entre 0 et 50°C.

La réaction de V sur II est suivie éventuellement, lorsque R représente ou contient un radical -COOR₆ ou -PO(OR₉)₂ dans lesquels R₆ ou R₉ représentent un radical alcoyle, de la saponification du produit obtenu pour obtenir un produit de formule générale (I) dans laquelle R représente ou contient un radical carboxy ou de la transformation au moyen d'agent nucléophile du produit obtenu pour obtenir un produit de formule générale (I) dans laquelle R représente ou contient un radical -PO₃H₂. La transformation éventuelle des radicaux -COOR₆ et PO(OR₉)₂ respectivement par des radicaux carboxy et PO₃H₂ s'effectue dans les conditions décrites précédemment.

Les produits de formule générale (I) dans laquelle :
- R représente un radical de formule générale

   -(CH₂)ₘ-X₁-(CH₂)ₙ-Z

   avec X₁, m et n définis comme précédemment et
   Z représentant un radical -COOR₆ avec R₆ représentant un radical alcoyle droit ou ramifié contenant 1 à 3 atomes de carbone,
peuvent être obtenus par estérification d'un produit de formule générale (I) dans laquelle Z représente un radical carboxy.

Généralement, l'estérification est effectuée au moyen d'un alcool de formule générale R₆-OH dans laquelle R₆ est défini comme précédemment en opérant en milieu acide ou au moyen d'un halogénure d'alcoyle de formule générale R₆-Hal dans laquelle Hal représente un atome d'halogène (iode) en opérant en milieu alcalin (carbonate de métal alcalin ou alcalino-terreux tel que le carbonate de césium) en opérant dans un solvant organique tel que le diméthylformamide à une température comprise entre 0 et 50°C.

Les produits de formule générale (I) dans laquelle:
R représente un radical de formule générale

   -(CH₂)ₘ-X₁-(CH₂)ₙ-Z

   dans laquelle:
   X₁, m et n sont définis comme précédemment et
   Z représente un radical -CON(R₇)(R₈)
      dans lequel :
      R₇ représente un atome d'hydrogène ou un radical alcoyle droit ou ramifié contenant 1 à 6 atomes de carbone et
      R₈ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, alcoxy contenant 1 à 4 atomes de carbone, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs radicaux identiques ou différents choisis parmi les radicaux alcoxy contenant 1 à 4 atomes de carbone ou trifluorométhyle, naphtyl-1 ou -2, furyl-2 ou -3, thiényl-2 ou -3, imidazolyl-4 ou -5 ou thiazolyl-4 ou -5, pyridyl-2, -3 ou -4, ou un radical indanyle ou chromanyle
ou bien
R₇ représente un atome d'hydrogène ou un radical alcoyle droit ou ramifié contenant 1 à 6 atomes de carbone et
R₈ représente un radical hydroxy, amino, alcoyloxy contenant 1 à 6 atomes de carbone en chaîne droite ou ramifiée éventuellement substitué par un radical phényl, alcoylamino ou dialcoylamino dont les parties alcoyles contiennent de 1 à 4 atomes de carbone,
ou bien préférentiellement dans lequel
- R₇ représente un atome d'hydrogène ou un radical alcoyle droit ou ramifié contenant 1 à 6 atomes de carbone et
- R₈ représente
   - un atome d'hydrogène,
   - un radical hydroxy,
   - un radical arylsulfonyle, tel que phénylsulfonyle, éventuellement substitué par un ou plusieurs atomes ou radicaux, identiques ou différents, choisis parmi les atomes d'halogène et les radicaux alcoyles, alcoyloxy, avec, pour ces radicaux alcoyl contenant 1 à 4 atomes de carbone,
   - un hétérocycle de 5 à 7 chaînons incorporant un ou plusieurs hétéroatomes choisis parmi les atomes d'azote, d'oxygène ou de soufre, ledit hétérocycle pouvant être lié par un bétéroatome,
   - un radical amino éventuellement substitué par un ou deux radicaux, identiques ou différents choisis parmi les radicaux
      - alcoyle contenant 1 à 4 atomes de carbone,
      - aryle, tel que phényle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
      - hétérocyclyle de 5 à 7 chaînons et contenant un ou plusieurs hétéroatomes choisis parmi les atomes d'azote, d'oxygène et de soufre,
      - arylcarbonyle, tel que benzoyle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
   - un radical alcoyloxy contenant 1 à 6 atomes de carbone en chaîne droite ou ramifiée éventuellement substitué par un radical phényle,
   - un radical alcoyle droit ou ramifié contenant 1 à 6 atomes de carbone, tel que méthyle, éventuellement substitué par un radical amino, alcoylamino, dialcoylamino, hydroxy, alcoxy contenant 1 à 4 atomes de carbone, mercapto, alcoylthio, alcoyloxycarbonyle, carboxy, cyano, un radical aromatique mono- ou polycyclique et ayant de 5 à 12 chaînons, incorporant ou non, un ou plusieurs hétéroatomes choisis parmi les atomes d'oxygène, d'azote et de soufre éventuellement substitué, ledit radical aromatique pouvant être notamment le radical 2-, ou 3-, ou 4- pyridyle, préférentiellement le 3-pyridyle ou le 4-pyridyle, ou le N-oxyde de pyridine, ou pouvant être également un radical phényle éventuellement substitué par un ou plusieurs atomes d'halogène ou par un ou plusieurs groupements hydroxy, amino, ou trifluorométhyle, ou par un ou plusieurs radicaux alcoyle ou alcényle, alcoxy, alcoylthio, alcoylamino, alcoylcarbonyle ou alcoxycarbonyle en C2 à C4, carbamoyle, alcoylcarbamoyle ou dialcoylcarbamoyle dont la partie alcoyle est en C1 à C8 , formyle ou encore un radical naphtyle-1 ou -2,
préférentiellement R₇ représente un atome d'hydrogène, R₈ représente un radical méthyle substitué par le radical 3-pyridyle, peuvent être obtenus par action d'un produit de formule générale :

HN(R₇)(R₈)

dans laquelle R₇ et R₈ sont définis comme ci-dessus
sur un produit de formule générale (I) dans laquelle Z représente un radical carboxy.

Il est particulièrement avantageux :
- soit de faire réagir d'abord le chlorure d'oxalyle avec un composé de formule générale (I) dans lequel R représente un radical carboxy en solution dans du dichlorométhane pour former intermédiairement le chlorure d'acide, puis de faire réagir le composé de formule générale HN(R₇)(R₈), éventuellement en présence d'une base telle que la triéthylamine,
- soit de faire réagir directement le composé de formule générale HN(R₇)(R₈) avec un composé de formule générale (I) dans lequel R représente un radical carboxy dans un solvant organique tel qu'un alcool (éthanol) ou un solvant halogéné tel que le dichlorométhane en présence d'un agent de condensation tel que le N,N'-carbonyldiimidazole, le 1,1-dicyclohexylcarbodiimide, le chlorhydrate de 1-éthyl-3-[3-(diméthylamino)propyl]carbodiimide, l'hexafluorophosphate de benzo-triazol-1-yloxytris(diméthylamino) phosphonium à une température comprise entre 0 et 50°C.

Lorsque l'un au moins des symboles R₇ et R₈ est substitué par un radical amino, il est particulièrement avantageux de le protéger par un groupement protecteur tel qu'un radical *tert*-butoxycarbonyle, benzyloxycarbonyle ou benzyle préalablement à la condensation de l'amine de formule générale HN(R₇)(R₈) sur l'acide approprié puis à remplacer le groupement protecteur par un atome d'hydrogène par exemple par hydrogénolyse au moyen d'hydrogène en présence d'un catalyseur tel que le palladium sur charbon, lorsqu'il représente un radical benzyle ou benzyloxycarbonyle, ou par hydrolyse en milieu acide, lorsqu'il représente un radical *tert*-butoxycarbonyle ou benzyloxycarbonyle.

Lorsque l'un au moins des symboles R₇ et R₈ est substitué par un radical carboxy, il est particulièrement avantageux de le protéger par un groupement protecteur tel qu'un radical alcoyle éventuellement substitué par un radical phényle tel que le radical benzyle préalablement à la condensation de l'amine de formule générale HN(R₇)(R₈) sur l'acide approprié puis de remplacer le groupement protecteur par un atome d'hydrogène par exemple par hydrogénolyse au moyen d'hydrogène en présence d'un catalyseur tel que le palladium sur charbon ou par saponification dans les conditions décrites ci-dessus.

Lorsque, dans le produit de formule générale (I), R₇ représente un atome d'hydrogène ou un radical alcoyle contenant 1 à 6 atomes de carbone et R₈ représente un radical alcoyloxy substitué par un radical phényle, le remplacement du radical alcoyloxy, substitué par un radical phényle, par un radical hydroxy, effectué :
- soit par hydrogénolyse en présence d'un catalyseur tel que le palladium sur charbon,
- soit par traitement avec du chlorure d'aluminium en présence d'anisole dans un solvant organique tel que le nitrométhane à une température comprise entre -20°C et l'ambiante lorsque le radical alcoyle subtitué par un radical phényle est un radical benzyle,
permet d'obtenir un produit de formule générale (I) dans laquelle R₇ représente un atome d'hydrogène ou un radical alcoyle contenant 1 à 4 atomes de carbone et R₈ représente un radical hydroxy.

Les produits de formule générale (I) dans laquelle:
- R représente un radical de formule générale :

   -NHCO-T

   dans laquelle T représente un atome d'hydrogène ou un radical alcoyle (1 à 6 atomes de carbone) éventuellement substitué par un radical amino, carboxy, alcoyloxycarbonyle, hydroxy, alcoyloxy, mercapto ou alcoylthio,
peuvent être obtenus par action d'un acide de formule générale :

T-CO-OH

dans laquelle T est défini comme ci-dessus
sur un produit de formule générale VI : 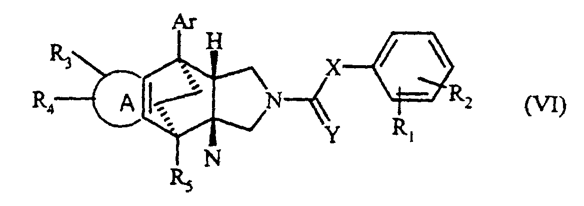 dans laquelle :
A, Ar, R₁, R₂, R₃, R₄, R₅ X et Y sont définis selon la formule générale I.

Généralement, la réaction de l'acide de formule générale T-CO-OH sous forme acide sur le produit de formule générale (VI) est effectuée en opérant dans un solvant organique tel qu'un hydrocarbure aliphatique halogéné tel que le dichlorométhane en présence d'un agent de condensation tels que le chlorhydrate de 1-éthyl-3-[3-(diméthylamino)propyl]carbodiimide, le 1,1-dicyclohexylcarbodiimide ou l'hexafluorophosphate de benzotriazol-1-oxy tris(diméthylamino)phosphonium, éventuellement en présence d'un agent d'activation tel que l'hydroxybenzotriazole à une température comprise entre 0 et 50°C.

Généralement, la réaction de l'acide de formule générale T-CO-OH sous forme d'halogénure, lorsque T est différent d'un atome d'hydrogène, sur le produit de formule générale (VI) est effectuée en opérant dans un solvant organique tel qu'un hydrocarbure aliphatique halogéné tel que le dichlorométhane en présence d'une base (amine aliphatique tertiaire) à une température comprise entre 0 et 50°C.

Généralement, la réaction de l'acide de formule générale T-CO-OH sous forme d'anhydride sur le produit de formule générale (VI) est effectuée en opérant dans un solvant organique tel qu'un hydrocarbure aliphatique halogéné comme le dichlorométhane en présence d'une base (amine aliphatique tertiaire, pyridine ou 4-diméthylaminopyridine) à une température comprise entre 0 et 50°C.

La réaction d'un composé T-CO-OH sur un composé VI peut être suivie éventuellement du remplacement des fonctions esters ou des fonctions amines protégées portées par T respectivement par des radicaux carboxy ou amino dans les conditions décrites précédemment.

Lorsque T est substitué par un radical amino, il est particulièrement avantageux de le protéger par un groupement protecteur tel qu'un radical benzyloxycarbonyle ou *tert*-butoxycarbonyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par hydrolyse en milieu acide.

Lorsque T est substitué par un radical carboxy, il est particulièrement avantageux de le protéger par un groupement protecteur tel qu'un radical méthyle, éthyle ou benzyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par saponification dans les conditions décrites précédemment.

Les produits de formule générale (I) dans laquelle: R représente un radical de formule générale 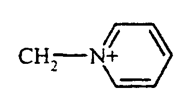 peuvent être obtenus par action d'un excès de pyridine et d'un acide fort ou d'un dérivé de cet acide sur un produit de formule générale : 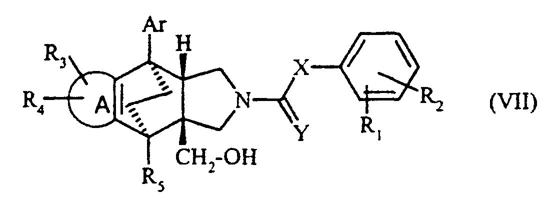 dans laquelle A, Ar, R₁, R₂, R₃, R₄, R₅, X et Y sont définis selon la formule générale I.

De préférence l'acide fort est l'acide trifluorométhanesulfonique éventuellement en présence d'anhydride trifluorométhanesulfonique.

Les produits de formule générale (I) dans laquelle Y représente un atome de soufre peuvent être obtenus par thionation d'un produit de formule générale (I) dans laquelle Y représente un atome d'oxygène.

Généralement, la thionation est effectuée dans les conditions habituelles au moyen de pentasulfure de phosphore en opérant dans un solvant organique tel que le tétrahydrofurane à une température comprise entre 0 et 50°C.

Les produits de formule générale (I) dans laquelle l'un des symboles R₁ ou R₂ représente un radical alcoylcarbonyloxy peuvent être obtenus par acylation d'un produit de formule générale (I) dans laquelle l'un des symboles R₁ ou R₂ représente un radical hydroxy au moyen d'un acide aliphatique ou d'un dérivé de cet acide tel qu'un halogénure ou l'anhydride dans les conditions habituelles d'estérification.

En ce qui concerne les produits intermédiaires décrits ci-dessus, nous proposons ci-après également des protocoles opératoires et des composés utiles pour les obtenir.

Les produits de formule générale (III) sous forme d'isomères optiques, de préférence l'isomère dextrogyre, peuvent être obtenus à partir des produits de formule générale (IX) sous forme d'isomères optiques, de préférence l'isomère lévogyre, selon les voies possibles suivantes :

i. Classiquement, les produits de formule générale (III) dans laquelle R représente un radical carboxy ou un radical de formule générale COOR₆ peuvent être obtenus par action de l'acide trifluorométhanesulfonique sur un produit de formule générale (VIII) : 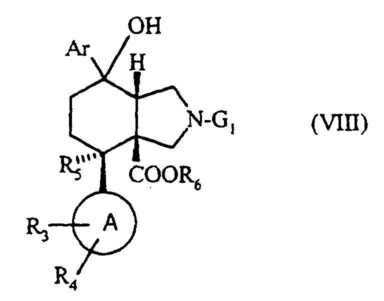 sous forme d'isomères optiques, de préférence l'isomère lévogyre, dans laquelle:
- A, Ar, R₃, R₄, et R₅, R₆ sont définis comme précédemment,
- G₁ représente un radical benzyle,
- R₆ représente un radical alcoyle contenant 1 à 3 atomes de carbone
suivie du remplacement du groupe G₁ par un atome d'hydrogène
- soit par hydrogénolyse dans les conditions décrites ci-dessus puis éventuellement selon le cas du remplacement de l'atome d'hydrogène par un radical *tert*-butoxycarbonyle, par action de l'anhydride *tert*-butoxycarbonyle dans un solvant organique, ou par un radical benzyloxycarbonyle, par action du chlorure de benzyloxycarbonyle dans un solvant organique
- soit par action d'un chloroformiate d'alcoyle, tel que le chloroformiate de vinyle ou le chloroformiate d'éthyle ou le chloroformiate de 2-chloroéthyle ou le chloroformiate de 2,2,2-trichloroéthyle, dans un solvant organique tel que le dichlorométhane à une température comprise entre 0°C et l'ambiante, suivie de l'hydrolyse acide du carbamate intermédiairement formé, généralement à l'aide d'une solution aqueuse 1 à 6 M d'acide chlorhydrique, éventuellement dans un solvant organique tel qu'un alcool, comme le méthanol ou l'éthanol, ou un éther comme le tétrahydrofurane ou le dioxane.

Généralement la cyclisation intramoléculaire de type Friedel-Crafts du produit de formule générale (VIII) en produit de formule générale (III) peut être effectuée par l'action d'un excès, de 3 à 20 équivalents molaires, d'un acide fort tel que l'acide trifluorométhanesulfonique, éventuellement en présence d'anhydride trifluorométhanesulfonique en quantité catalytique ou éventuellement ajouté en ajouts successifs, en opérant dans un solvant organique tel que le dichlorométhane à une température comprise entre 0°C et le reflux de quelques minutes à plusieurs jours. Il est également possible d'effectuer la cyclisation intramoléculaire de type Friedel-Crafts par action d'un acide de Lewis, tel que le chlorure d'aluminium ou le tétrachlorure de titane ou le trifluorure de bore, éventuellement sous forme de complexe avec l'oxyde de diéthyle, dans un solvant organique tel que le dichlorométhane ou le nitrométhane ou le nitrobenzène.

Cette réaction peut éventuellement être suivie de la saponification du produit obtenu, et suivie éventuellement selon le cas du remplacement du radical benzyle par un atome d'hydrogène.

Le produit de formule générale (VIII), sous forme d'isomères optiques, de préférence l'isomère lévogyre, peut être obtenu quant à lui de manière classique par action d'un dérivé organomagnésien de formule générale Ar-Mg-X dans laquelle Ar est défini comme précédemment et X représente un atome d'halogène, ou d'un organolithien de formule générale Ar-Li dans laquelle Ar est défini comme précédemment sur un produit de formule générale (IX) : 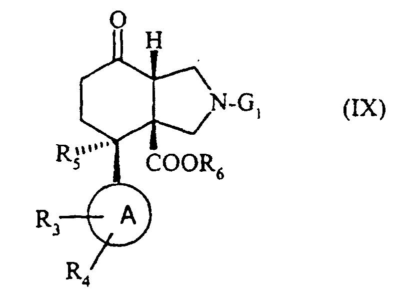 sous forme d'isomères optiques, de préférence l'isomère lévogyre, dans laquelle A, R₃, R₄, R₅, R₆ et G₁ sont définis comme précédemment, dans les conditions habituelles.

Généralement la réaction d'un arylmagnésien, obtenu classiquement et éventuellement en présence de chlorure de cérium (III) anhydre dans les conditions décrites par Imamoto (Tetrahedron Lett., 1985 p.4763), sur le dérivé cétonique de formule générale (IX) est effectuée dans un solvant organique comme l'oxyde de diéthyle ou le tétrahydrofurane, en opérant à une température comprise entre 0°C et le reflux du mélange réactionnel de quelques minutes à 24 heures. Cependant il a été trouvé particulièrement avantageux d'opérer dans le toluène, éventuellement en mélange avec de l'oxyde de diéthyle ou du tétrahydrofurane.

Généralement la réaction d'un aryllithien, obtenu classiquement, sur le dérivé cétonique de formule générale (IX) est effectuée dans un solvant organique comme l'oxyde de diéthyle ou le tétrabydrofurane, en opérant à une température comprise entre -78° et -20°C de quelques minutes à 4 heures. Cependant il a été trouvé particulièrement avantageux d'opérer dans le toluène, éventuellement en mélange avec de l'oxyde de diéthyle ou du tétrahydrofurane.

ii. Selon une deuxième voie particulièrement avantageuse, les composés de formule générale (III) sous forme d'isomères optiques, de préférence l'isomère dextrogyre peuvent être obtenus à partir des composés de formule générale (IX) sous forme d'isomères optiques, de préférence l'isomère lévogyre via la formation d'un intermédiaire stable et caractérisable de formule générale (XV) sous forme d'isomères optiques, de préférence l'isomère lévogyre caractérisé par la présence d'une fonction aryléthylénique en position 7.

Plus précisément, les composés de formule générale III 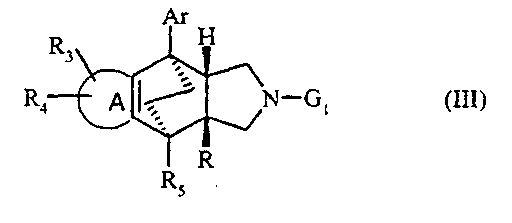 sous forme d'isomères optiques, de préférence l'isomère dextrogyre dans laquelle A, R₃, R₄, R₅ , Ar et R sont tels que définis en formule générale I et G 1 représente un radical benzyle peuvent être obtenus à partir de composés de formule générale (IX) : 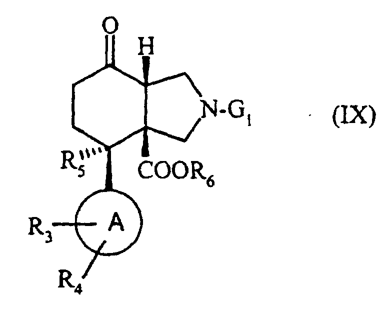 sous forme d'isomères optiques, de préférence l'isomère lévogyre dans laquelle A, R₃, R₄, R₅, R₆ et G₁ sont tels que définis précédemment via la formation d'un intermédiaire de formule générale (XV) 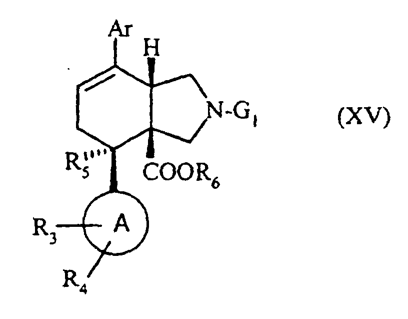 sous forme d'isomères optiques, de préférence l'isomère lévogyre dans laquelle A, R₃, R₄, R₅,R₆, Ar et G₁ sont tels que définis précédemment

Le protocole opératoire mis au point, implique successivement :
- soit la condensation, en position 7, d'un dérivé cétonique de formule générale (IX) avec de l'hydrazine, pour conduire à une hydrazone, suivie de l'action de l'iode pour conduire, selon la réaction de Barton (J. Chem. Soc. 1962 p.470), à un dérivé iodoéthylènique ; puis d'une réaction de couplage au palladium avec un acide arylboronique, réaction de Suzuki (Tetrahedron Lett. 1979 p.3437), de formule générale Ar-B(OH)₂, ou éventuellement avec l'anhydride trimérique de l'acide aryleboronique, dans laquelle Ar est défini comme précédemment, ou avec un arylstannane, réaction de Stille (Angew. Chem. Int. Ed. Engl., 1986 p.508), de formule générale Ar-SnMe₃ dans laquelle Ar est défini comme précédemment, pour conduire à cet intermédiaire aryléthylénique de formule générale (XV) dont la transformation par cyclisation intramoléculaire de type Friedel-Crafts conduit au produit (III) attendu,
- soit la réaction d'un dérivé cétonique de formule générale (IX) avec de l'anhydride trifluorométhanesulfonique, pour conduire à un triflate d'énol en position 7 (Org. Synth., 1990 p.116) ; puis d'une réaction de couplage au palladium avec un acide arylboronique, réaction de Suzuki (Tetrahedron Lett. 1979 p.3437), de formule générale Ar-B(OH)₂ dans laquelle Ar est défini comme précédemment, ou avec un arylstannane, réaction de Stille (Angew. Chem. Int. Ed. Engl., 1986 p.508), de formule générale Ar-SnMe₃ dans laquelle Ar est défini comme précédemment, pour conduire à cet intermédiaire aryléthylénique de formule générale (XV) dont la transformation par cyclisation intramoléculaire de type Friedel-Crafts conduit au produit (III) attendu.

Généralement le dérivé cétonique de formule générale (IX) est traité par un excès d'hydrate d'hydrazine, de 3 à 20 équivalents molaires, au reflux dans un solvant, tel que l'éthanol, de quelques minutes à quelques heures. L'hydrazone ainsi obtenue est alors agitée avec un excès d'iode, en présence d'une amine tertiaire aliphatique telle que la triéthylamine, à une température voisine de 20°C pendant quelques heures pour conduire à un dérivé iodoéthylénique.

Généralement le dérivé cétonique de formule générale (IX) est traité :
- soit par de l'anhydride trifluorométhanesulfonique en présence d'une base organique, telle que la 2,6-di-*tert*-butyl-4-méthylpyridine, dans un solvant organique tel que le dichlorométhane à une température voisine de l'ambiante pendant quelques heures , selon Stang (Synthesis, 1980 p283),
- soit par un bis(trifluorométhylsulfonyl)amide, comme la N,N-bis (trifluorométhylsulfonyl)aniline selon Mac Murry (Tetrahedron Lett., 1983 p979) ou la 2-[N,N-bis(trifluorométhylsulfonyl)amino]pyridine selon Comins (Tetrahedron Lett., 1992 p979), en présence d'une base comme le diisoprolylamidure de lithium dans un solvant organique comme le dichlorométhane ou le 1,2-diméthoxyéthane, pour conduire à un triflate d'énol.

Généralement le couplage entre le dérivé iodoéthylènique, ou le triflate d'énol, obtenu précédemment et un acide arylboronique, obtenu classiquement et éventuellement isolé sous forme d'anhydride trimérique, est effectué par agitation dans un système biphasique constitué d'un solvant organique, préférentiellement un mélange de toluène et de méthanol, et d'une solution aqueuse basique, préférentiellement une solution 2N de carbonate de sodium, en présence d'une quantité catalytique de dérivé de palladium(0), préférentiellement du tétrakis(triphénylphosphine)palladium, à une température voisine du reflux pendant quelques heures pour conduire au composé aryléthylènique de formule générale (XV).

Généralement le couplage entre le dérivé iodoéthylènique, ou le triflate d'énol, obtenu précédemment avec un arylestannane, obtenu classiquement, est effectué par agitation dans un solvant organique aprotique polaire, préférentiellement le diméthylformamide ou la N-méthyl-pyrrolidone, en présence d'une quantité catalytique de dérivé de palladium(0), préférentiellement du tétrakis (triphénylphosphine)palladium, à une température comprise entre 50° et 100°C pendant quelques heures pour conduire au composé aryléthylènique de formule générale (XV).

Généralement la cyclisation intramoléculaire de type Friedel-Crafts du composé de formule générale (XV) en produit de formule générale (III) est effectuée dans les conditions décrites précédemment pour la cyclisation intramoléculaire des produits de formule générale (VIII).

iii. Selon une troisième voie, un second procédé de préparation permet d'obtenir les composés de formule générale (III) sous forme d'isomères optiques, de préférence l'isomère dextrogyre tels que définis précédemment à partir des composés de formule générale (IX) sous forme d'isomères optiques, de préférence l'isomère lévogyre via la formation de l'intermédiaire de formule générale (XV) sous forme d'isomères optiques, de préférence l'isomère lévogyre éventuellement isolable. Ce second procédé est tout particulièrement avantageux lorsque le radical aryle Ar représente un noyau phényle susbtitué, en positions para ou méta-para ou méta-para-méta' par des groupements donneurs d'électrons, ou un radical hétérocyclique naturellement riche en électrons ou un radical hétérocyclique convenablement substitué par des groupements donneurs d'électrons, ce second procédé consiste à faire réagir directement, selon des réactions tandem de cyclisations intermoléculaire puis intramoléculaire de type Friedel-Crafts, un hydrocarbure aromatique ou hétérocyclique Ar-H avec un composé de formule générale (IX) dans un solvant organique en présence d'un excès d'acide fort, tel que l'acide trifluorométhanesulfonique, ou éventuellement d'un acide de Lewis, tel que le chlorure d'aluminium.

Le mode opératoire mis au point consiste à condenser le produit de formule générale (IX) avec un excès d'acide trifluorométhanesulfonique (de 5 à 20 équivalents molaires) dans un solvant organique tel que le dichlorométhane à une température voisine de l'ambiante de quelques heures à plusieurs jours. Selon le nombre d'équivalents molaires et la concentration de l'acide trifluorométhane-sulfonique ainsi que la nature du radical Ar et des substituants qu'il porte, cette réaction conduit soit directement aux composés de formule générale (III) soit intermédiairement aux composés de formule générale (XV) qui sont alors cyclisés comme décrit précédemment en composés de formule générale (III).

En outre, les composés de formule générale (I) ou (III) peuvent être obtenus par fonctionnalisation des substituants du cycle aromatique Ar des composés de formule générale (I) ou (III) correspondants, par application ou adaptation des méthodes connues de fonctionnalisations habituelles, telles que, et à titre non limitatif : les réactions de substitution fonctionnelle (par exemple le remplacement d'un atome d'halogène par un groupement cyano par un couplage au palladium), les réactions de déalkylation (par exemple par BBr₃), les réactions d'alkylation (notamment les réactions d'alkylation-cyclisation par action de BBr₂).

Un autre objet de la présente invention se rapporte également à l'utilisation des composés de formule générale (XV) dans le procédé revendiqué : 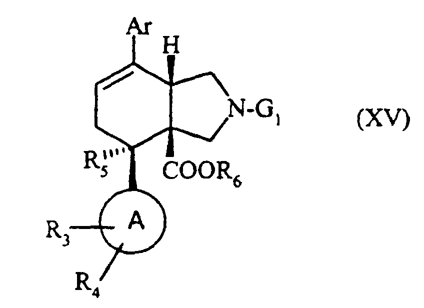 sous forme d'isomères optiques, de préférence l'isomère lévogyre dans laquelle A, R₃, R₄, R₅et Ar sont tels que définis en formule générale I, G₁ représente un radical benzyle et R₆ représente un radical alcoyle contenant 1 à 3 atomes de carbone.

Les produits de formule générale (III) 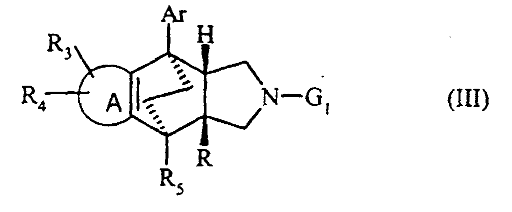 dans laquelle R représente un radical

(CH₂)ₘ-X₁(CH₂)ₙ-Z

dans lequel :
m est égal à 0,
X₁ représente une liaison,
n est égal à 0 et
Z représente un radical -COOR₆ ou -CON(R₇)(R₈)
peuvent être obtenus à partir d'un produit de formule générale (VIII) dans laquelle R représente un radical carboxy par estérification et amidification dans les conditions décrites ci-dessus, suivie d'une cyclisation par action de l'acide trifluorométhanesulfonique dans les conditions décrites ci-dessus.

Les produits de formule générale (III) dans laquelle R représente un radical

(CH₂)ₘ-X₁(CH₂)ₙ-Z

dans lequel :
m est égal à 1
X₁ représente une liaison,
n est égal à 0 et
Z représente un radical -COOR₆, -CON(R₇)(R₈) ou PO(OR₉)₂
peuvent être obtenus à partir d'un produit de formule générale : 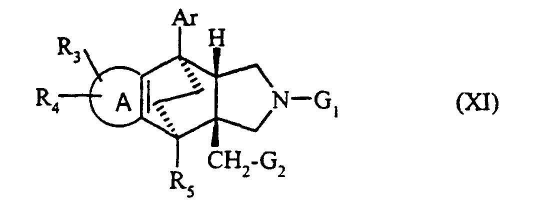 dans laquelle A, Ar, R₃, R₄ et R sont définis comme précédemment et, G₁ représente un groupement protecteur de la fonction amine (benzyle, benzyloxycarbonyle ou *tert*-butoxycarbonyle et G₂ représente un groupement partant tel qu'un radical trifluorométhylsulfonyloxy.

Plus particulièrement, pour obtenir un produit de formule générale (III) dans laquelle R représente un radical

(CH₂)ₘ-X₁(CH₂)ₙ-Z

dans lequel :
m est égal à 1,
X₁ représente une liaison,
n est égal à 0 et
Z représente un radical carboxy, -COOR₆ dans lequel R₆ représente un radical alcoyle ou -CON(R₇)(R₈),
il est particulièrement avantageux de passer par l'intermédiaire du nitrile correspondant, qui peut être obtenu par action d'un cyanure de métal alcalin sur le produit de formule générale (XI) en opérant dans un solvant organique polaire tel que le diméthylsulfoxyde à une température comprise entre 0 et 50°C, qui est hydrolysé en acide correspondant qui peut alors être estérifié ou amidifié dans les conditions habituelles.

Plus particulièrement, pour obtenir un produit de formule générale (III) dans laquelle R représente un radical

(CH₂)ₘ-X₁(CH₂)ₙ-Z

dans lequel :
m est égal à 1,
X₁ représente une liaison,
n est égal à 0 et
Z représente un radical -PO(OR₉)₂,
il est particulièrement avantageux de faire réagir un trialcoylphosphite sur un produit de formule générale (XI), puis à transformer éventuellement le phosphonate obtenu en acide phosphonique correspondant.

Les produits de formule générale (III) 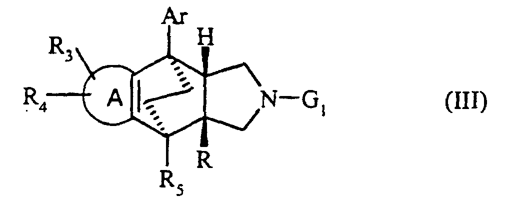 dans laquelle :
- R représente un radical (CH₂)ₘ-X₁-(CH₂)ₙ-Z avec m égal à 1, X₁ représentant un atome d'oxygène ou de soufre, n égal à 1 ou 2 et Z représentant un radical carboxy, -COOR₆ dans lequel R₆ représente un radical alcoyle ou -CON(R₇)(R₈) et
- G₁ représente un groupement protecteur de la fonction amine
peuvent être obtenus par action d'un ester ou d'un amide de formule générale :

H-X₁-(CH₂)ₙ-Z

dans laquelle X₁, n et Z sont définis comme ci-dessus sur un produit de formule générale (XI), en opérant dans un solvant organique anhydre tel que le dioxane en présence d'un hydrure de métal alcalin tel que l'hydrure de sodium, suivie éventuellement selon le cas de la saponification du produit de formule générale (III) ainsi obtenu.

Les produits de formule générale (III) dans laquelle :
- m est égal à 1,
- X₁ représente une liaison,
- n est égal à 1 avec le groupement méthylène pouvant être substitué par un radical carboxy ou alcoxycarbonyle ou carbamoyle ou alcoylcarbamoyle ou dialcoylcarbamoyle,
- G₁ représente un groupement protecteur de la fonction amine et
- Z représente un radical carboxy, -COOR₆ dans lequel R₆ représente un radical alcoyle ou -CON(R₇)(R₈),
peuvent être obtenus par action d'un acide malonique préalablement anionisé, ou d'un dérivé de l'acide malonique, de préférence un diester, sur un produit de formule générale (XI) en opérant dans un solvant organique anhydre tel que le dioxane en présence d'un hydrure de métal alcalin tel que l'hydrure de sodium, à une température comprise entre 0°C et la température de reflux du mélange réactionnel, suivie selon le cas de la saponification, de l'estérification, de l'amidification ou de la décarboxylation du produit de formule générale (III) ainsi obtenu.

Les produits de formule générale (III) dans laquelle :
R représente un radical -NH-CO-T dans lequel T est défini comme précédemment peuvent être obtenus par amidification d'un produit de formule générale (III) dans laquelle :
   - R₃ est défini comme précédemment,
   - G₁ représente un groupement protecteur de la fonction amine et
   - R représente un radical amino au moyen d'un acide de formule générale T-CO-OH dans laquelle T est défini comme précédemment dans les conditions décrites ci-dessus pour l'amidification d'un produit de formule générale (VI).

Les produits de formule générale (III) dans laquelle R représente un radical amino ou les produits de formule générale (VI) peuvent être obtenus selon les méthodes qui permettent de transformer un radical carboxy en radical amino sans toucher au reste de la molécule.

Généralement, la fonction carboxy d'un produit de formule générale (III) ou (I) est transformée en radical amino en passant par un isocyanate qui peut être obtenu par pyrolyse de l'azoture d'acide qui peut lui-même être obtenu par action d'un azoture de métal alcalin sur l'halogénure d'acide correspondant. Classiquement l'isocyanate intermédiaire ainsi obtenu est condensé avec de l'alcool benzylique, puis le carbamate de benzyle obtenu est transformé en radical amino soit par hydrogénolyse soit par hydrolyse acide dans les conditions décrites précédemment.

Les produits de formule générale (III) dans laquelle R représente
- un radical 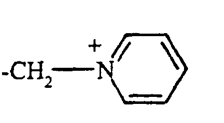 peuvent être obtenu par action dela pyridine et d'un acide fort ou d'un dérivé de cet acide sur un produit de formule générale (XII) : 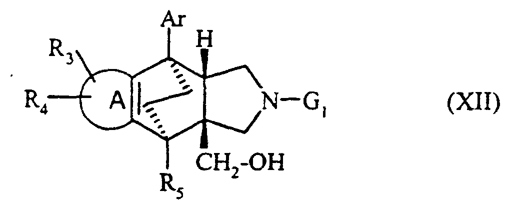 dans laquelle A, Ar, R₃, R₄ et R₅ sont définis comme précédemment et G₁ représente un groupement protecteur de la fonction amine.

Les produits de formule générale (VII) ou de formule générale (XII) peuvent être obtenus respectivement par réduction d'un produit de formule générale (I) ou d'un produit de formule générale (III) dans laquelle R représente un radical de formule générale -COOR₆ dans laquelle R₆ représente de préférence un radical alcoyle contenant 1 à 3 atomes de carbone.

Généralement, la réduction est effectuée au moyen d'un hydrure double de lithium et d'aluminium en opérant dans un solvant organique tel qu'un éther comme le tétrahydrofurane à une température comprise entre 0 et 50°C.

2. Selon une deuxième voie, les produits de formule générale (I) sous forme d'isomères optiques, de préférence dextrogyre peuvent également être obtenus à partir des produits de formule générale (IX) sous forme d'isomères optiques, de préférence l'isomère lévogyre par couplage de la chaine latérale pour former le produit de formule générale (VIII) : 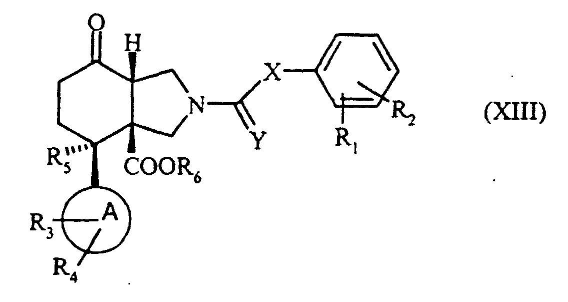 sous forme d'isomères optiques, de préférence l'isomère lévogyre dans laquelle A, R₁, R₂, R₃, R₄, R₅, R₆, X et Y sont définis comme précédemment, puis indifféremment par cyclisation du noyau cycloperhydroisoindole et éventuellement modification du substituant R :

Les produits de formule générale (I) dans laquelle A, Ar, R₁, R₂, R₃, R₄, R₅, X et Y sont définis comme précédemment et R représente un radical COOR₆ dans lequel R₆ est défini comme précédemment peuvent être également obtenus à partir d'un produit de formule générale (IX) dans laquelle R₃, R₄, R₅ et R₆ sont définis comme précédemment et G₁ représente un atome d'hydrogène.

Le produit de formule générale (IX) 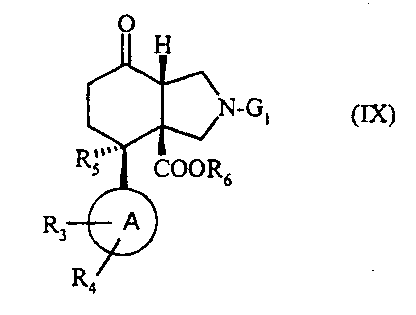 dans laquelle G₁ représente un atome d'hydrogène est obtenu à partir du produit de formule générale (IX) dans laquelle G₁ représente un groupement protecteur de la fonction amino; dans les conditions décrites précédemment pour la préparation d'un produit de formule générale (III) dans laquelle G, représente un atome d'hydrogène.

Le produit de formule générale (IX) sous forme d'isomères optiques, de préférence l'isomère lévogyre dans laquelle G₁ représente un atome d'hydrogène est transformé en produit de formule générale : 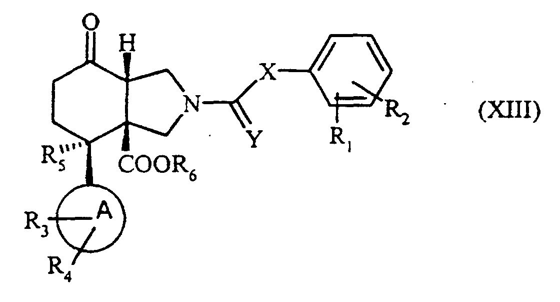 sous forme d'isomères optiques, de préférence l'isomère lévogyre dans laquelle A, R₁, R₂, R₃, R₄, R₅, R₆, X et Y sont définis comme précédemment, en opérant, selon les significations de X et Y, de la manière suivante :
- les produits de formule générale (XIII) dans laquelle Y représente un atome d'oxygène ou de soufre et X représente un groupement -CO-, méthylène, vinyldiyle , alcèn-1,1-diyle ou cycloalcan-1,1-diyle, peuvent être obtenus par action d'un acide de formule générale (II), ou de son chlorure, ou de son anhydride sur un produit de formule générale (IX) dans laquelle G₁ représente un atome d'hydrogène en opérant dans les conditions décrites précédemment pour l'action d'un produit de formule générale (II) sur un produit de formule générale (III) dans laquelle G₁ représente un atome d'hydrogène,
- les produits de formule générale (XIII) dans laquelle Y représente un atome d'oxygène ou de soufre et X représente un atome d'oxygène peuvent être obtenus par action d'un halogénoformiate ou d'un halogénothioformiate de formule générale (IV) sur un produit de formule générale (IX) dans laquelle G₁ représente un atome d'hydrogène en opérant dans les conditions décrites précédemment pour l'action d'un produit de formule générale (IV) sur un produit de formule générale (III) dans laquelle G₁ représente un atome d'hydrogène,
- les produits de formule générale (XIII) dans laquelle Y représente un atome d'oxygène ou de soufre et X représente un groupement NH peuvent être obtenus par action d'un isocyanate ou d'un isothiocyanate de formule générale (V) sur un produit de formule générale (IX) dans laquelle G, représente un atome d'hydrogène en opérant dans les conditions décrites précédemment pour l'action d'un produit de formule générale (V) sur un produit de formule générale (III) dans laquelle G₁ représente un atome d'hydrogène.

Le produit de formule générale (XIII) sous forme d'isomères optiques, de préférence l'isomère lévogyre est transformé en produit de formule générale : 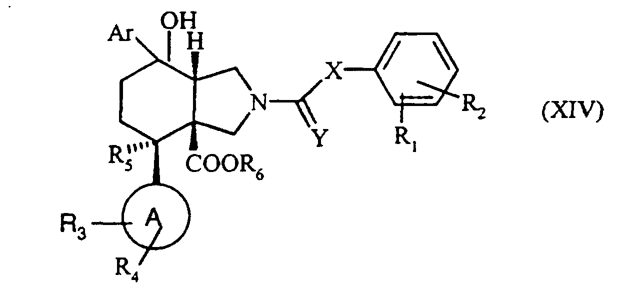 sous forme d'isomères optiques, de préférence l'isomère lévogyre dans laquelle A, Ar, R₁, R₂, R₃, R₄, R₅, R₆, X et Y sont définis comme précédemment, par action d'un dérivé métallique de formule générale Ar-Mg-X ou Ar-Li dans laquelle X représente un atome d'halogène sur un produit de formule générale (XIII) en opérant dans les conditions décrites précédemment pour l'action d'un dérivé organo-magnésien ou organo-lithien de formule générale Ar-Mg-X ou Ar-Li sur un produit de formule générale (IX).

Le produit de formule générale (XIV) sous forme d'isomères optiques, de préférence l'isomère lévogyre est transformé en produit de formule générale (I) sous forme d'isomères optiques, de préférence l'isomère dextrogyre dans laquelle A, Ar, R₁, R₂, R₃, R₄, R₅, R₆, X et Y sont définis comme précédemment, par action de l'acide trifluorométhanesulfonique, ou d'un acide de Lewis, sur le produit de formule générale (XIV) en opérant dans les conditions décrites précédemment pour l'action de l'acide trifluorométhanesulfonique, ou d'un acide de Lewis, sur un produit de formule générale (VIII).

L'action d'un hydrocarbure aromatique ou d'un hétérocycle aromatiqueAr-H, tel que défini précédemment, en présence d'acide trifluorométhanesulfonique ou d'un acide de Lewis, sur un produit de formule générale (XIII) dans laquelle R₁, R₂, R₃, R₄, R₅, R₆, X et Y sont définis comme précédemment, conduit à un produit de formule générale (I) dans laquelle Ar est défini comme précédemment et R représente un radical COOR₆ dans lequel R₆ représente un radical alcoyle de 1 à 3 atomes de carbone.

Les produits de formule générale (I) sous forme d'isomères optiques, de préférence l'isomère dextrogyre dans laquelle :
- A, Ar, R₁, R₂, R₃, R₄, R₅, X et Y sont définis comme précédemment et
- R représente un radical

   CH₂)ₘ-X₁-(CH₂)ₙ-Z

   dans laquelle m, n, X₁ et Z sont définis comme précédemment
peuvent aussi être préparés à partir d'un produit de formule générale (VII) sous forme d'isomères optiques, de préférence l'isomère dextrogyre dans les conditions décrites précédemment pour la préparation des produits de formule générale (III) à partir d'un produit de formule générale (XI), après remplacement du radical hydroxy du produit de formule générale (VII) par un groupement partant tel qu'un radical trifluorométhanesulfonyloxy.

Comme décrit dans les demandes WO 98/29390, FR2772764, les produits de formule générale (X) peuvent être obtenus par estérification des acides 3-oxo-cyclohex-1-ène-1-carboxyliques correspondants 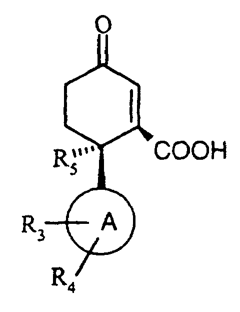 dans lesquels A, R₃, R₄ et R₅ sont définis comme ci-dessus au moyen d'un alcool aliphatique contenant 1 à 3 atomes de carbone en présence d'un acide minéral tel que l'acide chlorhydrique ou l'acide sulfurique à une température comprise entre 0°C et la température de reflux du mélange réactionnel, ou au moyen d'un halogénure d'alcoyle (iodure), en présence d'une base organique, telle que le 1,8-diazabicyclo[5.4.0]undéc-7-ène, ou minérale, telle que le carbonate de césium, en opérant dans un solvant choisi parmi le tétrahydrofurane, le diméthylformamide, l'acétone ou le dioxane.

Les acides 3-oxo-cyclohex-1-ène-1-carboxyliques, dans lesquels R₃, R₄ et R₅ sont définis comme ci-dessus, peuvent être obtenus à partir des acides 3-oxo-6-phénylcyclohexane-1-ol-1-carboxyliques 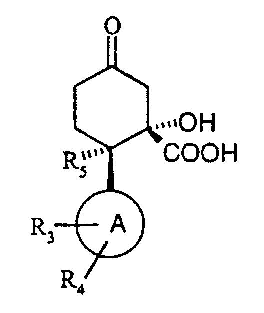 dans lesquels A, R₃, R₄ et R₅ sont définis comme ci-dessus, soit par deshydratation thermique, par chauffage à une température voisine de 190°C (selon J. Org. Chem., 1971 p.3707) ou par chauffage au reflux du toluène en présence d'acide p.toluènesulfonique, soit par action d'une base minérale telle que la soude à une température comprise entre 0 et 50°C.

Les acides 3-oxo-6-phényl-cyclohexane-1-ol-1-carboxyliques, dans lesquels R₃, R₄ et R₅ sont définis comme ci-dessus, peuvent être obtenus par action des acides pyruviques, 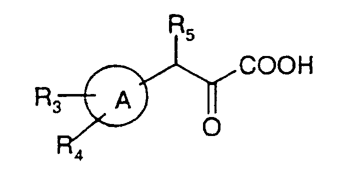 où A, R₃ , R ₄, R ₅ sont définis comme précédemment, ou éventuellement des esters correspondants, plus particulièrement dans le cas où R₅ ne représente pas un atome d'hydrogène, sur la méthylvinylcétone en opérant généralement en milieu hydroalcoolique tel qu'un mélange méthanol-eau en présence d'une base minérale telle que la soude (selon J. Org. Chem., 1971, 3707).

Les acides pyruviques peuvent être obtenus, plus particulièrement dans le cas où R₅ représente un atome d'hydrogène, soit par hydrolyse des acides α-acétamidovinyliques correspondants 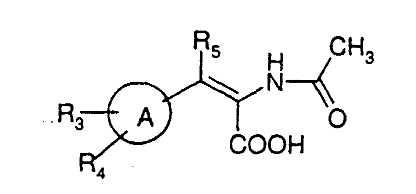 où A, R₃ , R ₄, R ₅ sont définis comme précédemment, par chauffage dans l'acide chlorhydrique selon Org. Synth. 1943 p.519, soit par hydrolyse des hydantoines correspondantes, 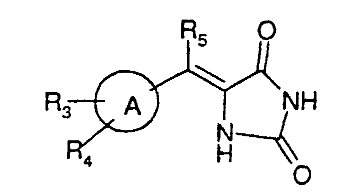 où A, R₃ , R ₄, R ₅ sont définis comme précédemment par chauffage dans la soude à 20 % selon Org. Synth. Coll. Vol. V p.627.

Les acides α-acétamidovinyliques peuvent être obtenus, à partir des aldéhydes correspondants 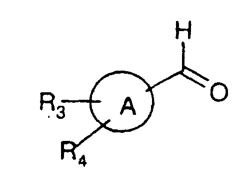 où A, R₃ , R ₄ sont définis comme précédemment selon Org. Synth. 1939 pl, par action de la N-acétylglycine au reflux de l'anhydride acétique en présence d'acétate de sodium. Les azlactones intermédiaires ainsi obtenues sont ensuite hydrolysées en acides a-acétamidocinnamiques par chauffage au reflux dans l'acétone aqueux.

Les hydantoines peuvent être obtenues par chauffage des aldéhydes correspondants, selon Org. Synth. Coll. Vol V p.267, avec de l'hydantoine en présence d'une base organique telle que la pipéridine à une température voisine de 130°C.

Les pyruvates peuvent être obtenus, plus particulièrement dans le cas où R₅ ne représente pas un atome d'hydrogène mais un radical alcoyle ou alcoylthio, à partir des acides carboxyliques, 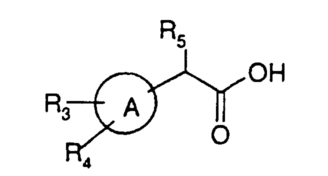 par action du dianion correspondant, obtenu généralement par action d'une base organique telle que le n-butyllithium sur l'acide, sur l'oxalate d'éthyle à une température voisine de -70°C, suivie de décarboxylation, en opérant dans les conditions décrites dans Tetrahedron Lett., 1981, 2439-42.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.
Les produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique, trifluoroacétique ou oxalique) ou avec les bases minérales (soude, potasse, lithine, chaux) ou organiques (amines tertiaires comme la triéthylamine, la pipéridine, la benzylamine) selon la nature des composés de formule générale (I).

Les exemples suivants sont présentés à titre illustratif et non limitatif de la présente invention.

### EXEMPLE 1

### Préparation de l'énantiomère dextrogyre du 4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS)

### Etape A

A une solution de 428 g (1,98 mol) d'acide 3-oxo-6-phényl-cyclohex-1-ène-1-carboxylique-(RS), qui peut être obtenu selon J. Org. Chem., 1971, 36, 3707, dans 4,5 dm³ d'acétone, on ajoute successivement 358 g (2,52 mol) d'iodure de méthyle et 361 g (2,38 mol) de 1,8-diazabicyclo[5.4.0]undéc-7-ène, puis on porte au reflux pendant cinq heures. L'acétone est ensuite distillée, puis le résidu est agité avec 2,5 dm³ d'eau. Après refroidissement à 10°C, le précipité formé est essoré, lavé à l'eau glacée, puis séché à 30°C. On obtient ainsi 423 g (93 %) de 3-oxo-6-phénylcyclohex-1-ène-1-carboxylate de méthyle-(RS), sous forme d'une poudre jaune dont les caractéristiques sont les suivantes :
- point de fusion = 66°C
- spectre de R.M.N. (300 MHz, CDCl₃, d en ppm) : 2,1-2,4 (mt, 4H: H en 4 et en 5), 3,72 (s, 3H: CH₃) ; 4,25 (dt, 1H: H en 6) ; 6,88 (s, 1H: H en 2) ; 7,2-7,52 (mt, 5H: aromatiques).

### Etape B

### Préparation du 3-oxo-6-phenyl-cyclohex-1-ène-carboxylate de méthyle-(R,S) sous forme lévogyre résolue

202 g de 3-oxo-6-phenyl-cyclohex-1-ène-carboxylate de méthyle-(R,S) sous forme racémique sont dissous sous agitation vigoureuse dans 7,75 dm³ de cyclohexane à 30 °C et les particules insolubles sont extraites par décantation (solution 1). 513.6 g de K₂HPO₄ sont dissous dans 29.43 dm³ d'eau distillée dans un réacteur agité de 50 dm³ (solution 2). La solution 2 est ajustée à pH 7.2 avec 85 % (w/w) H₃PO₄, chauffée à 30°C et mélangée avec la solution 1. 2.8 dm³ d'une solution de lipase Candida antartica " fraction B " (e.g. Chirazyme® L2, solution commercialisée par Boehringer Mannheim) est ensuite ajoutée au réacteur. La réaction est effectuée sous forte agitation à 30°C. La résolution énantiosélective est suivie par de la HPLC chirale, sur colonne : Chiralpak® AD (Daicel Chemical Industries Ltd), phase mobile : heptane/ethanol 90/10 (V/V), détection UV : 220 nm, débit : 1 mL/mn. Après 342 h la phase organique sous forme d'émulsion (Solution 3) est séparée de la phase aqueuse par décantation. 7.75 dm³ de cyclohexane et 1.25 dm³ d'éthanol sont mélangés avec la solution 3. 15.5 dm³ de la phase supérieure sont séparés de la phase inférieure contenant 2.4 dm³ d'eau et d'éthanol. La phase supérieure est concentrée et séchée sous vide à 40°C, conduisant à 53.5 g d'un solide jaune : 3-oxo-6-phényl-cyclohex-1-ène-carboxylate de méthyle-(R,S) sous forme lévogyre résolue à 93% d'excès énantiomérique, dont les caractéristiques sont les suivantes :
- pouvoir rotatoire : [α]₃₆₅²⁰ = -183,3 +/- 2,2° (c = 0,5 / méthanol).
- HPLC (colonne : Chiralpak® AD commercialisée par Daicel Chemical Industries Ltd, phase mobile : Heptane/Ethanol 90/10 (V/V), détection UV: 220 nm, débit: 1 mL/mn): - Temps de rétention: 3-oxo-6-phényl-cyclohex-1-ène-carboxylate de méthyle-(R,S) sous forme lévogyre résolue: 8.4 min

### Etape C

Une solution de 95 g (0,413 mol) de l'énantiomère lévogyre du 6-phényl-3-oxo-cyclohexène-1-carboxylate de méthyle-(R,S), d'excès énantiomérique supérieur à 90%, et de 0,47 cm³ d'acide trifluoroacétique dans 500 cm³ de dichlorométhane est portée au reflux. On ajoute alors, goutte à goutte en quinze minutes, 138.5 g (0,496 mol) de N-n.butoxyméthyl-N-triméthylsilylméthyl-benzylamine, qui peut être obtenue selon Chem. Pharm. Bull., 1985, 276, on maintient le reflux pendant dix minutes. Le milieu réactionnel est alors refroidi à 20°C. Après agitation pendant une solution aqueuse saturée d'hydrogénocarbonate de sodium, la phase organique est concentrée et l'huile jaune obtenue est purifiée par chromatographie sur gel de silice (230-400 Mesh), en éluant avec un mélange cyclohexane-acétate d'éthyle (90-10 puis 80-20 en volumes). L'huile ainsi obtenue est reprise par 645 cm³ de cyclohexane, la phase organique est lavée successivement par une solution aqueuse N d'acide méthanesulfonique puis de l'eau distillée. On obtient alors 118,2 g (79%) de l'énantiomère lévogyre du 2-benzyl-7-oxo-4-phényl-perhydroisoindole-3a-carboxylate de méthyle-(3aRS,4RS,7aSR), sous forme d'une huile viqueuse jaune, dont les caractéristiques sont les suivantes :
- spectre de masse (IE) : M/Z = 363 (M⁺)
- pouvoir rotatoire : [α]₃₆₅²⁰= -58,7 +/- 1° (c = 0,5 / méthanol)

### Etape D

76,2 g (0,445 mol) de 4-bromotoluène et 10,8 g (0,445 mol) de magnésium en tournures dans 500 cm³ d'oxyde de diéthyle sont chauffés au reflux pendant une heure. Après refroidissement à une température voisine de 5°C, une solution de 124,65 g (0,343 mol) de l'énantiomère lévogyre 2-benzyl-7-oxo-4-phényl-perhydroisoindole-3a-carboxylate de méthyle-(3aRS,4SR,7aRS) dans 500 cm³ de toluène est additionnée. Le mélange réactionnel est agité pendant une heure à une température voisine de 20°C puis hydrolysé par 450 cm³ d'une solution aqueuse saturée en chlorure d'ammonium. La phase aqueuse est séparée par décantation et extraite par deux fois 500 cm³ d'acétate d'éthyle. Les phases organiques sont réunies, lavées successivement par 100 cm³ d'eau distillée et 100 cm³ d'une solution aqueuse saturée en chlorure de sodium, séchées sur sulfate de magnésium et concentrées sous pression réduite. Après purification par flash-chromatographie sur gel de silice (230-400 mesh), en éluant avec des mélanges cyclohexane-acétate d'éthyle (95-5 puis 80-20 en volumes), on obtient 135,9 g (87%) de l'énantiomère lévogyre du 2-benzyl-7-hydroxy-7-(4-méthylphényl)-4-phényl-perhydroisoindole-3a-carboxylate de méthyle-(3aRS,4SR,7RS,7aRS) sous forme de solide blanc dont les caractéristiques sont les suivantes :
- pouvoir rotatoire : [α]₃₆₅²⁰= -60,3 +/- 1° (c = 0,5 / méthanol)
- spectre de R.M.N. ¹H (250 MHz, CDCl₃, d en ppm). ; 1,84 et 2,60 (d mt et mt, J = 12,5 Hz, 1H chacun : CH₂ en 5) ; de 2,05 à 2,20 (mt, 2H : 1H du CH₂ en 1 et 1H du CH₂en 6) ; 2,32 (s, 3H : ArCH₃) ; 2,40 et 2,95 (2d, J = 10,5 Hz, 1H chacun : CH₂ en 3) ; 2,45 (mt, 1H : l'autre H du CH₂ en 6) ; 2,64 (mt, 1H : l'autre H du CH₂ en 1) ; 2,85 (mt, 1H : H en 7a) ; 3,32 (s, 3H : COOCH₃) ; 3,40 et 3,70 (2d, J = 12,5 Hz, 1H chacun : NCH₂Ar) ; 3,50 (dd, J = 12,5 et 3 Hz, 1H : H en 4) ; 6,68 (s; 1H : OH en 7) ; de 7,00 à 7,50 (mt, 10H : H aromatiques en 4 et H aromatiques du benzyle) ; 7,12 et 7,40 (2d, J = 8 Hz, 2H chacun : H en ortho et méta de l'aromatique en 7).

### Etape E

A une solution de 135,9 g (0,335 mol) de l'énantiomère lévogyre de 2-benzyl-7-hydroxy-7-(4-méthylphényl)-4-phényl-perhydroisoindole-3a-carboxylate de méthyle-(3aRS,4SR,7RS,7aRS) dans 1 dm³ de dichlorométhane, maintenue à une température voisine de 0°C, on ajoute goutte à goutte 250 cm³ d'acide trifluorométhanesulfonique. Le mélange réactionnel est agité pendant 30 minutes à une température voisine de 0°C, 2 heures à une température voisine de 20°C puis refroidi à une température voisine de 0°C. On ajoute alors 200 cm³ d'eau distillée puis le pH de la phase aqueuse est amené entre 8 et 9 par addition de 450 cm³ d'une solution aqueuse 4N d'hydroxyde de sodium. La phase organique est séparée par décantation, lavée successivement par 100 cm³ d'eau distillée et 100 cm³ d'une solution aqueuse saturée en chlorure de sodium, séchée sur sulfate de magnésium et concentrée sous pression réduite. On obtient ainsi 94,12 g (72%) de l'énantiomère dextrogyre de 2-benzyl-4,9-éthano-9-(4-méthylphényl)-2,3,3a,4,9,9a-hexahydro-1H-benzo[f] isoindole-3a-carboxylate de méthyle-(3aRS,4SR,9SR,9aRS) sous forme de solide blanc dont les caractéristiques sont les suivantes :
- pouvoir rotatoire : [α]₃₆₅²⁰= +38,2 +/- 1° (c = 0,5 / méthanol)
- spectre de R.M.N. ¹H (300 MHz, CDCl3, d en ppm) : 1,61 - 1,90 et 2,67 (3 mts, respectivement 1H -1H et 2H : CH₂CH₂) ; de 2,50 à 2,60 (mt, 2H : 1H du CH₂ en 1 et 1H CH₂ en 3) ; 2,57 (s, 3H : ArCH₃) ; 2,95 (d, J = 10 Hz, l'autre H du CH₂ en 1) ; 3,37 (d, J = 10 Hz, l'autre H du CH₂ en 3) ; 3,44 (d large, J = 10 Hz, 1H : H en 9a) ; 3,54 et 3,85 (2d, J = 12,5 Hz, 1H chacun : NCH₂Ar) ; 3,57 (s large, 1H : H en 4) ; 3,72 (s, 3H : COOCH₃) ; 6,70 (d large, J = 7,5 Hz, 1H : H en 8) ; de 7,10 à 7,60 (mt, 12H : H en 5 - H en 6 - H en 7 - H en ortho et méta de l'aromatique en 9 et H aromatiques du benzyle).

### Etape F

93,62 g (0,214 mol) de l'énantiomère dextrogyre 2-benzyl-4,9-éthano-9-(4-méthylphényl)-2,3,3a,4,9,9a-hexahydro-1H-benzo[f]isoindole-3a-carboxylate de méthyle-(3aRS,4SR,9SR,9aRS), en solution dans 1,5 dm³ de méthanol, sont réduits par 41 g de formiate d'ammonium en présence de 9,4 g de palladium sur charbon à 10 % (p/p) en chauffant au reflux pendant deux heures. Après refroidissement, le catalyseur est séparé par filtration, rincé par trois fois 100 cm³ de méthanol et le filtrat concentré sous pression réduite. Le résidu est dissout dans 500 cm³ d'acétate d'éthyle et la phase organique lavée successivement par 2 fois 100 cm³ d'une solution aqueuse saturée en hydrogénocarbonate de sodium, 100 cm³ d'eau distillée et 100 cm³ d'une solution aqueuse saturée en chlorure de sodium, séchée sur sulfate de magnésium et concentrée sous pression réduite. On obtient 68,19 g de l'énantiomère dextrogyre de 4,9-éthano-9-(4-méthylphényl)-2,3,3a,4,9,9a-hexahydro-1H-benzo[f]isoindole-3a-carboxylate de méthyle-(3aRS,4SR,9SR,9aRS) sous forme de solide blanc dont les caractéristiques sont les suivantes :
- pouvoir rotatoire : [α]₃₆₅²⁰= +44,1 +/- 0,9° (c = 0,5 / méthanol)
- spectre de R.M.N. ¹H (250 MHz, CDCl₃, d en ppm) : 1,56 - 1,78 et 2,24 (3 mts, respectivement 1H-1H et 2H : CH₂CH₂) ; 2,42 (s, 3H : ArCH₃) ; 2,79 et 2,96 (2 dd, respectivement J = 12,5 et 5,5 Hz et J = 12,5 et 8 Hz, 1H chacun : CH₂ en 1) ; 3,10 et 3,39 (2 d, J = 12,5 Hz, 1H chacun : CH₂ en 3) ; 3,30 (mt, 1H : H en 9a) ; 3,51 (s large, 1H : H en 4) ; 3,60 (s, 3H : COOCH₃) ; 6,56 (d large, J = 7,5 Hz, 1H : H en 8); de 6,95 à 7,45 (mt, 7H : H en 5 - H en 6 - H en 7 et H en ortho et méta de l'aromatique en 9).

### Etape G

A une solution de 38,6 g (0,216 mol) d'acide 2-(2-méthoxyphényl)propènoïque, qui peut être obtenu selon WO 98/29390, dans 300 cm³ de dichlorométhane et 1,8 cm³ de N,N-diméthylformamide, on ajoute goutte à goutte une solution de 18,6 cm³ de chlorure d'oxalyle dans 18 cm³ de dichlorométhane. Le mélange réactionnel est encore agité pendant deux heures à une température voisine de 20°C puis refroidi à une température voisine de 0°C et coulé goutte à goutte dans une solution de 68,09 g (0,196 mol) de l'énantiomère dextrogyre de 4,9-éthano-9-(4-méthylphényl)-2,3,3a,4,9,9a-hexahydro-1H-benzo[f]isoindole-3a-carboxylate de méthyle-(3aRS, 4SR,9SR,9aRS) dans 500 cm³ de dichlorométhane et 55,4 cm³ de triéthylamine en maintenant la température au voisinage de 0°C. Le mélange réactionnel est encore agité pendant une heure à une température voisine de 0°C puis pendant une nuit à une température voisine de 20°C et versé dans 350 cm³ d'eau distillée. La phase organique est séparée par décantation, lavée par 450 cm³ d'une solution aqueuse N d'acide chlorhydrique puis 200 cm³ d'une solution aqueuse saturée en chlorure de sodium, séchée sur sulfate de magnésium et concentrée sous pression réduite. Après purification par flash-chromatographie sur gel de silice (230-400 mesh), en éluant avec un mélange cyclohexane-acétate d'éthyle (70-30 en volumes), on obtient 93,96 g (94%) de l'énantiomère dextrogyre de 4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS) sous forme de solide blanc dont les caractéristiques sont les suivantes :
- pouvoir rotatoire : [α]₃₆₅²⁰ = +60,8 +/- 1 ° (c = 0,5 / méthanol)
- spectre de R.M.N. ¹H (250 MHz, DMSO d6, à une température de 383 K, d en ppm) : 1,44 - 1,68 et de 2,00 à 2,30 (3 mts, respectivement 1H - 1H et 2H : CH₂CH₂) ; 2,40 (s, 3H : ArCH₃) ; de 3,35 à 3,50 (mt, 3H : CH₂ en 1 et H en 9a) ; 3,46 (mt, 1H : H en 4) ; 3,55 (s, 3H : COOCH₃) ; 3,60 et 4,10 (2d, J = 12,5 Hz, 1H chacun : CH₂ en 3) ; 3,73 (s, 3H : ArOCH₃) ; 5,54 et 5,70 (2s, 1H chacun : =CH₂) ; 6,46 (d large, J = 7,5 Hz, 1H : H en 8) ; de 6,90 à 7,40 (mt, 11H : H en 5 - H en 6 - H en 7 - H en ortho et méta de l'aromatique en 9 et H aromatiques en ortho - méta et para du OCH₃).

### EXEMPLE 2

### Préparation de l'énantiomère dextrogyre de l'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)

93,46 g (0,184 mol) de l'énantiomère dextrogyre de 4,9-éthano-2-[2-(2-méthoxyphényl)propènoyl]-9-(4-méthyl-phényl)-2,3,3a,4,9,9a-hexahydro-1H-benzo[f]isoindole-3a-carboxylate de méthyle-(3aRS,4SR,9SR,9aRS) sont chauffés à reflux, pendant trois heures, dans 1,6 dm³ d'éthanol en présence de 370 cm³ d'une solution aqueuse normale d'hydroxyde de sodium. Le mélange réactionnel est ensuite concentré sous pression réduite et le résidu dissout dans 500 cm³ d'eau distillée. La phase aqueuse est lavée par trois fois 250 cm³ d'oxyde de diéthyle puis acidifiée par 400 cm³ d'une solution aqueuse N d'acide chlorhydrique jusqu'à un pH voisin de 2. La phase aqueuse est extraite par 1 dm³ puis deux fois 200 cm³ de dichlorométhane, les phases organiques jointes sont lavées par trois fois 200 cm³ d'eau, séchées sur sulfate de magnésium puis concentrées sous pression réduite. Après purification par flash-chromatographie sur gel de silice (230-400 mesh), en éluant avec un mélange dichlorométhane-méthanol (97,5-2,5 en volumes), puis par deux recristallisations successives dans 850 puis 740 cm³ d'isopropanol, on obtient 46,11 g de l'énantiomère dextrogyre d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS), de pureté énantiomérique supérieure à 99,9%, sous forme de solide blanc dont les caractéristiques sont les suivantes :
- point de fusion = 233°C
- spectre de masse (IE) : M/Z= 493 (M⁺) et 449 (M⁺ - CO₂)
- pouvoir rotatoire: [α]₃₆₅²⁰ = +71,3 +/- 1,2° (c = 0,5/ méthanol).
- HPLC :

Phase stationnaire : silice chirale porteuse de greffons (3,5-dinitrobenzoyl)-R-phénylalanine décrite dans WO 98/29390,

Phase mobile : mélange de dichlorométhane/éthanol/n-heptane/acide trifluoroacétique (50/1/50/0,1 en volumes) à un débit de 2 cm³/mn

Temps de rétention : 9,53 mn

### EXEMPLE 3

### Préparation de l'acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS), énantiomère dextrogyre

On opère selon les étapes A, B, C de l'exemple 1, puis comme décrit ci-dessous :

### Etape D

Une solution de 100 g (0,275 mol) de l'énantiomère lévogyre du 2-benzyl-4-phényl-7-oxo-perhydroisoindole-3a-carboxylate de méthyle-(3aRS,4RS,7aSR) et de 50 g (1 mol) d'hydrate d'hydrazine dans 750 cm³ d'éthanol est portée au reflux pendant une heure et demie. Après concentration de l'éthanol sous pression réduite, le résidu est repris par 400 cm³ de dichlorométhane, et la phase organique est lavée avec de l'eau distillée puis avec une solution aqueuse saturée de chlorure de sodium, séchée sur sulfate de magnésium et concentrée sous pression réduite. On obtient ainsi 100 g (96%) de l'énantiomère lévogyre du 2-benzyl-7-hydrazono-4-phényl-perhydro-isoindole-3a-carboxylate de méthyle-(3aRS,4SR,7aRS) sous forme d'une huile jaune utilisée telle quelle à l'étape suivante.

### Etape E

A une solution de 100 g (0,265 mol) de l'énantiomère lévogyre du 2-benzyl-7-hydrazono-4-phényl-perhydroisoindole-3a-carboxylate de méthyle-(3aRS,4SR,7aRS) et de 110 cm³ (0,795 mol) de triéthylamine dans 1,4 dm³ de tétrahydrofurane, on ajoute goutte à goutte une solution de 134,5 g (0,53 mol) d'iode dans 600 cm³ de tétrahydrofurane. Après une heure d'agitation à température ambiante, on cencentre sous pression réduite le milieu réactionnel à environ 500 cm³ puis on ajoute 1 dm³ d'acétate d'éthyle. La phase organique est lavée avec deux fois 800 cm³ d'une solution aqueuse saturée en hydrogénocarbonate de sodium puis avec deux fois 500 cm³ d'une solution aqueuse saturée en thiosulfate de sodium, séchée sur sulfate de magnésium et concentrée sous pression réduite. Après purification par chromatographie sur gel de silice (230-400 Mesh), en éluant avec un mélange cyclohexane-acétate d'éthyle (95-5 en volumes), on obtient 56 g (45%) de l'énantiomère lévogyre du 2-benzyl-7-iodo-4-phényl-1,2,3,4,5,7a-hexahydroisoindole-3a-carboxylate de méthyle-(3aRS,4SR,7aRS) sous forme d'une huile visqueuse brune dont les caractéristiques sont les suivantes :
- spectre de masse (IE) : M/Z = 473 (M⁺)
- pouvoir rotatoire : [α]₃₆₅²⁰= -51,8 +/- 1° (c = 0,5 / méthanol)

### Etape F

On ajoute, goutte à goutte à -40°C, 10,43 cm³ (69 mmol) de N,N,N',N'-tétraméthyléthylènediamine à une solution de 13,41 g (63 mmol) de 5-bromobenzothiophène, qui peut être obtenu selon J. Het Chem. 1988, 25, 1271-2, dans 100 cm³ de tétrahydrofurane. Après quinze minutes d'agitation à - 40°C, on ajoute goutte à goutte, 43,3 cm³ d'une solution 1,6M (0,069 mol) de n-butyllithium dans l'hexane et on agite deux heures entre - 30 et - 40°C. On ajoute ensuite à -30°C, 7,86 cm³ (69 mmol) de triméthylborate et on maintient l'agitation pendant deux heures tout en laissant la température revenir entre 0 et - 5°C. La solution rouge obtenue est hydrolysée par addition de 80 cm³ d'une solution aqueuse 5N d'acide chlorhydrique, puis on ajoute 200 cm³ d'acétate d'éthyle. La phase organique est décantée, lavée par une solution aqueuse saturée de chlorure de sodium, séchée sur sulfate de magnésium et concentrée sous pression réduite. On obtient ainsi 9,3 g (76 %) d'une poudre jaune contenant très majoritairement l'acide benzothiophène-2-boronique sous forme d'anydride trimérique, utilisé tel quel dans la suite de la synthèse, dont les caractéristiques sont les suivantes :
- spectre de masse (IE) : M/Z = 480 (M⁺)
- point de fusion = 260-63°C.

### Etape G

A une solution de 22,36 g (47 mmol) de l'énantiomère lévogyre du 2-benzyl-7-iodo-4-phényl-1,2,3,4,5,7a-hexahydroisoindole-3a-carboxylate de méthyle-(3aRS,4SR,7aRS) et de 6,03 g (5,2 mmol) de tétrakis(triphénylphosphine)palladium dans 250 cm³ de 1,2-diméthoxyéthane, on ajoute successivement une solution de 9,3 g (17,3 mmol) d'anhydride trimérique de l'acide benzothiophène-2-boronique dans 100 cm³ de méthanol et 400 cm³ d'une solution aqueuse 2 N de carbonate de sodium, puis on porte au reflux pendant deux heures, et on maintient l'agitation pendant la nuit à température ambiante. Après retour à température voisine de 20°C, le mélange réactionnel est extrait avec 800 cm³ d'acétate d'éthyle, puis la phase organique est lavée avec une solution aqueuse saturée de chlorure de sodium, séchée sur sulfate de magnésium et concentrée sous pression réduite. Le résidu marron obtenu est purifié par flash-chromatographie sur gel de silice (230-400 mesh), en éluant avec un mélange cyclohexane-acétate d'éthyle (80-20 en volumes). On obtient ainsi 11,04 g (50 %) de l'énantiomère lévogyre du 2-benzyl-4-phényl-7-(2-benzothiényl)-1,2,3,4,5,7a-hexahydroisoindole-3a-carboxylate de méthyle-(3aRS,4SR,7aRS) sous forme d'une meringue jaune dont les caractéristiques sont les suivantes :
- spectre de masse (IE) : M/Z = 479 (M⁺)
- spectre de R.M.N. ¹H (400 MHz, CDCl₃, d en ppm) : 2,53 (dt, J = 18 et 5 Hz : 1H) ; de 2,80 à 2,95 (mt : 1H) ; 2,84 (AB, J = 10 Hz : 2H) ; 3,17 (t, J = 10 Hz : 1H) ; 3,48 (dd, J = 9,5 et 5 Hz : 1H) ; de 3,55 à 3,70 (mt : 4H) ; 3,56 (s : 3H) ; 6,44 (mt : 1H) ; de 7,05 à 7,40 (mt : 13H) ; 6,67 (dd, J = 6,5 et 2 Hz : 1H) ; 7,75 (d large, J = 7Hz : 1H).
- pouvoir rotatoire : [α]₃₆₅²⁰ = -28,1 +/- 0,7° (c = 0,5 / dichlorométhane)

### Etape H

On opère comme à l'étape E de l'exemple 1, mais à partir de 11 g (23 mmol) de l'énantiomère lévogyre du 2-benzyl-4-phényl-7-(2-benzothiényl)-1,2,3,4,5,7a-hexahydroisoindole-3a-carboxylate de méthyle-(3aRS,4SR,7aRS) dans 100 cm³ de dichlorométhane et de 15 cm³ d'acide trifluorométhanesulfonique. On obtient ainsi, après lavage à l'oxyde de diisopropyle, 7,96 g (72%) de l'énantiomère dextrogyre du 9-(2-benzothiényl)-2-benzyl-4,9-éthano-2,3,3a,4,9,9a-hexahydro-1H-benzo[f]isoindole-3a-carboxylate de méthyle-(3aRS,4SR,9SR,9aRS) sous forme d'une huile jaune visqueuse, utilisée telle quelle dans la suite de la synthèse, dont les caractéristiques sont les suivantes :
- spectre de masse (IE) : M/Z = 479 (M⁺)
- pouvoir rotatoire : [α]₃₆₅²⁰= +22,7 +/- 0,7° (c = 0,5 / dichlorométhane).

### Etape I

Une solution de 4,80 g (10 mmol) de l'énantiomère dextrogyre du 9-(2-benzothiényl)-2-benzyl-4,9-éthano-2,3,3a,4,9,9a-hexahydro-1H-benzo[f]isoindole-3a-carboxylate de méthyle-(3aRS,4SR,9SR,9aRS) et de 3,37 cm³ (50 mmol) de chloroformiate de vinyle dans 100 cm³ de dichlorométhane est agitée pendant une nuit à température ambiante.

Après concentration sous pression réduite, le résidu est dissout dans 60 cm³ d'une solution N d'acide chlorhydrique dans le méthanol et porté à reflux pendant deux heures. Après refroidissement, on ajoute 100 cm³ d'eau distillée, on amène à pH= 8 par addition d'une solution aqueuse N d'hydroxyde de sodium et on extrait à l'acétate d'éthyle. La phase organique est séchée sur sulfate de sodium puis concentrée sous pression réduite, on obtient alors 3,84 g ( 99%) de l'énantiomère dextrogyre du 9-(2-benzothiényl)-4,9-éthano-2,3,3a,4,9,9a-hexahydro-1H-benzo[f]isoindole-3a-carboxylate de méthyle-(3aRS,4SR,9SR,9aRS), sous forme d'une meringue blanche utilisée telle quelle dans la suite de la synthèse, dont les caractéristiques sont les suivantes :
- spectre de masse (IE) : M/Z = 389 (M⁺)
- pouvoir rotatoire : [α]₃₆₅²⁰= +21,5 +/- 0,5° (c = 0,5 / dichlorométhane).

### Etape J

On opère comme à l'étape G de l'exemple 1, mais à partir de 1,93 g (11 mmol) d'acide 2-(2-méthoxyphényl)propènoïque dans 50 cm³ de dichlorométhane contenant 1 cm³ de N,N-diméthylformamide, de 0,96 cm³ (11 mmol) de chlorure d'oxalyle dans 5 cm³ de dichlorométhane, et de 3,84 g (9,9 mmol) de l'énantiomère dextrogyre du 9-(2-benzothiényl)-4,9-éthano-2,3,3a,4,9,9a-hexahydro-1H-benzo[f]isoindole-3a-carboxylate de méthyle-(3aRS,4SR,9SR,9aRS) dans 50 cm³ de dichlorométhane et 3,39 cm³ (24 mmol) de triéthylamine. On obtient ainsi, après purification par flash-chromatographie sur gel de silice (230-400 mesh), en éluant avec un mélange cyclohexane-acétate d'éthyle (60-40 en volumes), 3,26 g (60%) de l'énantiomère dextrogyre du 9-(2-benzothiényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une meringue blanche dont les caractéristiques sont les suivantes :
- spectre de masse (IE) : M/Z = 549 (M⁺)
- pouvoir rotatoire : [α]₃₆₅²⁰= +43,2 +/- 0,8° (c = 0,5 / dichlorométhane).

### Etape K

On opère comme à l'exemple 2, mais à partir de 4,49 g (8 mmol) de l'énantiomère dextrogyre du 9-(2-benzothiényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) au reflux pendant deux heures dans 100 cm³ d'éthanol en présence de 20 cm³ d'une solution aqueuse N d'hydroxyde de sodium. On obtient ainsi, après deux recristallisations successives dans de l'éthanol aqueux à 50 % puis dans un mélange d'hexane et d'isopropanol (50-50 en volumes), 2,55 g (58 %) de l'énantiomère dextrogyre de l'acide 9-(2-berizothiényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme de cristaux blancs dont les caractéristiques sont les suivantes :
- point de fusion = 170°C
- spectre de masse (IE) : M/Z = 535 (M⁺)
- spectre de RMN¹ H (250 MHz, (CD₃)₂SO d6, à une température de 373 K, d en ppm) : 1,40 (mt : 1H) ; 1,64 (mt : 1H) ; 1,98 (mt : 1H) ; 2,13 (mt : 1H) ; 3,24 (mt : 3H) ; 3,46 (t large, J = 2,5 Hz : 1H) ; 3,59 (d, J = 13 Hz : 1H) ; 3,72 (s : 3H) ; 4,07 (d large, J = 13 Hz : 1H) ; 5,54 (s : 1H) ; 5,68 (s : 1H) ; 6,40 (d large, J = 7,5 Hz : 1H) ; de 6,90 à 7,45 (mt : 9H) ; 7,52 (d large, J = 8,5 Hz : 2H).
- pouvoir rotatoire : [α]₃₆₅²⁰ = +43,5 +/- 1 ° (c = 0,5 / dichlorométhane).

### EXEMPLE 4

### Préparation de l'énantiomère dextrogyre de l'acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS).

On opère selon les étapes A, B, C de l'exemple 1, puis D, E, F de l'exemple 3, puis comme décrit ci-dessous :

### Etape A

On opère comme à l'étape G de l'exemple 3, mais à partir de 22,5 g (47,6 mmol) de l'énantiomère lévogyre du 2-benzyl-7-iodo-4-phényl-1,2,3,4,5,7a-hexahydroisoindole-3a-carboxylate de méthyle-(3aRS,4SR,7aRS) et de 2,43 g (2,1 mmol) de tétrakis(triphénylphosphine)palladium dans 400 cm³ de toluène, puis de 10g (52,4 mmol) d'acide 2,4-dichlorophénylboronique dans 200 cm³ de méthanol et de 400 cm³ d'une solution aqueuse 2 N de carbonate de sodium, au reflux pendant deux heures. On obtient, après purification par flash-chromatographie sur gel de silice (230-400 mesh), en éluant avec un mélange cyclohexane-acétate d'éthyle (90-10 en volumes), 20,3 g (87 %) de l'énantiomère lévogyre du 2-benzyl-4-phényl-7-(2,4-dichlorophényl)-1,2,3,4,5,7a-hexahydroisoindole-3a-carboxylate de méthyle-(3aRS,4SR,7aRS), sous forme d'une huile jaune très visqueuse dont les caractéristiques sont les suivantes :
- spectre de masse (IE) : M/Z = 492 (M⁺)
- spectre de RMN
- pouvoir rotatoire : [α]₃₆₅²⁰= -41,6 +/-1° (c = 0,5 / méthanol)

### Etape B

On opère comme à l'étape E de l'exemple 1, mais à partir de 20,15 g (40,9 mmol) de l'énantiomère lévogyre du 2-benzyl-4-phényl-7-(2,4-dichlorophényl)-1,2,3,4,5,7a-hexahydroisoindole-3a-carboxylate de méthyle-(3aRS,4SR,7aRS) dans 30 cm³ de dichlorométhane et 73 cm³ (0,82 mol) d'acide trifluorométhanesulfonique pendant quatre heures au reflux. On obtient, après purification par flash-chromatographie sur gel de silice (230-400 mesh), en éluant avec un mélange cyclohexane-acétate d'éthyle (60-40 en volumes), 8,8 g (44%) de l'énantiomère dextrogyre du 2-benzyl-9-(2,4-dichlorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue blanche, dont les caractéristiques sont les suivantes :
- spectre de masse (IE) : M/Z = 492 (M⁺)
- pouvoir rotatoire : [α]₃₆₅²⁰ = +86,5 +/- 1,5° (c = 0,5 / dichlorométhane).

### Etape C

On opère comme à l'étape 1 de l'exemple 3, mais à partir de 8,8 g (17,9 mmol) de l'énantiomère dextrogyre du 2-benzyl-9-(2,4-dichlorophényl)-4,9-éthano-2,3,3a,4,9,9a-hexahydro-1H-benzo[f]isoindole-3a-carboxylate de méthyle-(3aRS,4SR,9SR,9aRS) et de 2,9 cm³ (44,8 mmol) de chloroformiate de vinyle dans 150 cm³ de dichlorométhane pendant vingt-quatre heures à température ambiante, puis en reprenant le concentrat au reflux pendant huit heures dans 180 cm³ d'une solution 4N d'acide chlorhydrique dans le dioxane. On obtient 5,44 g (66 %) de l'énantiomère dextrogyre du 9-(2,4-dichlorophényl)-4,9-éthano-2,3,3a,4,9,9a-hexahydro-1H-benzo[f]isoindole-3a-carboxylate de méthyle-(3aRS,4SR,9SR,9aRS), sous forme d'une meringue jaune pâle utilisée telle quelle dans la suite de la synthèse, dont les caractéristiques sont les suivantes :
- spectre de masse (IE) : M/Z = 438 (M⁺)
- pouvoir rotatoire : [α]₃₆₅²⁰= +103 +/- 1,7° (c = 0,5 / dichlorométhane).

### Etape D

On opère comme à l'étape G de l'exemple 1, mais à partir de 2,4 g (13,5 mmol) d'acide 2-(2-méthoxyphényl)propènoïque et de 1,2 cm³ (13,7 mmol) de chlorure d'oxalyle dans 25 cm³ de dichlorométhane contenant 1 cm³ de N,N-diméthylformamide, puis de 5,44 g (12,3 mmol) de l'énantiomère dextrogyre du 9-(2,4-dichlorophényl)-4,9-éthano-2,3,3a,4,9,9a-hexahydro-1H-benzo[f]isoindole-3a-carboxylate de méthyle-(3aRS,4SR,9SR,9aRS) dans 50 cm³ de dichlorométhane et 4,2 cm³ (30 mmol) de triéthylamine pendant dix-huit heures à température ambiante.

On obtient, après purification par flash-chromatographie sur gel de silice (230-400 mesh) en éluant avec un mélange cyclohexane-acétate d'éthyle (60-40 en volumes), 5,4 g (67 %) de l'énantiomère dextrogyre du 9-(2,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une meringue blanche dont les caractéristiques sont les suivantes :
- spectre de masse (IE) : M/Z = 562 (M⁺)
- pouvoir rotatoire : [α]₃₆₅²⁰= +106,3 +/- 1,7° (c = 0,5 / méthanol).

### Etape E

On opère comme à l'exemple 2, mais à partir de 5,4 g (9,6 mmol) de l'énantiomère dextrogyre du 9-(2,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), au reflux pendant trois heures et demie dans 130 cm³ d'éthanol et 20 cm³ d'une solution aqueuse normale d'hydroxyde de sodium. On obtient, après purification par flash-chromatographie sur gel de silice (230-400 mesh) en éluant avec un mélange acétate d'éthyle-acide acétique (99-1 en volumes) puis recristallisation dans l'acétate d'isopropyle 1,94 g (36%) de l'énantiomère dextrogyre de l'acide 9-(2,4-dichlorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme de cristaux blancs dont les caractéristiques sont les suivantes :
- point de fusion = 194°C
- spectre de masse (IE) : M/Z = 548 (M⁺)
- pouvoir rotatoire : [α]₃₆₅²⁰= +126,6 +/- 1,8° (c = 0,5 / méthanol).

### EXEMPLE 5

### Préparation de l'énantiomère dextrogyre de l'acide 9-(3,4-dichlorophényl)-4,9-éthano-2-[2-(2-méthoxyphényl) propènoyl]-2,3,3a,4,9,9a-hexahydro-1H-benzo [f] isoindole-3a-carboxylique-(3aRS,4SR,9SR,9aRS)

On opère selon les étapes A, B, C de l'exemple 1, puis comme décrit ci-dessous :

### Etape A

On opère comme à l'étape D de l'exemple 1, mais à partir de 9,95 g (44 mmol) de 3,4-dichloro-bromobenzène dans 120 cm³ d'oxyde de diéthyle et de 1,07 g (44 mmol) de magnésium au reflux pendant 30 minutes puis de 8 g (22 mmol) de l'énantiomère lévogyre du 2-benzyl-7-oxo-4-phényloctahydroisoindole-3a-carboxylate de méthyle-(3aRS,4SR,7aRS) dans 120 cm³ d'oxyde de diéthyle pendant dix minutes à une température voisine de 10°C. On obtient, après purification par flash-chromatographie sur gel de silice (230-400 mesh) en éluant avec un mélange cyclohexane-acétate (85-15 en volumes), 7,19 g (64%) de l'énantiomère lévogyre du 2-benzyl-7-(3,4-dichlorophényl)-7-hydroxy-4-phényloctahydroisoindole-3a-carboxylate de méthyle-(3aRS,4SR,7RS,7aRS) sous forme d'un solide blanc dont la caractéristique est la suivante :
- pouvoir rotatoire : [α]₃₆₅²⁰= -28,6 +/- 0,7° (c = 0,5 / méthanol).

### Etape B

On opère comme à l'étape E de l'exemple 1, mais à partir de 9 g (17 mmol) de l'énantiomère lévogyre du 2-benzyl-7-(3,4-dichlorophényl)-7-hydroxy-4-phényl-1,3,3a,4,5,6-hexahydroisoindole-3a-carboxylate de méthyle-(3aRS,4SR,7RS,7aRS), de 23 cm³ d'acide trifluorométhanesulfonique et de 150 cm³ de dichlorométhane. On obtient 8,41 g (97 %) de l'énantiomère dextrogyre du 2-benzyl-9-(3,4-dichlorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue blanche dont la caractéristique est la suivante :
- pouvoir rotatoire : [α]₃₆₅²⁰ = +43,9 +/- 0,3° (c = 0,5 / méthanol).

### Etape C

On opère comme à l'étape I de l'exemple 3, mais à partir de 8,3 g (16,8 mmol) de l'énantiomère dextrogyre du 2-benzyl-9-(3,4-dichlorophényl)-4,9-éthano-2,3,3a,4,9,9a-hexahydro-1H-benzo[f]isoindole-3a-carboxylate de méthyle-(3aRS,4SR,9SR,9aRS) et de 3,86 cm³ (33,7 mmol) de chloroformiate de vinyle dans 50 cm³ de dichlorométhane pendant vingt-quatre heures à température ambiante. En reprenant le concentrat au reflux pendant huit heures dans 135 cm³ d'une solution 1N d'acide chlorhydrique dans l'oxyde de diéthyle, on obtient 5,47 g (75%) de l'énantiomère dextrogyre du 9-(2,4-dichlorophényl)-4,9-éthano-2,3,3a,4,9,9a-hexahydro-1H-benzo[f]isoindole-3a-carboxylate de méthyle-(3aRS,4SR,9SR,9aRS), sous forme d'une meringue
- pouvoir rotatoire : [α]₃₆₅²⁰= +67,3 +/- 1,2° (c = 0,5 / dichlorométhane).

### Etape D

On opère comme à l'étape G de l'exemple 1, mais à partir de 5,4 g (12,3 mmol) de l'énantiomère dextrogyre du 9-(3,4-dichlorophényl)-4,9-éthano-2,3,3a,4,9,9a-hexahydro-1H-benzo[f]isoindole-3a-carboxylate de méthyle-(3aRS,4SR,9SR,9aRS), de 2,3 g (12,9 mmol) d'acide 2-(2-méthoxyphényl)propènoïque, de 1,13 cm³ (12,9 mmol) de chlorure d'oxalyle et de 3,6 cm³ (25,8 mmol) de triéthylamine. On obtient, après purification par flash-chromatographie sur gel de silice (230-400 mesh) en éluant avec des mélanges cyclohexane-acétate (60-40 puis 50-50 en volumes), 5,45 g (79 %) de l'énantiomère dextrogyre du 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :
- pouvoir rotatoire : [α]₃₆₅²⁰= +78,6 +/- 1,4° (c = 0,5 / méthanol).

### Etape E

On opère comme à l'exemple 2, mais à partir de 5,2 g (9,2 mmol) de l'énantiomère dextrogyre du 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), de 46 cm³ d'une solution aqueuse normale d'hydroxyde de sodium et 100 cm³ de dioxane. On obtient, après recristallisation dans un mélange de 150 cm³ de cyclohexane et de 20 cm³ d'acétate d'éthyle d'acétate d'isopropyle, 4,23 g (83 %) de l'énantiomère dextrogyre de l'acide 9-(3,4-dichlorophényl)-4,9-éthano-2-[2-(2-méthoxyphényl)propènoyl]-2,3,3a,4,9,9a-hexahydro-1H-benzo[f] isoindole-3a-carboxylique-(3aRS,4SR,9SR,9aRS) dont les caractéristiques sont les suivantes :
- point de fusion = 210°C.
- pouvoir rotatoire : [α]₃₆₅²⁰= +85,2 +/- 1,3° (c = 0,5 / méthanol).

### EXEMPLE 6

### Préparation de l'énantiomère dextrogyre de l'acide 9-(4-bromophényl)-4,9-éthano-2-[2-(2-méthoxyphényl)propènoyl]- 2,3,3a,4,9,9a-hexahydro-1H-benzo[f]isoindole-3a-carboxylique -(3aRS, 4SR, 9SR,9aRS)

On opère selon les étapes A, B, C de l'exemple 1, puis D, E de l'exemple 3, puis comme ci-dessous :

### Etape A

On opère comme à l'étape G de l'exemple 3, mais à partir de 12 g (25 mmol) de l'énantiomère lévogyre du 2-benzyl-7-iodo-4-phényl-2,3,a,4,5,7a-hexahydroisoindole-3a-carboxylate de méthyle-(3aRS,4SR, 7aRS) dans 240 cm³ de toluène, de 1,46 g (1,25 mmol) de tétrakis(triphénylphosphine)palladium, de 5,56 g (27 mmol) d'acide 4-bromophénylboronique dans 120 cm³ de méthanol et de 240 cm³ d'une solution aqueuse 2N de carbonate de sodium au reflux pendant dix huit heures. On obtient, après purification par flash-chromatographie sur gel de silice (230-400 Mesh) en éluant par un mélange de cyclohexane et d'acétate d'éthyle (90-10 en volumes), 10,44 g (83 %) de l'énantiomère lévogyre du 2-benzyl-7-(4-bromophényl)-4-phényl-2,3,3a,4,5,7a-hexahydro-1H-isoindole-3a-carboxylate de méthyle-(3aRS,4SR,7aRS), sous forme d'une meringue blanche dont les caractéristiques sont les suivantes :
- pouvoir rotatoire : [α]₃₆₅²⁰= -10,4 +/- 0,7° (c = 0,5 / méthanol).
- spectre de masse (IE) : M/Z = 502 (M⁺).

### Etape B

On opère comme à l'étape E de l'exemple 1, mais à partir de 10,44 g (21 mmol) de l'énantiomère lévogyre du 2-benzyl-7-(4-bromophényl)-4-phényl-2,3,3a,4,5,7a-hexahydro-1H-isoindole-3a-carboxylate de méthyle-(3aRS,4SR,7aRS) et de 13,8 cm³ d'acide trifluorométhanesulfonique dans 100 cm³ de dichlorométhane pendant 18 heures à température ambiante. On obtient ainsi 9,26 g (89 %) de l'énantiomère dextrogyre du 2-benryl-9-(4-bromophényl)-4,9-éthano-2,3,3a,4,9,9a-hexahydro-1H-benzo[f]isoindole-3a-carboxylate de méthyle-(3aRS,4SR,9SR,9aRS), sous forme d'une poudre beige, utilisée telle quelle dans la suite de la synthèse, dont les caractéristiques sont les suivantes :
- pouvoir rotatoire : [α]₃₆₅²⁰= +38,5 +/- 0,9° (c = 0,5 / méthanol).
- spectre de masse (IE) : M/Z = 502 (M⁺).

### Etape C

On opère comme à l'étape I de l'exemple 3, mais à partir de 9,26 g (18 mmol) de l'énantiomère dextrogyre du 2-benzyl-9-(4-bromophényl)-4,9-éthano-2,3,3a,4,9,9a-hexahydro-1H-isoindole-3a-carboxylate de méthyle-(3aRS,4SR,9SR,9aRS) et de 5,06 cm³ (55 mmol) de chloroformiate de vinyle pendant soixante douze heures à température ambiante dans 200 cm³ de dichlorométhane, puis en reprenant le concentrat dans 200 cm³ de méthanol et 55 cm³ d'une solution 4M d'acide chlorhydrique dans le dioxane au reflux pendant trois heures. On obtient ainsi 7,32 g (80%) de chlorhydrate de l'énantiomère dextrogyre du 9-(4-bromophényl)-4,9-éthano-2,3,3a,4,9,9a-hexahydro-1H-isoindole-3a-carboxylate de méthyle-(3aRS,4SR,9SR,9aRS), sous forme de cristaux blancs dont les caractéristiques sont les suivantes :
- pouvoir rotatoire : [α]₃₆₅²⁰ = +59,8 +/- 1,1 ° (c = 0,5 / méthanol).
- spectre de masse (IE) : M/Z = 412 (M⁺).

### Etape D

On opère comme à l'étape G de l'exemple 1, mais à partir de 7,32 g (16,3 mmol) de chlorhydrate de l'énantiomère dextrogyre du 9-(4-bromophényl)-4,9-éthano-2,3,3a,4,9,9a-hexahydro-1H-benzo[f]isoindole-3a-carboxylate de méthyle-(3aRS,4SR,9SR,9aRS), de 3,2 g (17,9 mmol) d'acide 2-(2-méthoxyphényl)propènoique, de 1,57 cm³ (17,9 mmol) de chlorure d'oxalyle et de 5 cm³ (35,8 mmol) de triéthylamine en solution dans 70 cm³ de dichlorométhane contenant 0,2 cm³ de DMF pendant vingt heures à température ambiante. On obtient, après purification sur gel de silice (230-400 Mesh) en éluant par un mélange de cyclohexane et d'acétate d'éthyle (70-30 en volumes), 7,01 g (75 %) de l'énantiomère dextrogyre du 9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre beige-clair dont les caractéristiques sont les suivantes :
- pouvoir rotatoire : [α]₃₆₅²⁰= +60,6 +/- 1,1° (c = 0,5 / méthanol).
- spectre de masse (IE) : M/Z = 572 (M⁺)

### Etape E

On opère comme à l'exemple 2, mais à partir de 7,01 g (12,2 mmol) de l'énantiomère dextrogyre du 9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR,9aRS) au reflux pendant sept heures dans 15,3 cm³ d'une solution aqueuse normale d'hydroxyde de sodium et de 250 cm³ d'éthanol. On obtient, après purification par flash-chromatographie sur gel de silice (230-400 Mesh) en éluant par un mélange d'acétate d'éthyle de méthanol et d'acide acétique (95-4-1 en volumes) puis recristallisation dans un mélange d'acétate d'éthyle et d'oxyde de diisopropyle (1-4 en volumes), 2,28 g (33 %) d'acide 9-(4-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), énantiomère dextrogyre sous forme d'un solide blanc dont les caractéristiques sont les suivantes :
- pouvoir rotatoire : [α]₃₆₅²⁰= +72,6 +/- 1,2° (c = 0,5 / méthanol).
- point de fusion = 205°C
- spectre de masse (IE) : M/Z = 558 (M⁺).

### EXEMPLE 7

### Préparation l'énantiomère dextrogyre de l'acide 4,9-éthano-2-[2-(2-méthoxyphényl)propènoyl]-9-(4-chlorophényl)-2,3,3a,4,9,9a-hexahydro-1H-benzo[f]isoindole-3a-carboxylique-(3aRS,4SR,9SR,9aRS)

On opère selon les étapes A, B, C de l'exemple 1 puis selon :

### Etape A

On opère comme à l'étape D de l'exemple 1, mais à partir de 10,2 g (53 mmol) de 4-chlorobenzène, de 1,2 g (53 mmol) de magnésium en tournures et de 9,67 g (26,6 mmol) de l'énantiomère lévogyre du 2-benzyl-7-oxo-4-phénylperhydroisoindole-3a-carboxylate de méthyle-(3aRS,4SR,7aRS) dans 60 cm³ d'oxyde de diéthyle et 60 cm³ de toluène. On obtient, après purification sur gel de silice (230-400 Mesh) en éluant par un mélange de cyclohexane et d'acétate d'éthyle (90-10 en volumes), 8,5 g (68%) de l'énantiomère lévogyre du 2-benzyl-7-(4-chlorophényl)-7-hydroxy-4-phényloctahydroisoindole-3a-carboxylate de méthyle-(3aRS,4SR,7RS,7aRS) sous forme d'un solide blanc dont la caractéristique est la suivante :
- pouvoir rotatoire : [α]₃₆₅²⁰= -24,2 +/- 0,7° (c = 0,5 / méthanol).

### Etape B

On opère comme à l'étape E de l'exemple 1, mais à partir de 9,3 g (19,5 mmol) de l'énantiomère lévogyre du 2-benzyl-7-(4-chlorophényl)-7-hydroxy-4-phényl-2,3,3a,4,5,6-hexahydroisoindole-3a-carboxylate de méthyle-(3aRS,4SR,7RS,7aSR), de 21,6 cm³ (0,2 mol) d'acide trifluorométhanesulfonique et de 100 cm³ de dichlorométhane. On obtient, après purification sur gel de silice (230-400 Mesh) en éluant par un mélange de cyclohexane et d'acétate d'éthyle (90-10 en volumes, 6,9 g (77 %) de l'énantiomère dextrogyre du 2-benzyl-9-(4-chlorophényl)-4,9-éthano-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :
- pouvoir rotatoire : [α]₃₆₅²⁰= +43,7 +/- 0,9° (c = 0,5 / méthanol).

### Etape C

On opère comme à l'étape I de l'exemple 3, mais à partir de 6,9 g (15 mmol) de l'énantiomère dextrogyre du 2-benzyl-9-(4-chlorophényl)-4,9-éthano-2,3,3a,4,9,9a-hexahydro-1H-benzo[f]isoindole-3a-carboxylate de méthyle-(3aRS,4SR,9SR,9aRS) et de 4,14 cm³ (45 mmol) de chloroformiate de vinyle dans 100 cm³ de dichlorométhane, suivi de purification sur gel de silice (230-400 Mesh) en éluant par un mélange de cyclohexane et d'acétate d'éthyle (70-30 en volumes) du carbamate intermédiaire, qui est ensuite repris dans 200 cm³ de méthanol et 48 cm³ d'une solution 4N d'acide chlorhydrique dans le dioxane. On obtient ainsi 5,34 g (85 %) de l'énantiomère dextrogyre du chlorhydrate de 9-(4-chlorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue blanche dont la caractéristique est la suivante :
- pouvoir rotatoire : [α]₃₆₅²⁰= +66,8 +/- 1,2° (c = 0,5 / méthanol).

### Etape D

On opère comme à l'étape G de l'exemple 1, mais à partir de 5,34 g (13,2 mmol) de l'énantiomère dextrogyre du chlorhydrate de 9-(4-chlorophényl)-4,9-éthano-2,3,3a,4,9,9a-hexahydro-1H-benzo[f]isoindole-3a-carboxylate de méthyle-(3aRS,4SR,9SR,9aRS), de 2,6 g (14,5 mmol) d'acide 2-(2-méthoxyphényl) propènoïque, de 1,3 cm³ (14,5 mmol) de chlorure d'oxalyle et de 4 cm³ (29 mmol) de triéthylamine. On obtient, après purification sur gel de silice (230-400 Mesh) en éluant par un mélange de cyclohexane et d'acétate d'éthyle (70-30 en volumes), 6,48 g (93 %) de l'énantiomère dextrogyre du 9-(4-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) dont la caractéristique est la suivante :
- pouvoir rotatoire : [α]₃₆₅²⁰= +66,4 +/- 1,1° (c = 0,5 / méthanol).

### Etape E

On opère comme à l'exemple 2, mais à partir de 6,4 g (12 mmol) de l'énantiomère dextrogyre du 9-(4-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), de 24 cm³ d'une solution aqueuse normale d'hydroxyde de sodium et 160 cm³ d'éthanol. On obtient, après purification par flash-chromatographie sur gel de silice (230-400 mesh) en éluant avec un mélange dichlorométhane-éthanol (96-4 en volumes) puis recristallisation dans l'acétate d'isopropyle, 1,55 g (25 %) d'acide 9-(4-chlorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), énantiomère dextrogyre dont les caractéristiques sont les suivantes :
- point de fusion: 210°C.
- pouvoir rotatoire : [α]₃₆₅²⁰ = +72,2, +/- 1,2° (c = 0,5 / méthanol).

### EXEMPLE 8

### Préparation de l'énantiomère dextrogyre de l'acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)

On opère selon les étapes A, B, C de l'exemple 1, puis on opère comme décrit dans les exemples 61 (étapes A, B, C) et 62 de la demande WO 98/29390 mais à partir de l'énantiomère lévogyre 2-benzyl-7-oxo-4-phényloctahydroisoindole-3a-carboxylate de méthyle-(3aRS,4SR,7aRS) pour former successivement :
énantiomère dextrogyre 2-benzyl-9-(2,3-dihydrobenzofuran-5-yl)-4,9-éthano-2,3,3a,4,9,9a-hexahydro-1H-benzo[f]isoindole-3a-carboxylate de méthyle-(3aRS,4SR,9SR,9aRS)
énantiomère dextrogyre 9-(2,3-dihydrobenzofuran-5-yl)-4,9-éthano-2,3,3a,4,9,9a-hexahydro-1H-benzo[f]isoindole-3a-carboxylate de méthyle-(3aRS,4SR, 9SR,9aRS),
énantiomère dextrogyre 9-(2,3-dihydrobenzofuran-5-yl)-4,9-éthano-2-[2-(2-méthoxyphényl)propènoyl]-2,3,3a,4,9,9a-hexahydro-1H-benzo[f] isoindole-3a-carboxylate de méthyle-(3aRS,4SR, 9SR, 9aRS
énantiomère dextrogyre d'acide 9-(2,3-dihydrobenzofuran-5-yl)-4,9-éthano-2-[2-(2-méthoxy phényl)propènoyl]-2,3,3a,4,9,9a-hexahydro-1H-benzo[f]isoindole-3a-carboxylique-(3aRS,4SR,9SR,9aRS), sous forme d'une poudre cristalline blanche, dont les caractéristiques sont les suivantes :
   - point de fusion = 241°C
   - spectre de masse (IE) : M/Z = 521 (M⁺)
   - pouvoir rotatoire : [α]₃₆₅²⁰= +75,5 +/- 1,3° (c = 0,5 / DMF).

### EXEMPLE 9

### Préparation de l'énantiomère dextrogyre de l'acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)

### Etape A

On chauffe, à 100°C pendant une heure, une solution de 45,77 g (0,4 mol) de thiophène-3-carboxaldéhyde et 33,43 g (0,28 mol) de N-acétylglycine dans 75 cm³ d'anhydride acétique contenant 16,8 g (0,28 mol) d'acétate de sodium sec, dans les conditions décrites par Org. Synth., II, 1-3, puis on laisse refroidir la nuit à une température voisine de 0°C. Les cristaux formés sont filtrés, on obtient alors 42,8 g de cristaux beiges qui sont portés au reflux pendant quatre heures dans 150 cm³ d'eau et 400 cm³ d'acétone. Après refroidissement, les solvants sont évaporés sous pression réduite, et le résidu est purifié par recristallisation dans 350 cm³ d'eau. On obtient ainsi 33,79 g (39 %) d'acide 1-acétylamino-3-(3-thiényl)-propèn-2-oique, sous forme de fins cristaux jaunes dont la caractéristique est la suivante:
- point de fusion = 228°C

### Etape B

On chauffe, au reflux pendant quatre heures, 33,78 g (89 mmol) d'acide 1-acétylamino-3-(3-thiényl)-propèn-2-oique dans 360 cm³ d'une solution aqueuse normale d'acide chlorhydrique, dans les conditions décrites dans Org. Synth, II, 519-20. Après trois heures de chauffage, des cristaux se forment et sont filtrés après refroidissement à 0°C. On obtient ainsi 22,4 g (99 %) d'acide (3-thiényl)pyruvique, sous forme de fins cristaux blancs dont les caractéristiques sont les suivantes :
- point de fusion = 203-4°C
- spectre de masse (IE) : M/Z = 170 (M⁺).

### Etape C

A une solution, refroidie à 0°C, de 22,4 g (13 mmol) d'acide (3-thiényl)pyruvique dans 150 cm³ de méthanol, on ajoute successivement 17,1 cm³ (21 mmol) de méthylvinylcétone et 7,2 g (18 mmol) d'hydroxyde de sodium en pastille, puis on agite à une température voisine de 20°C pendant deux heures. Après neutralisation avec 60 cm³ d'une solution aqueuse 3N d'acide chlorhydrique, le précipité formé est essoré, lavé à l'eau, puis séché à 20°C. On obtient ainsi 21,85 g (70%) d'acide 6-(3-thiényl)-3-oxo-cyclohexan-1-ol-1-carboxylique, sous forme d'une poudre jaune dont les caractéristiques sont les suivantes :
- point de fusion = 204°C
- spectre de R.M.N. ¹H (300 MHz, (CD₃)₂SO d6 avec ajout de quelques gouttes de CD₃COOD d4, d en ppm) : de 2,20 à 2,80 (mt, 4H : CH₂CH₂) ; 2,34 et 3,00 (2 d, J = 14 Hz, 1H chacun : COCH₂) ; 3,66 (dd, J = 12,5 et 3 Hz,1H : ArCH) ; 7,06 (d, J = 5Hz, 1H : H en 4 du thiényle) ; 7,24 (d large, J = 3 Hz, 1H : H en 2 du thiényle) ; 7,36 (dd, J = 5 et 3 Hz, 1H : H en 5 du thiényle).

### Etape D

21,84 g (9,1 mmol) d'acide 6-(3-thiényl)-3-oxo-cyclohexan-1-ol-1-carboxylique sont chauffés à reflux, pendant trois heures, dans 220 cm³ de toluène en présence de 2,2 g d'acide para toluène sulfonique. Le mélange réactionnel est ensuite refroidi à 5° et le précipité formé est filtré, lavé à l'éther isopropylique puis séché à 50°C. On obtient ainsi 18,47 g (91 %) d'un mélange contenant majoritairement de l'acide 6-(3-thiényl)-3-oxo-cyclohexène-1-carboxylique-(RS), sous forme d'un solide brun, utilisé tel quel à l'étape suivante, dont les caractéristiques sont les suivantes :
- spectre de masse (IE) : M/Z = 222 (M⁺)

### Etape E

A une solution de 18,45 g (8,3 mmol) d'acide 6-(3-thiényl)-3-oxo-cyclohexène-1-carboxylique-(RS), dans 250 cm³ d'acétone sec, on ajoute successivement 7,5 cm³ (12 mmol) d'iodure de méthyle et 16,8 cm³ (11,2 mol) de 1,8-diazabicyclo[5.4.0]undéc-7-ène goutte à goutte, puis on porte au reflux pendant deux heures trente minutes. Après filtration à chaud d'un insoluble et concentration des solvants sous pression réduite, le résidu est purifié par flash-chromatographie sur gel de silice (230-400 Mesh), en éluant au dichlorométhane. On obtient ainsi 14,84 g (76 %) de 6-(2-thiényl)-3-oxo-cyclohexène-1-carboxylate de méthyle-(RS), sous forme d'une poudre jaune dont les caractéristiques sont les suivantes :
- point de fusion = 69°C
- spectre de masse (IE) : M/Z = 236 (M⁺)
- spectre de R.M.N. ¹ H (250 MHz, CDCl₃, d en ppm) : de 2,05 à 2,55 (mt, 4H : CH₂CH₂) ; 3,77 (s, 3H : COOCH₃) ; 4,33 (mt, 1H : ArCH) ; 6,85 (s, 1H : =CH) ; 6,91 (dd, J = 2,5 et 2 Hz, 1H : H en 2 du thiényle) ; 6,99 (dd, J = 5 et 2 Hz, 1H : H en 4 du thiényle) ; 7,34 (dd, J = 5 et 2,5 Hz, 1H : H en 5 du thiényle).

### Etape F

On opère selon l'étape B de l'exemple 1, mais à partir de 6-(3-thiényl)-3-oxo-cyclohexène-1-carboxylate de méthyle-(RS), pour aboutir au 6-(3-thiényl)-3-oxo-cyclohexène-1-carboxylate de méthyle-(RS) sous forme lévogyre.

On opère ensuite comme dans les exemples 1 (étape F) et 2 (étapes A à G) de la demande FR 2772764, mais à partir de 6-(3-thiényl)-3-oxo-cyclohexène-1-carboxylate de méthyle-(RS) sous forme lévogyre, pour former successivement :
2-benzyl-4-(3-thiényl)-7-oxo-octahydroisoindole-3a-carboxylate de méthyle-(3aRS,4SR,7aRS), sous forme lévogyre
2-benzyl-7-hydrazono-4-(3-thiényl)-octahydro-isoindole-3a-carboxylate de méthyle-(3aRS,4SR,7aRS) sous forme lévogyre
2-benzyl-7-iodo-4-(3-thiényl)-1,2,3,4,5,7a-hexahydro-isoindole-3a-carboxylate de méthyle-(3aRS,4SR,7aRS) sous forme lévogyre
2-benzyl-7-(4-méthylphényl)-4-(3-thienyl)-1,2,3,4,5,7a-hexahydro-isoindole-3a-carboxylate de méthyle-(3aRS,4SR,7aRS) sous forme lévogyre
2-benzyl-4,8-éthano-8-(4-méthylphényl)-2,3,3a,4,8,8a-hexahydro-1H-thiéno[2,3-f]isoindole-3a-carboxylate de méthyle-(3aRS,4SR,8SR,8aRS) sous forme dextrogyre 4,8-éthano-8-(4-méthylphényl)-2,3,3a,4,8,8a-hexahydro-1H-thiéno[2,3-f]isoindole-3a-carboxylate de méthyle-(3aRS,4SR,8SR,8aRS), sous forme dextrogyre
4,8-éthano-2-[2-(2-méthoxyphényl)propènoyl)-8-(4-méthylphényl)-2,3,3a,4,8,8a-hexahydro-1H-thiéno[2,3-f]isoindole-3a-carboxylate de méthyle-(3aRS,4SR,8SR,8aRS) sous forme dextrogyre
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS) sous forme dextrogyre, sous forme d'une poudre jaune très pâle dont les caractéristiques sont les suivantes :
   - point de fusion = 176°C
   - pouvoir rotatoire : [α]₃₆₅²⁰= 101,6 +/- 1,4° (c = 0,5 / méthanol)
   - spectre de R.M.N. ¹ H (400 MHz, (CD₃)₂SO d6, à une température de 393 K, d en ppm) : 1,34 - 1,53 - et de 1,95 à 2,10 (3 mts, respectivement 1H - 1H et 2H : CH₂CH₂) ; 2,38 (s, 3H : ArCH₃) ; de 3,20 à 3,50 (mt, 3H : CH₂ en 1 et CH en 8a) ; 3,53 et 4,01 (respectivement d et d large, J = 12,5 Hz, 1H chacun : CH₂ en 3) ; 3,65 (s large, 1H : CH en 4) ; 3,73 (s, 3H : ArOCH₃) ; 5,54 et 5,68 (2 s larges, 1H chacun : =CH₂) ; de 6,90 à 7,40 (mt, 10H : H aromatiques en ortho et méta du 4 méthylphényle - H aromatiques du 2-méthoxyphényle - H en 5 et H en 6).

### EXEMPLE 10

Les composés correspondants aux exemples 1 à 101 de la demande WO98/29390 et aux exemples 1 à 6 de la demande FR 2772764 peuvent être préparés respectivement à partir de l'intermédiaire obtenu à l'étape B de l'exemple 1 et à l'étape F de l'exemple 9 de la présente demande, en opérant par application des procédures exemplifiées dans les demandes WO 98/29390 et FR 2772764 correspondantes.

### EXEMPLE 11

L'étape B de l'exemple 1 a été effectuée à partir du 3-oxo-6-phenyl-cyclohex-1-ène-carboxylate de méthyle-(R,S) sous forme racémique avec différentes enzymes, ; les résultats obtenus ont été les suivants :

### Séparation de l'énantiomère dextrogyre du 3-oxo-6-phenyl-cyclohex-1-ène-carboxylate de méthyle-(R,S) :

| **Enzyme (distributeur)** | **ee (%)** | **Rendement (%)** |
|---|---|---|
| Pronase (Boehr. Mann.) | 91 | 82 |
| | 100 | 41 |
| Subtilisin (NOVO) | 100 | 40 |
| Alacalase 2,4 L (NOVO) | 100 | 38 |
| | 92 | 40 |
| Protéase Nagarse (Sigma) | 100 | 32 |
| EH 16 (Altus Biol.) | 97 | 29 |
| EH 15 (Altus Biol.) | 91 | faible |
| EH 11 (Altus Biol.) | 45 | 39 |
| Lipase M (Amano) | 74 | 54 |

### Séparation de l'énantiomère lévogyre du 3-oxo-6-phenyl-cyclohex-1-ène-carboxylate de méthyle-(R,S) :

| **Enzyme** | **ee (%)** | **Rendement (%)** |
|---|---|---|
| Lipase L2 (Boehr. Mann.) | 95 | 30 |
| Lipase PS C (Amano) | 36 | 55 |
| ESL 01 (Rec. Biocatalyst) | 79 | 29 |
| SP 525 (NOVO) | 100 | faible |
| Lipase L6 (Boehr. Mann.) | 50 | 22 |
| Estérase 30000 (Gist Broc) | 25 | 37 |
| **ee :** excés énantiomérique | | |

## Revendications

1. Procédé de préparation des composés de formule générale I, sous forme de leurs isomères optiques : 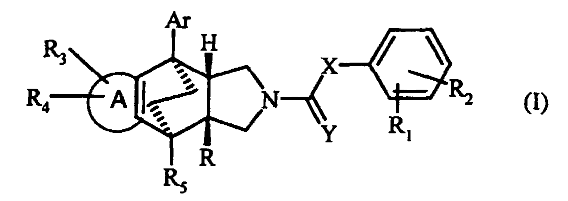 dans laquelle:
- A représente :
• soit un radical phényle fusionné avec le noyau isoindole
et dans ce cas :
- R₃ et R₄, identiques ou différents, représentent un atome d'hydrogène, d'halogène ou un radical alcoyle, hydroxy, alcoyloxy, alcoylcarbonyloxy, mercapto, alcoylthio, alcoylsulfonyle ou alcoylsulfinyle, amino, alcoylamino ou dialcoylamino, alcoyloxycarbonylamino, carboxy, alcoyloxycarbonyle, carbamoyle alcoylcarbamoyle ou dialcoylcarbamoyle, formyl, alcoylcarbonyle, cyano ou trifluorométhyle,
• soit A représente un système monocyclique ou, bi-, ou tri-cyclique condensé, pour lequel chaque cycle, saturé ou non, contient de 4 à 7 chaînons, et pour lequel au moins un des cycles contient de 1 à 4 hétéroatomes, identiques ou différents, choisis indépendamment parmi les atomes d'azote, d'oxygène et de soufre;
et dans ce cas R₃ et R₄ représentent un atome d'hydrogène.
- Ar représente
- un radical phényle éventuellement substitué par un ou plusieurs atomes ou radicaux, identiques ou différents, choisis parmi les atomes d'halogène et les radicaux alcoyles contenant 1 à 4 atomes de carbone, alcényles contenant 2 à 4 atomes de carbone, hydroxy, mercapto, alcoylthio, alcoylsulfonyle ou alcoylsulfinyle, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle, alcoxy contenant 1 à 4 atomes de carbone, dont la portion alcoyle est éventuellement perhalogénée, ou
- un radical phényle condensé à un hétérocycle de 4 à 7 chaînons contenant un ou plusieurs hétéroatomes choisis parmi les atomes d'oxygène, d'azote et de soufre, ou
- un radical polycyclique aromatique ou non aromatique,
- un radical hétérocyclique aromatique ou non aromatique de 5 à 12 chaînons incorporant un ou plusieurs hétéroatomes choisis parmi les atomes d'oxygène, d'azote et de soufre, lié au cycle condensé par une liaison carbone-carbone, ledit radical étant substitué le cas échéant par un ou plusieurs atomes ou radicaux, identiques ou différents, choisis parmi les atomes d'halogène et les radicaux alcoyles, alcényles contenant 2 à 4 atomes de carbone, hydroxy, alcoxy contenant 1 à 4 atomes de carbone, mercapto, alcoylthio, alcoylsulfonyle ou alcoylsutfinyl, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle,
avec pour l'ensemble de ces radicaux, alcoyl contenant 1 à 4 atomes de carbone,
- R représente
un radical de formule générale
- (CH₂)ₘ-X₁-(CH₂)ₙ-Z
dans laquelle
- X₁ représente une simple liaison ou un atome d'oxygène ou de soufre,
- m représente un nombre entier égal à 0 ou 1,
- n représente un nombre entier égal à 0, 1 ou 2,
- un ou plusieurs radicaux méthylènes peuvent être substitués par un radical carboxy, alcoxycarbonyle, carbamoyle, alcoylcarbamoyle, dialcoylcarbamoyle, amino, alcoylamino, dialcoylamino avec pour l'ensemble de ces radicaux, alcoyl contenant 1 à 4 atomes de carbone,
- Z représente
- un radical carboxy,
- un radical COOR₆, dans lequel R₆ représente un radical alcoyle droit ou ramifié contenant 1 à 3 atomes de carbone,ou
- un radical de formule CON(R₇)(R₈) dans lequel
- R₇ représente un atome d'hydrogène ou un radical alcoyle droit ou ramifié contenant 1 à 6 atomes de carbone et
- R₈ représente
- un atome d'hydrogène,
- un radical hydroxy,
- un radical arylsulfonyle, éventuellement substitué par un ou plusieurs atomes ou radicaux, identiques ou différents, choisis parmi les atomes d'halogène et les radicaux alcoyles, alcoyloxy, avec, pour ces radicaux alcoyl contenant 1 à 4 atomes de carbone,
- un hétérocycle de 5 à 7 chaînons incorporant un ou plusieurs hétéroatomes choisis parmi les atomes d'azote, d'oxygène ou de soufre, ledit hétérocycle pouvant être lié par un hétéroatome,
- un radical amino éventuellement substitué par un ou deux radicaux, identiques ou différents choisis parmi les radicaux
- alcoyle contenant 1 à 4 atomes de carbone,
- aryle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
- hétérocyclyle de 5 à 7 chaînons et contenant un ou plusieurs hétéroatomes choisis parmi les atomes d'azote, d'oxygène et de soufre,
- arylcarbonyle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
- un radical alcoyloxy contenant 1 à 6 atomes de carbone en chaîne droite ou ramifiée éventuellement substitué par un radical phényle,
- un radical alcoyle droit ou ramifié contenant 1 à 6 atomes de carbone, tel que méthyle, éventuellement substitué par un radical amino, alcoylamino, dialcoylamino, hydroxy, alcoxy contenant 1 à 4 atomes de carbone, mercapto, alcoylthio, alcoyloxycarbonyle, carboxy, cyano, un radical aromatique mono- ou polycyclique et ayant de 5 à 12 chaînons, incorporant ou non, un ou plusieurs hétéroatomes choisis parmi les atomes d'oxygène, d'azote et de soufre éventuellement substitué, ou pouvant être également un radical phényle éventuellement substitué par un ou plusieurs atomes d'halogène ou par un ou plusieurs groupements hydroxy, amino, ou trifluorométhyle, ou par un ou plusieurs radicaux alcoyle ou alcényle, alcoxy, alcoylthio, alcoylamino, alcoylcarbonyle ou alcoxycarbonyle en C2 à C4, carbamoyle, alcoylcarbamoyle ou dialcoylcarbamoyle dont la partie alcoyle est en C1 à C8 , formyle ou encore un radical naphtyle-1 ou -2 ou,
- Z représente
- un radical PO(OR₉)₂ dans lequel R₉ représente un atome d'hydrogène ou un radical alcoyle droit ou ramifié contenant 1 à 6 atomes de carbone, ou
- un radical -NH-CO-T dans lequel T représente un atome d'hydrogène ou un radical alcoyle droit ou ramifié contenant 1 à 6 atomes de carbone éventuellement substitué par un radical amino, carboxy, alcoyloxycarbonyle, hydroxy, alcoyloxy, mercapto ou alcoylthio, ou bien
- un radical 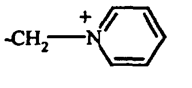 ayant pour contre-ion un anion, tel que le trifluorométhanesulfonate,
avec pour l'ensemble des radicaux possédant un groupement alcoyle, proposés dans la définition de Z, alcoyl contenant 1 à 4 atomes de carbone.
- R₁ et R₂, identiques ou différents, représentent un atome d'hydrogène, un atome d'halogène ou un radical alcoyle, un radical alcoyloxy, chacun éventuellement substitué par un radical dialcoylamino dont chaque partie alcoyle contient 1 à 4 atomes de carbone ou forme avec l'atome d'azote un hétérocycle saturé contenant 5 ou 6 chaînons, un radical alcoylthio, un radical alcoyloxycarbonyle, ou bien
situés en ortho l'un par rapport à l'autre, R₁ et R₂ forment un hétérocycle saturé ou non saturé contenant 1 ou 2 hétéroatomes choisis parmi l'azote et l'oxygène, éventuellement substitué par un atome d'halogène ou par un radical alcoyle ou alcoyloxy,
avec pour l'ensemble des radicaux possédant un groupement alcoyle, proposés dans la définition de R₁ et R₂, alcoyle contenant 1 à 4 atomes de carbone.
- R₅ représente un atome d'hydrogène ou un radical alcoyle, un radical alcoylthio, avec pour la définition de R₅, alcoyle contenant 1 à 4 atomes de carbone;
- X représente un atome d'oxygène ou de soufre ou un groupement -NH-, -CO-, méthylène, alcèn-1,1-diyle tel que vinyldiyle ou cycloalcan-1,1-diyle contenant 3 à 6 atomes de carbone,
et
- Y représente un atome d'oxygène ou de soufre,
ainsi que les sels du produit de formule générale (I),
**caractérisé en ce que** l'on part du produit de formule générale (X') sous forme d'isomère optique : 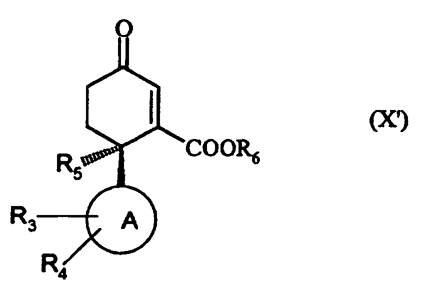 qui est obtenu à partir du produit de formule générale (X) sous forme racémique : 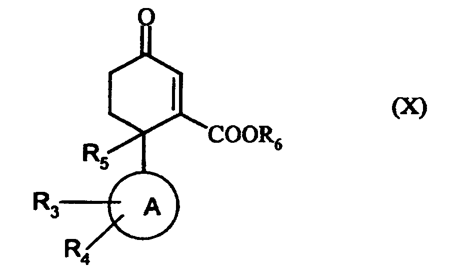 par action d'une enzyme,
puis le produit de formule générale (X'), sous forme d'isomères optiques, est transformé en produit de formule générale (IX) : 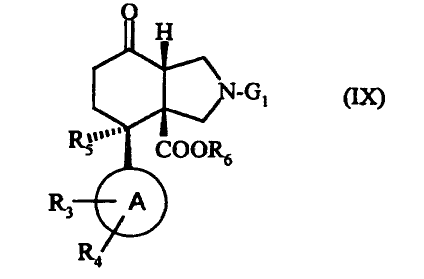 sous forme d'isomères optiques, dans laquelle A, R₃, R₄, R₅, R₆ sont tels que définis en formule générale I, G₁ représente un groupement protecteur de la fonction amine, par action d'une N-trialcoylsilylméthyl-N-alcoxyméthyl-amine portant un groupement protecteur de la fonction amine,
le produit de formule générale (IX) étant ensuite transformé en produit de formule générale (I) :
1. par cyclisation du noyau cycloperhydroisoindole pour former les composés de formule générale (III) 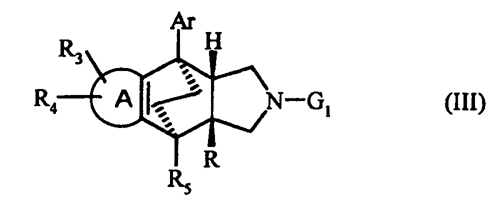 sous forme d'isomères optiques,
dans laquelle :
- A, Ar, R, R_{3,} R₄ et R₅ sont définis selon la formule générale I et
- G₁ représente un atome d'hydrogène,
via la formation d'un intermédiaire de formule générale (XV) 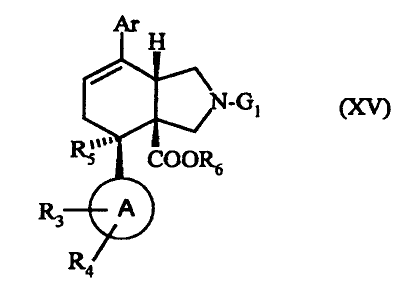 sous forme d'isomères optiques, de préférence l'isomère lévogyre dans laquelle A, R₃, R₄, R₅, R₆, Ar et G₁ sont tels que définis pour le composé (IX),
puis indifféremment par couplage avec la chaîne latérale, et éventuellement modification du substituant R pour former les produits de formule générale (I), ou
2. par couplage de la chaîne latérale pour former le produit de formule générale (VIII) : 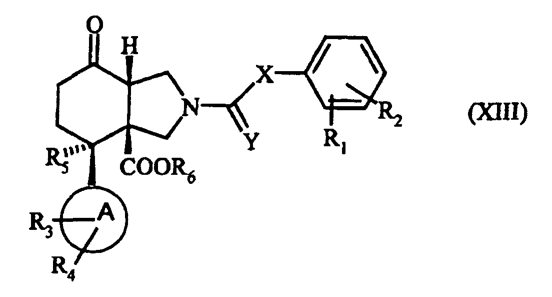 sous forme d'isomères optiques, dans laquelle A, R₁, R₂, R₃, R₄, R₅, R₆, X et Y sont définis comme précédemment, puis indifféremment par cyclisation du noyau cycloperhydroisoindole et éventuellement modification du substituant R pour former les produits de formule générale (I) sous forme d'isomères optiques.

2. Procédé de préparation des composés de formule générale (I) selon la revendication 1 pour lequel, dans la formule générale (I) :
A représente
• soit un radical phényle fusionné au cycle isoindole
• soit un radical thiényle, indolyle ;
et R₃ et R₄ représentent un atome d'hydrogène,
Ar représente un radical 2,3-dihydro-1,4-benzodioxin-6-yle ou 2,3-dihydrobenzofuran-5-yl, benzothièn-2-yle ou un radical phényle éventuellement substitué en position 4, de préférence par un radical méthyle, trifluorométhyle, méthoxy ou un atome de chlore ou de brome, ou en position 2, 4 par un atome de chlore ;
R représente un radical carboxy, ou un radical -COOMe, ou encore un radical -CON(R₇)(R₈) pour lequel lorsque R₇ représente un atome d'hydrogène, R₈ représente un radical méthyle substitué par le radical 3-pyridyle,
l'un des symboles R₁ ou R₂ représente un atome d'hydrogène et l'autre des symboles représente un radical méthoxy, et plus avantageusement fixé en position *ortho* du cycle phénylique,
R₅ représente un atome d'hydrogène ou un radical méthyle,
X représente un groupement méthylène ou vinyldiyle,
Y représente un atome d'oxygène;
sous forme de leurs isomères optiques ainsi que leurs sels.

3. Procédé de préparation des composés de formule générale (I) selon l'une quelconque des revendications précédentes, **caractérisé en ce que** les produits de formule générale (I) sont choisis individuellement parmi :
4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS)
acide 4,9-éthano-2-[(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS)
acide 4,9-éthano-2-[(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS),
acide 4,9-éthano-9-(4-méthoxyphényl)-2-[(2-méthoxyphényl)acétyl]-2,3,3a,4,9,9a-hexahydro-1H-benzo[f]isoindole-3a-carboxylique (3aRS,4SR,9SR,9aRS),
acide 4,9-éthano-2-[(2-méthoxyphényl)acétyl]-9-(4-méthylsulfanylphényl)-2,3,3a,4, 9,9a-hexahydro-1H-benzo[f]isoindole-3a-carboxylique (3aRS,4SR,9SR,9aRS),
acide 4,9-éthano-9-(4-fluorophényl)-2-[(2-méthoxyphényl)acétyl]-2,3,3a,4,9,9a-hexahydro-1H-benzo[f]isoindole-3a-carboxylique-(3aRS,4SR,9SR,9aRS),
acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS),
acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS),
9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS),
acide 9-(3,4-diméthylphényl)-4,9-éthano-2-[(2-méthoxyphényl)acétyl]-2,3,3a,4,9,9a-hexahydro-1H-benzo[f]isoindole-3a-carboxylique-(3aRS,4SR,9SP,9aRS),
3a-N-benzylcarbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),
3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),
4,9-éthano-2-[2-(2-méthoxyphényl)propènoyl]-9-(4-méthylphényl)-2,3,3a,4,9,9a-hexahydro-1H-benzo[f]isoindole-3a-hydroxamate de benzyle-(3aRS,4SR,9SR,9aRS),
acide 4,9-éthano-2-[2-(2-méthoxyphényl)propènoyl]-9-(4-méthylphényl)-3,3a,4,9,9a-hexahydro-1H-benzo[f]isoindole-3a-hydroxamique-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR, 9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N-(4-pyridylméthyl)-carboxamide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-hydroxamate de méthyle-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N',N'-diméthylcarbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-phénylcarbohydrazide-(3aRS,4SR, 9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N',N'-pentaméthylènecarbohydrazide-(3aRS,4SR,9SR,9aRS)
4,9-éthano-2-[2-(méthoxyphényl)propènoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N-(N-oxo-3pyridyl)méthyl-carboxamide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-(4-méthoxyphényl)carbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-méthyl,N'-phénylcarbohydrazide-(3aRS,4SR,9SR,9aRS)
4,9-éthano-2-[2-(2-méthoxyphényl)propènoyl]-9-(4-méthylphényl)-2,3,3a,4,9,9a-hexahydro-1H-benzo[f]isoindole-3a-N'-(2-méthylphényl)carbohydrazide-(3aRS,4SR,9SR,9aRS)
9-(2,3-dihydro-1,4-benzodioxin-6-yl)-4,9-éthano-2-[2-(2-méthoxy phényl)propènoyl]-2,3,3a,4,9,9a-hexahydro-1H-benzo[f]isoindole-3a-carboxylate de méthyle-(3aRS,4SR,9SR,9aRS)
acide 9-(2,3-dihydro-1,4-benzodioxin-6-yl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)
9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f]isoindole-3a-[N-(3-pyridylméthyl)carboxamide-(3aRS,4SR,9SR,9aRS)
9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f]isoindole -3a-N-(2-thiénylméthyl)carboxamide-(3aRS,4SR,9SR,9aRS)
acide 4,9-éthano-2-[(2-méthoxyphényl)acétyl]-9-(3,4,5-triméthylphényl)-2,3,3a,4,9,9a-hexahydro-1H-benzo[f]isoindole-3a-carboxylique-(3aRS,4SR,9SR,9aRS)
acide 4,9-éthano-2-[2-(2-méthoxyphényl)propènoyl]-9-(2-méthylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-N-(4-pyridylméthyl)-carboxamide-(3aRS,4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-N'-benzoyl-carbohydrazide-(3aRS,4SR,9SR,9aRS)
4,9-éthano-2-[2-(2-méthoxyphényl)propènoyl]-9-(4-trifluorométhylphényl)-2,3,3a,4,9,9a-hexahydro-1H-benzo[f]isoindole-3a-N'-phényl-carbohydrazide-(3aRS,4SR,9SR,9aRS)
4,9-éthano-2-[2-(2-méthoxyphényl)propènoyl]-9-(2-naphtyl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR, 9aRS)
4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR,9SR,9aRS)
acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR, 9SR,9aRS)
9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)
acide 9-(4-bromophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR, 9aRS)
acide 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(4-chlorophényl)-2,3,3a,4,9,9a-hexahydro-1H-benzo[f]isoindole-3a-carboxylate de méthyle-(3aRS, 4SR,9SR,9aRS)
acide 9-(4-chlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(3-indolyl)-2,3,3a,4,9,9a-hexahydro-1H-benzo[f]isoindole-3a-carboxylate de méthyle-(3aRS, 4SR,9SR,9aRS)
acide 4,9-éthano-9-(4-isopropylphényl)-2-[(2-méthoxyphényl)acétyl]-2,3,3a,4,9,9a-hexahydro-1H-benzo[f]isoindole-3a-carboxylique-(3aRS,4SR,9SR, 9aRS)
acide 4,9-éthano-9-(4-isopropylphényl)-2-[2-(2-méthoxyphényl) propènoyl]-2,3,3a,4,9,9a-hexahydro-1H-benzo[f]isoindole-3a-carboxylique-(3aRS, 4SR,9SR, 9aRS)
4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS, 4SR, 9SR,9aRS)
4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)
acide 4,9-éthano-9-(4-éthylphényl)-2-[2-(2-méthoxyphényl) propènoyl]-2,3,3a,4,9,9a-hexahydro-1H-benzo[f]isoindole-3a-carboxylique-(3aRS,4SR,9SR, 9aRS)
9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)
9-(2,3-dihydrobenzofuran-5-yl)-4,9-éthano-2-[2-(2-méthoxy phényl)propènoyl)]-2,3,3a,4,9,9a-hexahydro-1H-benzo[f]isoindole-3a-N-(3-pyridylméthyl)carboxamide-(3aRS,4SR,9SR,9aRS)
4,9-éthano-9-(4-fluorophényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 4,9-éthano-9-(4-fluorophényl)--2-[2-(2-méthoxyphényl)propènoyl]-2,3,3a,4,9,9a-hexahydro-1H-benzo[f]isoindole-3a-carboxylique-(3aRS, 4SR,9SR,9aRS)
9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-2-[2-(2-méthoxyphényl)propènoyl]-9-(3-méthylphényl)-2,3,3a,4,9,9a-hexahydro-1H-benzo[f]isoindole-3a-carboxylate de méthyle-(3aRS, 4SR,9SR,9aRS)
acide 4,9-éthano-2-[2-(2-méthoxyphényl)propènoyl]-9-(3-méthylphényl)-2,3,3a,4,9,9a-hexahydro-1H-benzo[f]isoindole-3a-carboxylique-(3aRS, 4SR,9SR,9aRS)
9-(1,3-benzodioxol-5-yl)-4,9-éthano-2-[2-(2-méthoxyphényl)propènoyl]2,3,3a,4,9,9a-hexahydro-1H-benzo[f]isoindole-3a-carboxylate de méthyle-(3aRS, 4SR,9SR,9aRS)
9-(3,4-diméthylphényl)-4,9-éthano-2[2-(2-méthoxy phényl)propènoyl]-2,3,3a,4,9,9a-hexahydro-1H-benzo[f]isoindole-3a-carboxylate de méthyle-(3aRS,4SR,9SR,9aRS)
acide-9-(3,4-diméthylphényl)-4,9-éthano-2[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS, 4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N-(3-pyridylméthyl)-carboxamide-(3aRS,4SR,9SR,9aRS)
4,9-éthano-9-(4-méthoxyphényl)-2-[2-(2-méthoxyphényl)propénoyl]-2,3,3a,4,9,9a-hexahydro-1H-benzo[f]isoindole-3a-N'-phénylcarbohydrazide-(3aRS,4SR,9SR,9aRS)
acide 4,9-éthano-1-9-(4-méthoxyphényl)-2-[2-(2-méthoxyphényl)propènoyl]-2,3,3a,4,9,9a-hexahydro-1H-benzo[f]isoindole-3a-hydroxamique-(3aRS,4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N'-(3-pyridyl)carbohydrazide-(3aRS,4SR,9SR,9aRS)
4,9-éthano-9-(4-méthoxyphényl)-2-[2-(2-méthoxyphényl)propènoyl]-2,3,3a,4,9,9a-hexahydro-1H-benzo[f]isoindole-3a-N-(3-thiénylméthyl)-carboxamide-(3aRS,4SR,9SR,9aRS)
acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétiques-(RS) et (SR)
acide 4,9-éthano-2-[2-(2-méthoxyphényl)propènoyl]-9-(4-trifluorométhoxyphényl)-2,3,3a,4,9,9,9a-hexahydro-1H-benzo[f]isoindole-3a-carboxylique-(3aRS, 4SR, 9SR,9aRS)
9-(3-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)
acide 9-(3-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS)
4,9-éthano-9-(3-fluorophényl-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 4,9-éthano-9-(3-fluorophényl)-2-[2-(2-méthoxyphényl)propènoyl])-2,3,3a,4,9,9a-hexahydro-1H-benzo[f]-isoindole-3a-carboxylique-(3aRS, 4SR, 9SR, 9aRS)
4,9-éthano-9-(3-fluorophényl)-2-[2-(2-méthoxyphényl)propènoyl)]-2,3,3a,4,9,9a-hexahydro-1H-benzo[f]isoindole-3a-[N-(3-pyridylméthyl)carboxamide-(3aRS,4SR,9SR,9aRS)
9-(3-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)
acide 9-(3-chlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)
9-(3-chlorophényl)-4,9-éthano-2-[2-(2-méthoxyphényl)propènoyl)]-2,3,3a,4,9,9a-hexahydro-1H-benzo[f]isoindole 3a-[N-(3-pyridylméthyl)carboxamide-(3aRS,4SR,9SR,9aRS)
9-(3-N,N-diméthylaminophényl)-4,9-éthano-2-[2-(2-méthoxyphényl) propènoyl]-2,3,3a,4,9,9,9a-hexahydro-1H-benzo[f]isoindole-3a-carboxylate de méthyle-(3aRS, 4SR, 9SR,9aRS)
acide 9-(3-N,N-diméthylaminophényl)-4,9-éthano-2-[2-(2-méthoxyphényl) propènoyl]-2,3,3a,4,9,9,9a-hexahydro-1H-benzo[f]isoindole-3a-carboxylique-(3aRS,4SR,9SR, 9aRS)
chlorhydrate de 9-(3-aminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
9-(4-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)
acide 9-(4-cyanophényl)-4,9-éthano-2-[2-(2-méthoxyphényl)propènoyl]-2,3,3a,4,9,9,9a-hexahydro-1H-benzo[f]isoindole-3a-carboxylique-(3aRS,4SR,9SR, 9aRS)
9-(3-cyanophényl)-4,9-éthano-2-[2-(2-méthoxyphényl)propènoyl)-2,3,3a,4,9,9,9a-hexahydro-1H-benzo[f]isoindole-3a-carboxylate de méthyle-(3aRS, 4SR, 9SR,9aRS)
acide 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)
4,9-éthano-5-méthoxy-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR, 9SR,9aRS)
acide 4,9-éthano-5-méthoxy-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)
acide 4,9-éthano-2[2-(2-méthoxyphényl)propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)
l'acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
l'acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 4,8-éthano-2-[2-(2-méthoxyphényl)propènoyl]-8-phényl-2,3,3a,4,8,8a-hexahydro-1H-thiéno[2,3-f]isoindole-3a-carboxylique-(3aRS,4SR,8SR,8aRS)
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)
acide 4,8-éthano-2-[2-(2-méthoxyphényl)propènoyl]-8-(4-trifluorométhylphényl)-2,3,3a,4,8,8a-hexahydro-1H-thiéno[2,3-f]isoindole-3a-carboxylique-(3aRS,4SR,8SR,8aRS)
N-benzyl-4,8-éthano-2-[2-(2-méthoxyphényl)propènoyl]-8-(4-trifluorométhylphényl)-2,3,3a,4,8,8a-hexahydro-1H-thiéno[2,3-f]isoindole-3a-carboxamide-(3aRS,4SR,8SR,8aRS)
acide 4,9-éthano-2-[2-(2-méthoxyphényl)propènoyl]-10-(4-méthylphényl)-2,3,3a,4,10,10a-hexahydro-1H-indolo[2,3-f]isoindole-3a-carboxylique-(3aRS,4SR,10SR,10aRS)
N-(3-pyridyl)méthyl-4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxamide-(3aRS,4SR,8SR,8aRS).
4,8-éthano-8-(4-méthoxyphényl)-2-[2-(2-méthoxyphényl]propènoyl]-2,3,3a,4,8,8a-hexahydro-1H-thiéno[2,3-f]isoindole-3a-carboxylate de méthyle-(3aRS,4SR,8SR,8aRS).
acide 4,8-éthano-8-(4-méthoxyphényl)-2-[2-(2-méthoxyphényl)propènoyl]-2,3,3a,4,8,8a-hexahydro-1H-thiéno[2,3-f]isoindole-3a-carboxylique-(3aRS,4SR,8SR,8aRS).
N-(3-pyridyl)méthyl-8-(benzo-1,4-dioxan-6-yl)-4,8-éthano-2-[2-(2-méthoxyphényl)propènoyl]-2,3,3a,4,8,8a-hexahydro-1H-thiéno[2,3-f]isoindole-3a-carboxamide-(3aRS,4SR,8SR,8aRS).
sous forme de leurs isomères optiques ainsi que leurs sels.

4. Procédé de préparation des composés de formule générale (I) selon l'une quelconque des revendications précédentes, **caractérisé en ce que** les composés de formule générale (I) sont choisis individuellement parmi :
acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)
acide 9-(benzothién-2-yl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS
acide 9-(2,4-dichlorophényl)-4,9-éthano-2-[2-(2-méthoxyphényl)propènoyl)]-2,3,3a,4,9,9a-hexahydro-1H-benzo[f]isoindole-3a-carboxylique-(3aRS,4SR,9SR,9aRS),
acide 9-(3,4-dichlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)
acide 9-(4-bromophényl)-4,9-éthano-2-[2-(2-méthoxyphényl)propènoyl]-2,3,3a,4,9,9a-hexahydro-1H-benzo[f]isoindole-3a-carboxylique -(3aRS, 4SR, 9SR,9aRS)
acide 4,9-éthano-2-[2-(2-méthoxyphényl)propènoyl]-9-(4-chlorophényl)-2,3,3a,4,9,9a-hexahydro-1H-benzo[f]isoindole-3a-carboxylique-(3aRS,4SR,9SR,9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)
acide 4,8-éthano-2-[2-(2-méthoxyphényl)propènoyl]-8-(4-méthylphényl)-2,3,3a,4,8,8a-hexahydro-1H-thiéno[2,3-f]isoindole-3a-carboxylique-(3aRS,4SR,8SR,8aRS)
sous forme de leurs isomères optiques ainsi que leurs sels.

5. Procédé de préparation des composés de formule générale (I) selon la revendication 1, **caractérisé en ce que** les produits de formule générale (I) sont obtenus sous forme de leur énantiomère dextrogyre.

6. Procédé de préparation des composés de formule générale (X') sous forme d'isomères optiques : 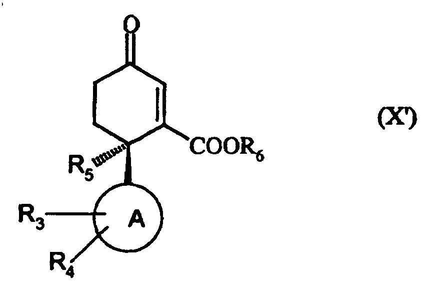 dans laquelle
A, R₃ et R₄, R₅, R₆ sont tels que définis en revendication 1, **caractérisé en ce que** l'on procède à partir des composés de formule générale (X) sous forme de mélange racémique : 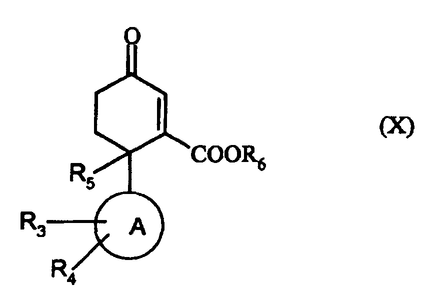 dans un mélange d'un solvant hydrocarboné et d'une solution aqueuse, à pH compris entre 6 et 8, par action d'une enzyme choisie parmi les lipases, estérases, protéases.

7. Procédé selon la revendication 6, **caractérisé en ce que** l'enzyme est choisie parmi les lipases, estérases.

8. Procédé selon la revendication 7, **caractérisé en ce que** les lipases et estérases sont choisies parmi : EC 3.1.1.3 *Candida antartica fraction B* (Chirazyme® L2, Roche Diagnostic, anciennement Boehringer Mannheim), EC 3.1.1.3 *Pseudomonas cepacia* (Lipase PS-C, Amano Enzymes), EC 3.1.1.1 (ESL 01, Diversa, anciennement Recombinant Biocatalyst), EC 3.1.1.3 *Candida antartica fraction B* (SP 525, Novozyme, anciennement Novo Nordisk), EC 3.1.1.3 *Pseudomonas sp.* (Chirazyme® L6, Roche Diagnostic, anciennement Boehringer Mannheim), EC 3.1.1.3 *Rhizomucor miehei* (Esterase 30000, DSM, anciennement Gist Brocades).

9. Procédé selon la revendication 8, **caractérisé en ce que** l'enzyme est EC 3.1.1.3 *Candida antartica fraction B* (Chirazyme® L2, Roche Diagnostic, anciennement Boehringer Mannheim).

10. Procédé selon la revendication 7, **caractérisé en ce que** les protéases et lipases sont choisies parmi: EC 3.4.21.80 *Streptomyces griseus* (Pronase®, Roche Diagnostic, anciennement Boehringer Mannheim), EC 3.4.21.62 *Bacillus licheniformis* (Subtilisin, Novozyme, anciennement Novo Nordisk), EC 3.4.21.62 *Bacillus licheniformis* (Alcalase 2,4 L, Novozyme, anciennement Novo Nordisk), EC 3.4.21.14 *Bacillus subtilis* (Protease Nagarse, Sigma Aldrich), EC 3.4.21.62 *Bacillus licheniformis* (EH 16, Altus Biologies), EC 3.4.21.62 *Bacillus sp*. (EH 15, Altus Biologies), EC 3.1.1.3 *Candida antartica fraction A* (EH 11, Altus Biologies), EC 3.1.1.3 *Mucor javanicus* (Lipase M, Amano Enzymes).

11. Procédé selon la revendication 6, **caractérisé en ce que** le solvant organique est le cyclohexane.

12. Procédé selon la revendication 6, **caractérisé en ce que** la solution aqueuse est une solution d'hydrogénophosphate de potassium.

## Patentansprüche

1. Verfahren zur Herstellung von Verbindungen der allgemeinen Formel I in Form ihrer optischen Isomere: 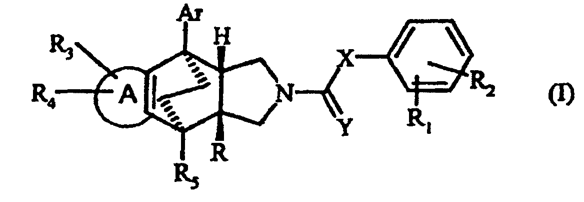 worin:
- A
• entweder für einen mit dem Isoindolkern anellierten Phenylrest steht
und in diesem Fall:
- R₃ und R₄ gleich oder verschieden sind und für ein Wasserstoff- oder Halogenatom oder einen Alkyl-, Hydroxyl-, Alkyloxy-, Alkylcarbonyloxy-, Mercapto-, Alkylthio-, Alkylsulfonyl- oder Alkylsulfinyl-, Amino-, Alkylamino- oder Dialkylamino-, Alkyloxycarbonylamino-, Carboxyl-, Alkyloxycarbonyl-, Carbamoyl-, Alkylcarbamoyloder Dialkylcarbamoyl-, Formyl-, Alkylcarbonyl-, Cyano- oder Trifluormethylrest stehen,
• oder für ein monocyclisches oder kondensiertes bi- oder tricyclisches System steht, wobei jeder gesättigte oder ungesättigte Ring 4 bis 7 Glieder enthält und mindestens einer der Ringe 1 bis 4 gleiche oder verschiedene, unabhängig von einander unter Stickstoff-, Sauerstoff- und Schwefelatomen ausgewählte Heteroatome enthält;
und in diesem Fall R₃ und R₄ für ein Wasserstoffatom stehen,
- Ar für
- einen gegebenenfalls durch ein oder mehrere Atome oder einen oder mehrere Reste, die gleich oder verschieden sind und unter Halogenatomen und Alkylresten mit 1 bis 4 Kohlenstoffatomen, Alkenylresten mit 2 bis 4 Kohlenstoffatomen, Hydroxyl-, Mercapto-, Alkylthio-, Alkylsulfonyl- oder Alkylsulfinyl-, Amino-, Alkylamino- oder Dialkylamino-, Formyl-, Alkylcarbonyl-, Carboxyl-, Alkoxycarbonyl-, Carbamoyl-, Alkylcarbamoyl- oder Dialkylcarbamoyl-, Cyano- oder Trifluormethylresten und Alkoxyresten mit 1 bis 4 Kohlenstoffatomen, deren Alkylteil gegebenenfalls perhalogeniert ist, ausgewählt sind, substituierten Phenylrest,
- einen mit einem 4- bis 7-gliedrigen Heterocyclus mit einem oder mehreren, unter Sauerstoff-, Stickstoff- und Schwefelatomen ausgewählten Heteroatomen kondensierten Phenylrest,
- einen aromatischen oder nicht aromatischen polycylischen Rest oder
- einen 5- bis 12-gliedrigen aromatischen oder nicht aromatischen heterocyclischen Rest, der ein oder mehrere, unter Sauerstoff-, Stickstoff- und Schwefelatomen ausgewählte Heteroatome enthält, über eine Kohlenstoff-Kohlenstoff-Bindung an den kondensierten Ring gebunden ist und gegebenenfalls durch ein oder mehrere Atome oder einen oder mehrere Reste, die gleich oder verschieden sind und unter Halogenatomen und Alkylresten, Alkenylresten mit 2 bis 4 Kohlenstoffatomen, Hydroxylresten, Alkoxyresten mit 1 bis 4 Kohlenstoffatomen, Mercapto-, Alkylthio-, Alkylsulfonyl- oder Alkylsulfinyl-, Amino-, Alkylamino- oder Dialkylamino-, Formyl-, Alkylcarbonyl-, Carboxyl-, Alkoxycarbonyl-, Carbamoyl-, Alkylcarbamoyl- oder Dialkylcarbamoyl-, Cyano- oder Trifluormethylresten ausgewählt sind, substituiert ist, steht,
wobei bei allen diesen Resten Alkyl 1 bis 4 Kohlenstoffatome enthält,
- R für einen Rest der allgemeinen Formel
- (CH₂)ₘ-X₁ - (CH₂)ₙ-Z,
worin
- X₁ für eine Einfachbindung oder ein Sauerstoff- oder Schwefelatom steht,
- m für eine ganze Zahl mit einem Wert von 0 oder 1 steht,
- n für eine ganze Zahl mit einem Wert von 0, 1 oder 2 steht,
- ein oder mehrere Methylenreste durch einen Carboxyl-, Alkoxycarbonyl-, Carbamoyl-, Alkylcarbamoyl-, Dialkylcarbamoyl-, Amino-, Alkylamino- oder Dialkylaminorest substituiert sein können, wobei bei allen diesen Resten Alkyl 1 bis 4 Kohlenstoffatome enthält,
- Z für
- einen Carboxylrest,
- einen Rest COOR₆, worin R₆ einen geradkettigen oder verzweigten Alkylrest mit 1 bis 3 Kohlenstoffatomen bedeutet, oder
- einen Rest der Formel CON(R₇) (R₈), worin
- R₇ ein Wasserstoffatom oder einen geradkettigen oder verzweigten Alkylrest mit 1 bis 6 Kohlenstoffatomen bedeutet und
- R₈
- ein Wasserstoffatom,
- einen Hydroxylrest,
- einen gegebenenfalls durch ein oder mehrere Atome oder einen oder mehrere Reste, die gleich oder verschieden sind und unter Halogenatomen und Alkyl- und Alkyloxyresten ausgewählt sind, wobei bei diesen Resten Alkyl 1 bis 4 Kohlenstoffatome enthält, substituierten Arylsulfonylrest,
- einen 5- bis 7-gliedrigen Heterocyclus, der ein oder mehrere, unter Stickstoff-, Sauerstoff- und Schwefelatomen ausgewählte Heteroatome enthält und über ein Heteroatom gebunden sein kann,
- einen gegebenenfalls durch einen oder zwei Reste, die gleich oder verschieden sind und unter
- Alkylresten mit 1 bis 4 Kohlenstoffatomen,
- gegebenenfalls durch einen oder mehrere Reste, die gleich oder verschieden sind und unter Alkylund Alkyloxyresten ausgewählt sind, wobei bei diesen Resten Alkyl 1 bis 4 Kohlenstoffatome enthält, substituierten Arylresten,
- 5- bis 7-gliedrigen Heterocyclylresten mit einem oder mehreren, unter Stickstoff-, Sauerstoff- und Schwefelatomen ausgewählten Heteroatomen und
- gegebenenfalls durch einen oder mehrere Reste, die gleich oder verschieden sind und unter Alkylund Alkyloxyresten ausgewählt sind, wobei bei diesen Resten Alkyl 1 bis 4 Kohlenstoffatome enthält, substituierten Arylcarbonylresten
ausgewählt sind, substituierten Aminorest,
- einen gegebenenfalls durch einen Phenylrest substituierten, geradkettigen oder verzweigten Alkyloxyrest mit 1 bis 6 Kohlenstoffatomen,
- einen geradkettigen oder verzweigten Alkylrest mit 1 bis 6 Kohlenstoffatomen, wie Methyl, der gegebenenfalls durch einen Aminorest, Alkylaminorest, Dialkylaminorest, Hydroxylrest, Alkoxyrest mit 1 bis 4 Kohlenstoffatomen, einen Mercaptorest, einen Alkylthiorest, einen Alkyloxycarbonylrest, einen Carboxylrest, einen Cyanorest, einen gegebenenfalls substituierten 5- bis 12-gliedrigen mono- oder polycyclischen aromatischen Rest, der gegebenenfalls ein oder mehrere, unter Sauerstoff-, Stickstoff- und Schwefelatomen ausgewählte Heteroatome enthält, substituiert ist, oder einen gegebenenfalls durch ein oder mehrere Halogenatome oder eine oder mehrere Hydroxyl-, Amino- oder Trifluormethylgruppen oder durch einen oder mehrere Alkylreste, Alkenylreste, Alkoxyreste, Alkylthioreste, Alkylaminoreste, Alkylcarbonylreste oder Alkoxycarbonylreste mit 2 bis 4 Kohlenstoffatomen, Carbamoyl-, Alkylcarbamoyl- oder Dialkylcarbamoylreste mit 1 bis 8 Kohlenstoffatomen im Alkylteil oder Formylreste substituierten Phenylrest oder auch einen 1-oder 2-Naphthylrest
bedeutet, steht oder
- Z für
- einen Rest PO(OR₉)₂, worin R₉ ein Wasserstoffatom oder einen geradkettigen oder verzweigten Alkylrest mit 1 bis 6 Kohlenstoffatomen bedeutet, oder
- einen Rest -NH-CO-T, worin T ein Wasserstoffatom oder einen gegebenenfalls durch einen Amino-, Carboxyl-, Alkyloxycarbonyl-, Hydrodxyl-, Alkyloxy-, Mercaptooder Alkylthiorest substituierten, geradkettigen oder verzweigten Alkylrest mit 1 bis 6 Kohlenstoffatomen bedeutet, oder auch
- einen Rest 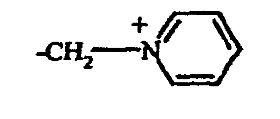 mit einem Anion, wie Trifluormethansulfonat, als Gegenion,
steht,
wobei bei allen bei der Definition von Z vorgeschlagenen Resten mit einer Alkylgruppe Alkyl 1 bis 4 Kohlenstoffatome enthält,
steht,
- R₁ und R₂ gleich oder verschieden sind und für ein Wasserstoffatom, ein Halogenatom oder einen gegebenenfalls durch einen Dialkylaminorest, worin jeder Alkylteil 1 bis 4 Kohlenstoffatome aufweist oder mit dem Stickstoffatom eines 5- oder 6-gliedrigen gesättigten Heterocyclus bildet, substituierten Alkyl- oder Alkyloxyrest, einen Alkylthiorest, einen Alkyloxycarbonylrest stehen oder auch
zueinander in ortho-Stellung stehen und einen gegebenenfalls ungesättigten Heterocyclus, der 1 oder 2, unter Stickstoff und Sauerstoff ausgewählte Heteroatome enthält und gegebenenfalls durch ein Halogenatom oder einen Alkyl- oder Alkyloxyrest substituiert ist, bilden
wobei bei allen bei der Definition von R₁ und R₂ vorgeschlagenen Resten mit einer Alkylgruppe Alkyl 1 bis 4 Kohlenstoffatome enthält,
- R₅ für ein Wasserstoffatom oder einen Alkyloder Alkylthiorest steht, wobei bei der Definition von R₅ Alkyl 1 bis 4 Kohlenstoffatome enthält,
- X für ein Sauerstoff- oder Schwefelatom oder eine -NH-Gruppe, -CO-Gruppe, Methylengruppe, Alken-1,1-diylgruppe, wie Vinyldiyl, oder Cycloalkan-1,1-diylgruppe mit 3 bis 6 Kohlenstoffatomen steht,
und
Y für ein Sauerstoff- oder Schwefelatom steht,
und Salzen des Produkts der allgemeinen Formel (I),
**dadurch gekennzeichnet, daß** man von dem Produkt der allgemeinen Formel (X') in Form eines optischen Isomers: 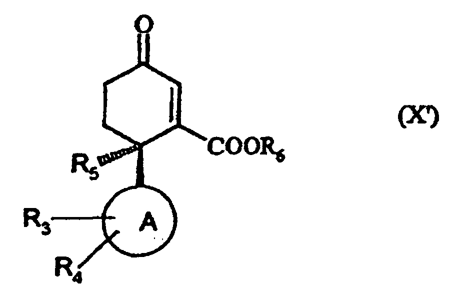 das aus einem Produkt der allgemeinen Formel (X) in racemischer Form: 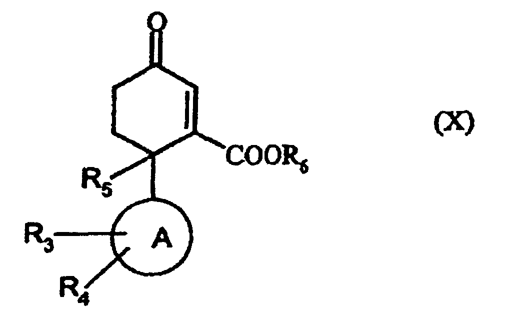 durch Einwirkung eines Enzyms erhalten wird, ausgeht und dann
das Produkt der allgemeinen Formel (X') in Form von optischen Isomeren durch Umsetzung mit einem N-Trialkylsilylmethyl-N-alkoxymethylamin mit einer Schutzgruppe für die Aminfunktion in das Produkt der allgemeinen Formel (IX): 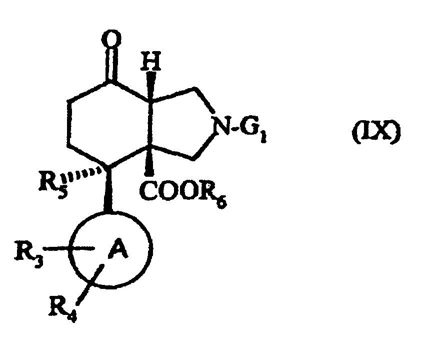 in Form von optischen Isomeren überführt, wobei A, R₃, R₄, R₅ und R₆ die bei der allgemeinen Formel I angegebene Bedeutung besitzen und G₁ für eine Schutzgruppe für die Aminfunktion steht,
wobei man das Produkt der allgemeinen Formel (IX) danach durch:
1. Cyclisierung des Cycloperhydroisoindolkerns unter Bildung der Verbindungen der allgemeinen Formel (III) 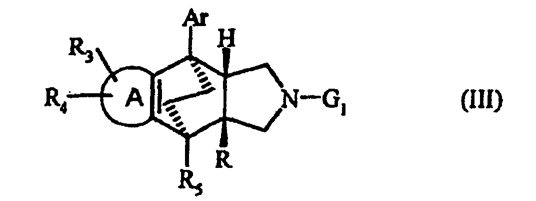 in Form von optischen Isomeren,
wobei:
- A, Ar, R, R₃, R₄ und R₅ die bei der allgemeinen Formel I angegebene Bedeutung besitzen und
- G₁ für ein Wasserstoffatom steht,
über die Bildung eines Zwischenprodukts der allgemeinen Formel (XV) 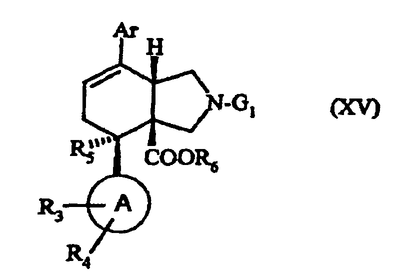 in Form von optischen Isomeren, vorzugsweise des linksdrehenden Isomers, wobei A, R₃, R₄, R₅, R₆, Ar und G₁ die bei Verbindung (IX) angegebene Bedeutung besitzen,
anschließende unterschiedslose Kupplung mit der Seitenkette und gegebenenfalls Modifizierung des Substituenten R zur Bildung der Produkte der allgemeinen Formel (I), oder
2. durch Kupplung der Seitenkette zur Bildung des Produkts der allgemeinen Formel (VIII): 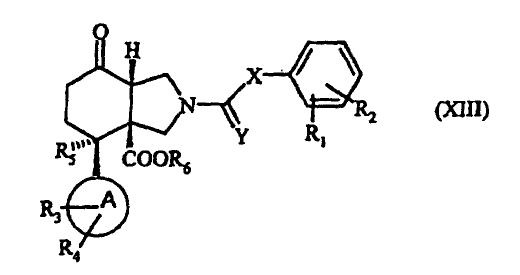 in Form von optischen Isomeren, wobei A, R₁, R₂, R₃, R₄, R₅, R₆, X und Y die oben angegebene Bedeutung besitzen,
anschließende unterschiedslose Cyclisierung des Cycloperhydroisoindolkerns und gegebenenfalls Modifizierung des Substituenten R zur Bildung der Produkte der allgemeinen Formel (I) in Form von optischen Isomeren
in das Produkt der allgemeinen Formel (I) umwandelt.

2. Verfahren zur Herstellung von Verbindungen der allgemeinen Formel (I) nach Anspruch 1, bei dem in der allgemeinen Formel (I):
A
• entweder für einen mit dem Isoindolring anellierten Phenylrest
• oder einen Thienyl- oder Indolylrest steht
und R₃ und R₄ für ein Wasserstoffatom stehen,
Ar für einen 2,3-Dihydro-1,4-benzodioxin-6-yl-, 2,3-Dihydrobenzofuran-5-yl- oder Benzothien-2-ylrest oder einen Phenylrest, der gegebenenfalls in 4-Stellung substituiert ist, vorzugsweise durch einen Methyl-, Trifluormethyl- oder Methoxyrest oder ein Chlor- oder Bromatom, oder in 2,4-Stellung durch ein Chloratom substituiert ist, steht,
R für einen Carboxylrest, einen Rest -COOMe oder auch einen Rest -CON(R₇)(R₈), worin R₈ für einen durch einen 3-Pyridylrest substituierten Methylrest steht, wenn R₇ für Wasserstoff steht,
eines der Symbole R₁ oder R₂ für ein Wasserstoffatom steht und das andere der Symbole für einen Methoxyrest steht und besonders vorteilhaft in ortho-Stellung des Phenylrings gebunden ist,
R₅ für ein Wasserstoffatom oder einen Methylrest steht,
X für eine Methylen- oder Vinyldiylgruppe steht und
Y für ein Sauerstoffatom steht,
in Form ihrer optischen Isomere und ihrer Salze.

3. Verfahren zur Herstellung von Verbindungen der allgemeinen Formel (I) nach einem der vorhergehenden Ansprüche, **dadurch gekennzeichnet, daß** man die Produkte der allgemeinen Formel (I) einzeln unter:
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3acarbonsäuremethylester
(3aRS,4SR,9SR,9aRS)-4,9-Ethano-2-[(2-methoxyphenyl)propenoyl]-9-(4-methylphenyl)-2,3,3a,4,9,9a-hexahydro-1H-benzo[f]isoindol-3acarbonsäure
(3aRS,4SR,9SR,9aRS)-4,9-Ethano-2-[(2-methoxyphenyl)acetyl]-9-(4-methylphenyl)-2,3,3a,4,9,9ahexahydro-1H-benzo[f]isoindol-3a-carbonsäure
(3aRS,4SR,9SR,9aRS)-4,9-Ethano-9-(4-methoxyphenyl)-2-[(2-methoxyphenyl)acetyl]-2,3,3a,4,9,9ahexahydro-1H-benzo[f]isoindol-3a-carbonsäure
(3aRS,4SR,9SR,9aRS)-4,9-Ethano-2-[(2-methoxyphenyl)acetyl]-9-(4-methylsulfanylphenyl)-2,3,3a,4,9,9a-hexahydro-1H-benzo[f]isoindol-3acarbonsäure
(3aRS,4SR,9SR,9aRS)-4,9-Ethano-9-(4-fluorphenyl)-2-[(2-methoxyphenyl)acetyl]-2,3,3a,4,9,9a-hexahydro-1H-benzo[f]isoindol-3a-carbonsäure
(3aRS,4SR,9SR,9aRS)-4,9-Ethano-2-[(2-methoxyphenyl)acetyl]-9-(3-methylphenyl)-2,3,3a,4,9,9ahexahydro-1H-benzo[f]isoindol-3a-carbonsäure
(3aRS,4SR,9SR,9aRS)-4,9-Ethano-9-(3-methoxyphenyl)-2-[(2-methoxyphenyl)acetyl]-2,3,3a,4,9,9ahexahydro-1H-benzo[f]isoindol-3a-carbonsäure
(3aRS,4SR,9SR,9aRS)-9-(3,4-Dimethylphenyl)-4,9-ethano-2-[(2-methoxyphenyl)acetyl]-2,3,3a,4,9,9ahexahydro-1H-benzo[f]isoindol-3a-carbonsäuremethylester
(3aRS,4SR,9SR,9aRS)-9-(3,4-Dimethylphenyl)-4,9-ethano-2-[(2-methoxyphenyl)acetyl]-2,3,3a,4,9,9ahexahydro-1H-benzo[f]isoindol-3a-carbonsäure
(3aRS,4SR,9SR,9aRS)-3a-N-Benzylcarbamoyl-4,9-ethano-2-[2-(2-methoxyphenyl)propenoyl]-9-(4-methylphenyl)-2,3,3a,4,9,9a-hexahydro-1H-benzo[f]-isoindol
(3aRS,4SR,9SR,9aRS)-3a-Carbamoyl-4,9-ethano-2-[2-(2-methoxyphenyl)propenoyl]-9-(4-methylphenyl)-2,3,3a,4,9,9a-hexahydro-1H-benzo[f]isoindol
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3ahydroxamsäurebenzylester
(3aRS,4SR,9SR,9aRS)-4,9-Ethano-2-[2-(2-methoxyphenyl)propenoyl]-9-(4-methylphenyl)-3,3a,4,9,9ahexahydro-1H-benzo[f]isoindol-3a-hydroxamsäure
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N-(3-pyridylmethyl)carboxamid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N-(4-pyridylmethyl)carboxamid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3ahydroxamsäuremethylester
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N',N'-dimethylcarbohydrazid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N'phenylcarbohydrazid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N',N'-pentamethylencarbohydrazid
(3aRS,4SR,9SR,9aRS)-4,9-Ethano-2-[2-(methoxyphenyl)propenoyl]-9-(4-methylphenyl)-3a-phenylsulfonylaminocarbonyl-2,3,3a,4,9,9a-hexahydro-1Hbenzo[f]isoindol
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N-(N-oxo-3pyridyl)methylcarboxamid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N'-(4-methoxyphenyl)carbohydrazid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N'methyl-N'-phenylcarbohydrazid
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N'-(2-methylphenyl)carbohydrazid
(3aRS,4SR,9SR,9aRS)-9-(2,3-Dihydro-1,4-benzodioxin-6-yl)-4,9-ethano-2-[2-(2-methoxyphenyl)-propenoyl]-2,3,3a,4,9,9a-hexahydro-1H-benzo[f]-isoindol-3a-carbonsäuremethylester
(3aRS,4SR,9SR,9aRS)-9-(2,3-Dihydro-1,4-benzodioxin-6-yl)-4,9-ethano-2-[2-(2-methoxyphenyl)-propenoyl]-2,3,3a,4,9,9a-hexahydro-1H-benzo[f]-isoindol-3a-carbonsäure
(3aRS,4SR,9SR,9aRS)-9-(2,3-Dihydro-1,4-benzodioxin-6-yl)-4,9-ethano-2-[2-(2-methoxyphenyl)-propenoyl)]-2,3,3a,4,9,9a-hexahydro-1H-benzo[f]-isoindol-3a-[N-(3-pyridylmethyl)carboxamid
(3aRS,4SR,9SR,9aRS)-9-(2,3-Dihydro-1,4-benzodioxin-6-yl)-4,9-ethano-2-[2-(2-methoxyphenyl)-propenoyl)]-2,3,3a,4,9,9a-hexahydro-1H-benzo[f]-isoindol-3a-N-(2-thienylmethyl)carboxamid
(3aRS,4SR,9SR,9aRS)-4,9-Ethano-2-[(2-methoxyphenyl)acetyl]-9-(3,4,5-trimethylphenyl)-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(2-methylphenyl)-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3acarbonsäuremethylester
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N-(4-pyridylmethyl)carboxamid
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N'benzoylcarbohydrazid
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N'phenylcarbohydrazid
(3aRS,4SR,9SR,9aRS)-4,9-Ethano-2-[2-(2-methoxyphenyl)propenoyl]-9-(2-naphthyl)-2,3,3a,4,9,9ahexahydro-1H-benzo[f]isoindol-3a-carbonsäuremethylester
(3aRS,4SR,9SR,9aRS)-4,9-Ethano-2-[2-(2-methoxyphenyl)propenoyl]-9-(2-naphthyl)-2,3,3a,4,9,9ahexahydro-1H-benzo[f]isoindol-3a-carbonsäure
(3aRS,4SR,9SR,9aRS)-4,9-Ethano-2-[2-(2-methoxyphenyl)propenoyl]-9-(5-methyl-2-thienyl)-2,3,3a,4,9,9a-hexahydro-1H-benzo[f]isoindol-3acarbonsäuremethylester
(3aRS,4SR,9SR,9aRS)-4,9-Ethano-2-[2-(2-methoxyphenyl)propenoyl]-9-(5-methyl-2-thienyl)-2,3,3a,4,9,9a-hexahydro-1H-benzo[f]isoindol-3acarbonsäure
(3aRS,4SR,9SR,9aRS)-9-(4-Bromphenyl)-4,9-ethano-2-[2-(2-methoxyphenyl)propenoyl]-2,3,3a,4,9,9a-hexahydro-1H-benzo[f]isoindol-3a-carbonsäuremethylester
(3aRS,4SR,9SR,9aRS)-9-(4-Bromphenyl)-4,9-ethano-2-[2-(2-methoxyphenyl)propenoyl]-2,3,3a,4,9,9a-hexahydro-1H-benzo[f]isoindol-3a-carbonsäure
(3aRS,4SR,9SR,9aRS)-9-(3,4-Dichlorphenyl)-4,9-ethano-2-[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(4-chlorphenyl)-2,3,3a,4,9,9ahexahydro-2H-benzo[f]isoindol-3a-carbonsäuremethylester
(3aRS,4SR,9SR,9aRS)-9-(4-Chlorphenyl)-4,9-ethano-2-[2-(2-methoxyphenyl)propenoyl]-2,3,3a,4,9,9ahexahydro-1H-benzo[f]isoindol-3a-carbonsäure
(3aRS,4SR,9SR,9aRS)-4,9-Ethano-2-[2-(2-methoxyphenyl)propenoyl]-9-(4-methoxy-3-methylphenyl)-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl)-9-(3-indolyl)-2,3,3a,4,9,9ahexahydro-1H-benzo[f]isoindol-3a-carbonsäuremethylester
(3aRS,4SR,9SR,9aRS)-4,9-Ethano-9-(4-isopropylphenyl)-2-[(2-methoxyphenyl)acetyl]-2,3,3a,4,9,9ahexahydro-1H-benzo[f]isoindol-3a-carbonsäure
(3aRS,4SR,9SR,9aRS)-4,9-Ethano-9-(4-isopropylphenyl)-2-[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(3-thienyl)-2,3,3a,4,9,9ahexahydro-1H-benzo[f]isoindol-3a-carbonsäuremethylester
(3aRS,4SR,9SR,9aRS)-4,9-Ethano-9-(4-ethylphenyl)-2-[2-(2-methoxyphenyl)propenoyl]-2,3,3a,4,9,9ahexahydro-1H-benzo[f]isoindol-3a-carbonsäuremethylester
(3aRS,4SR,9SR,9aRS)-4,9-Ethano-9-(4-ethylphenyl)-2-[2-(2-methoxyphenyl)propenoyl]-2,3,3a,4,9,9ahexahydro-1H-benzo[f]isoindol-3a-carbonsäure
(3aRS,4SR,9SR,9aRS)-9-(2,3-Dihydrobenzofuran-5-yl)-4,9-ethano-2-[2-(2-methoxyphenyl)propenoyl]-2,3,3a,4,9,9a-hexahydro-1H-benzo[f]isoindol-3acarbonsäuremethylester
(3aRS,4SR,9SR,9aRS)-9-(2,3-Dihydrobenzofuran-5-yl)-4,9-ethano-2-[2-(2-methoxyphenyl)propenoyl]-2,3,3a,4,9,9a-hexahydro-1H-benzo[f]isoindol-3acarbonsäure
(3aRS,4SR,9SR,9aRS)-9-(2,3-Dihydrobenzofuran-5-yl)-4,9-ethano-2-[2-(2-methoxyphenyl)propenoyl)]-2,3,3a,4,9,9a-hexahydro-1H-benzo[f]isoindol-3a-N-(3-pyridylmethyl)carboxamid
(3aRS,4SR,9SR,9aRS)-4,9-Ethano-9-(4-fluorphenyl)-2-[2-(2-methoxyphenyl)propenoyl]-2,3,3a,4,9,9ahexahydro-1H-benzo[f]isoindol-3a-carbonsäuremethylester
(3aRS,4SR,9SR,9aRS)-4,9-Ethano-9-(4-fluorphenyl)-2-[2-(2-methoxyphenyl)propenoyl]-2,3,3a,4,9,9ahexahydro-1H-benzo[f]isoindol-3a-carbonsäure
(3aRS,4SR,9SR,9aRS)-9-(4-Chlor-3-fluorphenyl)-4,9-ethano-2-[2-(2-methoxyphenyl)propenoyl]-2,3,3a,4,9,9a-hexahydro-1H-benzo[f]isoindol-3acarbonsäuremethylester
(3aRS,4SR,9SR,9aRS)-9-(4-Chlor-3-fluorphenyl)-4,9-ethano-2-[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-2-[2-(2-methoxyphenyl)propenoyl]-9-(3-methylphenyl)-2,3,3a,4,9,9a-hexahydro-1H-benzo[f]isoindol-3acarbonsäuremethylester
(3aRS,4SR,9SR,9aRS)-4,9-Ethano-2-[2-(2-methoxyphenyl)propenoyl]-9-(3-methylphenyl)-2,3,3a,4,9,9a-hexahydro-1H-benzo[f]isoindol-3acarbonsäure
(3aRS,4SR,9SR,9aRS)-9-(1,3-Benzodioxol-5-yl)-4,9-ethano-2-[2-(2-methoxyphenyl)propenoyl]-2,3,3a,4,9,9a-hexahydro-1H-benzo[f]isoindol-3acarbonsäuremethylester
(3aRS,4SR,9SR,9aRS)-9-(3,4-Dimethylphenyl)-4,9-ethano-2[2-(2-methoxyphenyl)propenoyl]-2,3,3a,4,9,9a-hexahydro-1H-benzo[f]isoindol-3acarbonsäuremethylester
(3aRS,4SR,9SR,9aRS)-9-(3,4-Dimethylphenyl)-4,9-ethano-2[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3acarbonsäuremethylester
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3a-N-(3-pyridylmethyl)carboxamid
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3a-N'phenylcarbohydrazid
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3ahydroxamsäure
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3a-N'-(3-pyridyl)carbohydrazid
(3aRS,4SR,9SR,9aRS)-4,9-Ethano-9-(4-methoxyphenyl)-2-[2-(2-methoxyphenyl)propenoyl]-2,3,3a,4,9,9a-hexahydro-1H-benzo[f]isoindol-3a-N-(3-thienylmethyl)carboxamid
(RS)- und (SR)-2-{(3aRS,4SR,9SR,9aRS)-4,9-Ethano-9-(4-methoxyphenyl)-2-[2-(2-methoxyphenyl)-propenoyl]-2,3,3a,4,9,9a-hexahydro-1H-benzo[f]-isoindol-3a-carbonylamino}phenylessigsäure
(3aRS,4SR,9SR,9aRS)-4,9-Ethano-2-[2-(2-methoxyphenyl)propenoyl]-9-(4-trifluormethoxyphenyl)-2,3,3a,4,9,9,9a-hexahydro-1H-benzo[f]isoindol-3acarbonsäure
(3aRS,4SR,9SR,9aRS)-9-(3-Bromphenyl)-4,9-ethano-2-[2-(2-methoxyphenyl)propenoyl]-2,3,3a,4,9,9ahexahydro-1H-benzo[f]isoindol-3a-carbonsäuremethylester
(3aRS,4SR,9SR,9aRS)-9-(3-Bromphenyl)-4,9-ethano-2-[2-(2-methoxyphenyl)propenoyl]-2,3,3a,4,9,9ahexahydro-1H-benzo[f]isoindol-3a-carbonsäure
(3aRS,4SR,9SR,9aRS)-4,9-Ethano-9-(3-fluorphenyl-2-[2-(2-methoxyphenyl)propenoyl]-2,3,3a,4,9,9ahexahydro-1H-benzo[f]isoindol-3a-carbonsäuremethylester
(3aRS,4SR,9SR,9aRS)-4,9-Ethano-9-(3-fluorphenyl)-2-[2-(2-methoxyphenyl)propenoyl])-2,3,3a,4,9,9ahexahydro-1H-benzo[f]isoindol-3a-carbonsäure
(3aRS,4SR,9SR,9aRS)-4,9-Ethano-9-(3-fluorphenyl)-2-[2-(2-methoxyphenyl)propenoyl)]-2,3,3a,4,9,9ahexahydro-1H-benzo[f]isoindol-3a-[N-(3-pyridylmethyl)carboxamid
(3aRS,4SR,9SR,9aRS)-9-(3-Chlorphenyl)-4,9-ethano-2-[2-(2-methoxyphenyl)propenoyl]-2,3,3a,4,9,9ahexahydro-1H-benzo[f]isoindol-3a-carbonsäuremethylester
(3aRS,4SR,9SR,9aRS)-9-(3-Chlorphenyl)-4,9-ethano-2-[2-(2-methoxyphenyl)propenoyl]-2,3,3a,4,9,9ahexahydro-1H-benzo[f]isoindol-3a-carbonsäure
(3aRS,4SR,9SR,9aRS)-9-(3-Chlorphenyl)-4,9-ethano-2-[2-(2-methoxyphenyl)propenoyl)]-2,3,3a,4,9,9ahexahydro-1H-benzo[f]isoindol-3a-[N-(3-pyridylmethyl)carboxamid
(3aRS,4SR,9SR,9aRS)-9-(3-N,N-Dimethylaminophenyl)-4,9-ethano-2-[2-(2-methoxyphenyl)propenoyl]-2,3,3a,4,9,9,9a-hexahydro-1H-benzo[f]isoindol-3acarbonsäuremethylester
(3aRS,4SR,9SR,9aRS)-9-(3-N,N-Dimethylaminophenyl)-4,9-ethano-2-[2-(2-methoxyphenyl)propenoyl]-2,3,3a,4,9,9,9a-hexahydro-1H-benzo[f]isoindol-3acarbonsäure
(3aRS,4SR,9SR,9aRS)-9-(3-Aminophenyl)-4,9-ethano-2-[2-(2-methoxyphenyl)propenoyl]-2,3,3a,4,9,9,9ahexahydro-1H-benzo[f]isoindol-3a-carbonsäuremethylester-hydrochlorid
(3aRS,4SR,9SR,9aRS)-9-(4-N,N-Dimethylaminophenyl)-4,9-ethano-2-[2-(2-methoxyphenyl)propenoyl]-2,3,3a,4,9,9,9a-hexahydro-1H-benzo[f]isoindol-3acarbonsäuremethylester
(3aRS,4SR,9SR,9aRS)-9-(4-Cyanophenyl)-4,9-ethano-2-[2-(2-methoxyphenyl)propenoyl]-2,3,3a,4,9,9,9ahexahydro-1H-benzo[f]isoindol-3a-carbonsäure
(3aRS,4SR,9SR,9aRS)-9-(3-Cyanophenyl)-4,9-ethano-2-[2-(2-methoxyphenyl)propenoyl]-2,3,3a,4,9,9,9ahexahydro-1H-benzo[f]isoindol-3acarbonsäuremethylester
(3aRS,4SR,9SR,9aRS)-4,9-Ethano-9-(3-hydroxy-4-methoxyphenyl)-2-[2-(2-methoxyphenyl)propenoyl]-2,3,3a,4,9,9a-hexahydro-1H-benzo[f]isoindol-3acarbonsäure
(3aRS,4SR,9SR,9aRS)-4,9-Ethano-5-methoxy-2-[2-(2-methoxyphenyl)propenoyl]-9-(4-methylphenyl)-2,3,3a,4,9,9a-hexahydro-1H-benzo[f]isoindol-3acarbonsäuremethylester
(3aRS,4SR,9SR,9aRS)-4,9-Ethano-5-methoxy-2-[2-(2-methoxyphenyl)propenoyl]-9-(4-methylphenyl)-2,3,3a,4,9,9a-hexahydro-1H-benzo[f]isoindol-3acarbonsäure
(3aRS,4SR,9SR,9aRS)-4,9-Ethano-2[2-(2-methoxyphenyl)propenoyl]-4-methyl-9-(4-methoxyphenyl)-2,3,3a,4,9,9a-hexahydro-1H-benzo[f]isoindol-3acarbonsäure
(3aRS,4SR,9SR,9aRS)-9-(Benzothien-2-yl)-4,9-ethano-2-[2-(2-methoxyphenyl)propenoyl)]-2,3,3a,4,9,9a-hexahydro-1H-benzo[f]isoindol-3acarbonsäure
(3aRS,4SR,9SR,9aRS)-9-(2,4-Dichlorphenyl)-4,9-ethano-2-[2-(2-methoxyphenyl)propenoyl)]-2,3,3a,4,9,9a-hexahydro-1H-benzo[f]isoindol-3acarbonsäure
(3aRS,4SR,8SR,8aRS)-4,8-Ethano-2-[2-(2-methoxyphenyl)propenoyl]-8-phenyl-2,3,3a,4,8,8ahexahydro-1H-thieno[2,3-f]isoindol-3a-carbonsäure
(3aRS,4SR,8SR,8aRS)-4,8-Ethano-2-[2-(2-methoxyphenyl)propenoyl]-8-(4-methylphenyl)-2,3,3a,4,8,8a-hexahydro-1H-thieno[2,3-f]isoindol-3a-carbonsäure
(3aRS,4SR,8SR,8aRS)-4,8-Ethano-2-[2-(2-methoxyphenyl)propenoyl]-8-(4-trifluormethylphenyl)-2,3,3a,4,8,8a-hexahydro-1H-thieno[2,3-f]isoindol-3a-carbonsäure
(3aRS,4SR,8SR,8aRS)-N-Benzyl-4,8-ethano-2-[2-(2-methoxyphenyl)propenoyl]-8-(4-trifluormethylphenyl)-2,3,3a,4,8,8a-hexahydro-1H-thieno[2,3-f]-isoindol-3a-carboxamid
(3aRS,4SR,10SR,10aRS)-4,9-Ethano-2-[2-(2-methoxyphenyl)propenoyl]-10-(4-methylphenyl)-2,3,3a,4,10,10a-hexahydro-1H-indolo[2,3-f]-isoindol-3a-carbonsäure
(3aRS,4SR,8SR,8aRS)-N-(3-Pyridyl)methyl-4,8-ethano-2-[2-(2-methoxyphenyl)propenoyl]-8-(4-methylphenyl)-2,3,3a,4,8,8a-hexahydro-1H-thieno-[2,3-f]isoindol-3a-carboxamid
(3aRS,4SR,8SR,8aRS)-4,8-Ethano-8-(4-methoxyphenyl)-2-[2-(2-methoxyphenyl)propenoyl]-2,3,3a,4,8,8a-hexahydro-1H-thieno[2,3-f]isoindol-3a-carbonsäuremethylester
(3aRS,4SR,8SR,8aRS)-4,8-Ethano-8-(4-methoxyphenyl)-2-[2-(2-methoxyphenyl)propenoyl]-2,3,3a,4,8,8a-hexahydro-1H-thieno[2,3-f]isoindol-3a-carbonsäure
(3aRS,4SR,8SR,8aRS)-N-(3-Pyridyl)methyl-8-(benzo-1,4-dioxan-6-yl)-4,8-ethano-2-[2-(2-methoxyphenyl)propenoyl]-2,3,3a,4,8,8a-hexahydro-1Hthieno[2,3-f]isoindol-3a-carboxamid
in Form ihrer optischen Isomere und ihrer Salze auswählt.

4. Verfahren zur Herstellung von Verbindungen der allgemeinen Formel (I) nach einem der vorhergehenden Ansprüche, **dadurch gekennzeichnet, daß** man die Verbindungen der allgemeinen Formel (I) einzeln unter:
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3acarbonsäure
(3aRS,4SR,9SR,9aRS)-9-(Benzothien-2-yl)-4,9-ethano-2-[2-(2-methoxyphenyl)propenoyl)]-2,3,3a,4,9,9a-hexahydro-1H-benzo[f]isoindol-3acarbonsäure
(3aRS,4SR,9SR,9aRS)-9-(2,4-Dichlorphenyl)-4,9-ethano-2-[2-(2-methoxyphenyl)propenoyl)]-2,3,3a,4,9,9a-hexahydro-1H-benzo[f]isoindol-3acarbonsäure
(3aRS,4SR,9SR,9aRS)-9-(3,4-Dichlorphenyl)-4,9-ethano-2-[2-(2-methoxyphenyl)propenoyl]-2,3,3a,4,9,9a-hexahydro-1H-benzo[f]isoindol-3acarbonsäure
(3aRS,4SR,9SR,9aRS)-9-(4-Bromphenyl)-4,9-ethano-2-[2-(2-methoxyphenyl)propenoyl]-2,3,3a,4,9,9ahexahydro-1H-benzo[f]isoindol-3a-carbonsäure
(3aRS,4SR,9SR,9aRS)-4,9-Ethano-2-[2-(2-methoxyphenyl)propenoyl]-9-(4-chlorphenyl)-2,3,3a,4,9,9a-hexahydro-1H-benzo[f]isoindol-3acarbonsäure
(3aRS,4SR,9SR,9aRS)-9-(2,3-Dihydrobenzofuran-5-yl)-4,9-ethano-2-[2-(2-methoxyphenyl)propenoyl]-2,3,3a,4,9,9a-hexahydro-1H-benzo[f]isoindol-3acarbonsäuremethylester
(3aRS,4SR,8SR,8aRS)-4,8-Ethano-2-[2-(2-methoxyphenyl)propenoyl]-8-(4-methylphenyl)-2,3,3a,4,8,8a-hexahydro-1H-thieno[2,3-f]isoindol-3a-carbonsäure
in Form ihrer optischen Isomere und ihrer Salze auswählt.

5. Verfahren zur Herstellung von Verbindungen der allgemeinen Formel (I) nach Anspruch 1, **dadurch gekennzeichnet, daß** man die Produkte der allgemeinen Formel (I) in Form ihres rechtsdrehenden Enantiomers erhält.

6. Verfahren zur Herstellung von Verbindungen der Formel (X') in Form von optischen Isomeren: 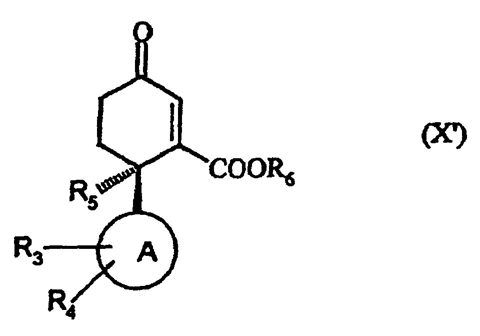 worin
A, R₃, R₄, R₅ und R₆ die in Anspruch 1 angegebene Bedeutung besitzen, **dadurch gekennzeichnet, daß** man auf Verbindungen der allgemeinen Formel (X) in Form eines racemischen Gemischs: 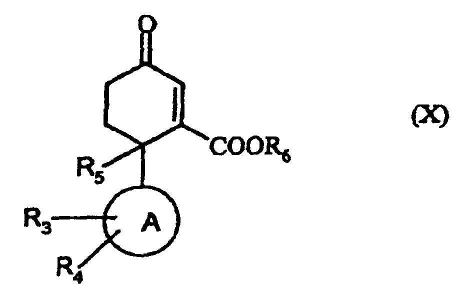 in einem Gemisch aus einem KohlenwasserstoffLösungsmittel und einer wäßrigen Lösung mit einem pH-Wert zwischen 6 und 8 ein unter Lipasen, Esterasen und Proteasen ausgewähltes Enzym einwirken läßt.

7. Verfahren nach Anspruch 6, **dadurch gekennzeichnet, daß** man das Enzym unter Lipasen und Esterasen auswählt.

8. Verfahren nach Anspruch 7, **dadurch gekennzeichnet, daß** man die Lipasen und Esterasen auswählt unter: EC 3.1.1.3 Candida antartica *fraction B* (Chirazyme® L2, Roche Diagnostic, früher Boehringer Mannheim), EC 3.1.1.3 Pseudomonas cepacia (Lipase PS-C, Amano Enzymes), EC 3.1.1.1 (ESL 01, Diversa, früher Recombinant Biocatalyst), EC 3.1.1.3 *Candida antartica fraction B* (SP 525, Novozyme, früher Novo Nordisk), EC 3.1.1.3 *Pseudomonas sp.* (Chirazyme® L6, Roche Diagnostic, früher Boehringer Mannheim), EC 3.1.1.3 *Rhizomucor miehei* (Esterase 30000, DSM, früher Gist Brocades).

9. Verfahren nach Anspruch 8, **dadurch gekennzeichnet, daß** es sich bei dem Enzym um EC 3.1.1.3 *Candida antartica fraction B* (Chirazyme® L2, Roche Diagnostic, früher Boehringer Mannheim) handelt.

10. Verfahren nach Anspruch 7, **dadurch gekennzeichnet, daß** man die Proteasen und Lipasen auswählt unter: EC 3.4.21.80 *Streptomyces griseus* (Pronase®, Roche Diagnostic, früher Boehringer Mannheim), EC 3.4.21.62 *Bacillus licheniformis* (Subtilisin, Novozyme, früher Novo Nordisk), EC 3.4.21.62 *Bacillus licheniformis* (Alcalase 2,4 L, Novozyme, früher Novo Nordisk), EC 3.4.21.14 *Bacillus subtilis* (Protease Nagarse, Sigma Aldrich), EC 3.4.21.62 *Bacillus licheniformis* (EH 16, Altus Biologics), EC 3.4.21.62 *Bacillus sp.* (EH 15, Altus Biologics), EC 3.1.1.3 *Candida antartica fraction A* (EH 11, Altus Biologics), EC 3.1.1.3 *Mucor javanicus* (Lipase M, Amano Enzymes).

11. Verfahren nach Anspruch 6, **dadurch gekennzeichnet, daß** es sich bei dem organischen Lösungsmittel um Cyclohexan handelt.

12. Verfahren nach Anspruch 6, **dadurch gekennzeichnet, daß** es sich bei der wäßrigen Lösung um eine Kaliumhydrogenphosphatlösung handelt.

## Claims

1. Process for the preparation of the compounds of general formula I, in the form of their optical isomers: 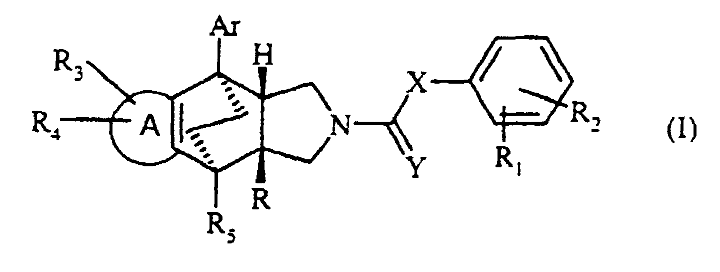 in which:
- A represents:
• either a phenyl radical fused with the isoindole nucleus,
and in this case:
- R₃ and R₄, which are identical or different, represent a hydrogen or halogen atom or an alkyl, hydroxyl, alkyloxy, alkylcarbonyloxy, mercapto, alkylthio, alkylsulphonyl or alkylsulphinyl, amino, alkylamino or dialkylamino, alkyloxycarbonylamino, carboxyl, alkyloxycarbonyl, carbamoyl, alkylcarbamoyl or dialkylcarbamoyl, formyl, alkylcarbonyl, cyano or trifluoromethyl radical,
• or A represents a monocyclic or condensed bi- or tricyclic system in which each saturated or unsaturated ring comprises from 4 to 7 members and in which at least one of the rings comprises from 1 to 4 identical or different heteroatoms chosen independently from nitrogen, oxygen and sulphur atoms;
and in this case R₃ and R₄ represent a hydrogen atom,
- Ar represents
- a phenyl radical optionally substituted by one or more atoms or radicals, which are identical or different, chosen from halogen atoms and the following radicals: alkyl comprising 1 to 4 carbon atoms, alkenyl comprising 2 to 4 carbon atoms, hydroxyl, mercapto, alkylthio, alkylsulphonyl or alkylsulphinyl, amino, alkylamino or dialkylamino, formyl, alkylcarbonyl, carboxyl, alkoxycarbonyl, carbamoyl, alkylcarbamoyl or dialkylcarbamoyl, cyano or trifluoromethyl, or alkoxy comprising 1 to 4 carbon atoms, the alkyl portion of which is optionally perhalogenated, or
- a phenyl radical condensed with a 4- to 7-membered heterocycle comprising one or more heteroatoms chosen from oxygen, nitrogen and sulphur atoms, or
- a polycyclic aromatic or nonaromatic radical,
- a 5- to 12-membered heterocyclic aromatic or nonaromatic radical incorporating one or more heteroatoms chosen from oxygen, nitrogen and sulphur atoms, bonded to the condensed ring via a carbon-carbon bond, said radical being substituted, if appropriate, by one or more atoms or radicals, which are identical or different, chosen from halogen atoms and the following radicals: alkyl, alkenyl comprising 2 to 4 carbon atoms, hydroxyl, alkoxy comprising 1 to 4 carbon atoms, mercapto, alkylthio, alkylsulphonyl or alkylsulphinyl, amino, alkylamino or dialkylamino, formyl, alkylcarbonyl, carboxyl, alkoxycarbonyl, carbamoyl, alkylcarbamoyl or dialkylcarbamoyl, cyano or trifluoromethyl,
with, for all of these radicals, alkyl comprising 1 to 4 carbon atoms,
- R represents
a radical of general formula
- (CH₂)ₘ-X₁-(CH₂)ₙ-Z
in which
- X₁ represents a single bond or an oxygen or sulphur atom,
- m represents an integer equal to 0 or 1,
- n represents an integer equal to 0, 1 or 2,
- one or more methylene radicals can be substituted by a carboxyl, alkoxycarbonyl, carbamoyl, alkylcarbamoyl, dialkylcarbamoyl, amino, alkylamino or dialkylamino radical with, for all of these radicals, alkyl comprising 1 to 4 carbon atoms,
- Z represents
- a carboxyl radical,
- a COOR₆ radical, in which R₆ represents a straight or branched alkyl radical comprising 1 to 3 carbon atoms, or
- a radical of formula CON(R₇) (R₈) in which
- R₇ represents a hydrogen atom or a straight or branched alkyl radical comprising 1 to 6 carbon atoms and
- R₈ represents
- a hydrogen atom,
- a hydroxyl radical,
- an arylsulphonyl radical, optionally substituted by one or more atoms or radicals, which are identical or different, chosen from halogen atoms and alkyl or alkyloxy radicals with, for all of these radicals, alkyl comprising 1 to 4 carbon atoms,
- a 5- to 7-membered heterocycle incorporating one or more heteroatoms chosen from nitrogen, oxygen or sulphur atoms, it being possible for said heterocycle to be bonded via a heteroatom,
- an amino radical optionally substituted by one or two radicals, which are identical or different, chosen from the following radicals:
- alkyl comprising 1 to 4 carbon atoms,
- aryl, optionally substituted by one or more radicals, which are identical or different, chosen from alkyl or alkyloxy radicals with, for these radicals, alkyl comprising 1 to 4 carbon atoms,
- 5- to 7-membered heterocyclyl comprising one or more heteroatoms chosen from nitrogen, oxygen and sulphur atoms,
- arylcarbonyl, optionally substituted by one or more radicals, which are identical or different, chosen from alkyl or alkyloxy radicals with, for these radicals, alkyl comprising 1 to 4 carbon atoms,
- an alkyloxy radical comprising 1 to 6 straight- or branched-chain carbon atoms optionally substituted by a phenyl radical,
- a straight or branched alkyl radical comprising 1 to 6 carbon atoms, such as methyl, optionally substituted by an amino, alkylamino, dialkylamino, hydroxyl, alkoxy comprising 1 to 4 carbon atoms, mercapto, alkylthio, alkyloxycarbonyl, carboxyl or cyano radical, an optionally substituted mono- or polycyclic aromatic radical having from 5 to 12 ring members which may or may not incorporate one or more heteroatoms chosen from oxygen, nitrogen and sulphur atoms, it being possible for said aromatic radical to be a phenyl radical optionally substituted by one or more halogen atoms or by one or more hydroxyl, amino or trifluoromethyl groups or by one or more alkyl or alkenyl, alkoxy, alkylthio, alkylamino, alkylcarbonyl or C2 to C4 alkoxycarbonyl, carbamoyl, alkylcarbamoyl or dialkylcarbamoyl, the alkyl part of which comprises 1 to 8 carbon atoms, or formyl radicals, or alternatively a 1- or 2-naphthyl radical, or,
- Z represents
- a PO(OR₉)₂ radical in which R₉ represents a hydrogen atom or a straight or branched alkyl radical comprising 1 to 6 carbon atoms, or
- an -NH-CO-T radical in which T represents a hydrogen atom or a straight or branched alkyl radical comprising 1 to 6 carbon atoms optionally substituted by an amino, carboxyl, alkyloxycarbonyl, hydroxyl, alkyloxy, mercapto or alkylthio radical, or alternatively
- a 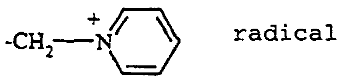 having an anion, such as trifluoromethanesulphonate, as counterion,
with, for all of the radicals possessing an alkyl group provided in the definition of Z, alkyl comprising 1 to 4 carbon atoms,
- R₁ and R₂, which are identical or different, represent a hydrogen atom, a halogen atom or an alkyl radical, an alkyloxy radical, each optionally substituted by a dialkylamino radical, each alkyl part of which comprises 1 to 4 carbon atoms or forms, with the nitrogen atom, a saturated heterocycle containing 5 or 6 ring members, an alkylthio radical or an alkyloxycarbonyl radical, or alternatively
situated at the ortho position with respect to one another, R₁ and R₂ form a saturated or unsaturated heterocycle comprising 1 or 2 heteroatoms chosen from nitrogen and oxygen, optionally substituted by a halogen atom or by an alkyl or alkyloxy radical,
with, for all of the radicals possessing an alkyl group provided in the definition of R₁ and R₂, alkyl comprising 1 to 4 carbon atoms,
- R₅ represents a hydrogen atom or an alkyl radical or an alkylthio radical, with, for the definition of R₅, alkyl comprising 1 to 4 carbon atoms;
- X represents an oxygen or sulphur atom or one of the following groups: -NH-, -CO-, methylene, alken-1,1-diyl, such as vinyldiyl, or cycloalkan-1,1-diyl comprising 3 to 6 carbon atoms,
and
- Y represents an oxygen or sulphur atom,
and the salts of the product of general formula (I),
**characterized in that** the starting material is the product of general formula (X') in the form of an optical isomer: 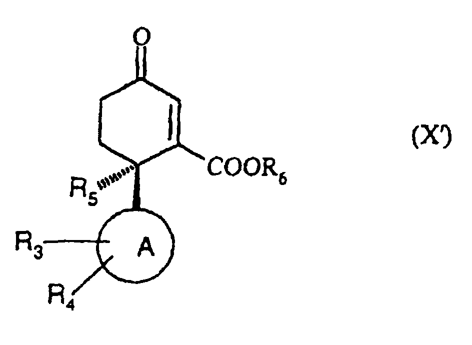 which is obtained from the product of general formula (X) in the racemic form: 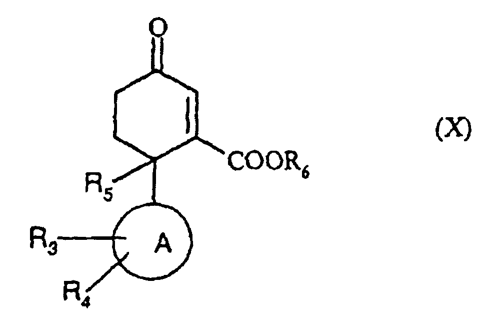 by the action of an enzyme,
and then the product of general formula (X'), in the form of optical isomers, is converted to the product of general formula (IX): 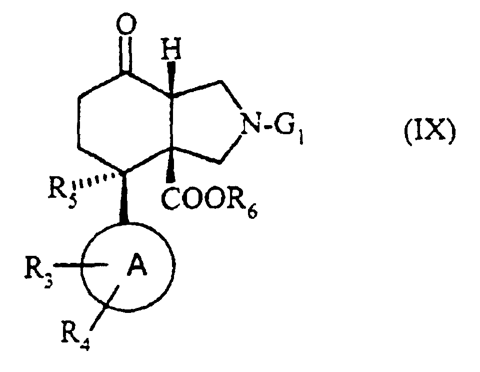 in the form of optical isomers, in which formula A, R₃, R₄, R₅ and R₆ are as defined in general formula I and G₁ represents a protective group for the amine functional group, by reaction with an N-trialkylsilylmethyl-N-(alkoxymethyl)amine carrying a protective group for the amine functional group,
the product of general formula (IX) subsequently being converted to the product of general formula (I):
1. by cyclization of the cycloperhydroisoindole nucleus, in order to form the compounds of general formula (III) 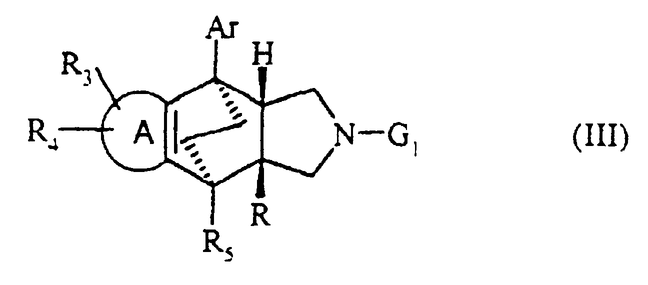 in the form of optical isomers,
in which formula:
- A, Ar, R, R₃, R₄ and R₅ are defined according to the general formula I and
- G₁ represents a hydrogen atom,
via the formation of an intermediate of general formula (XV) 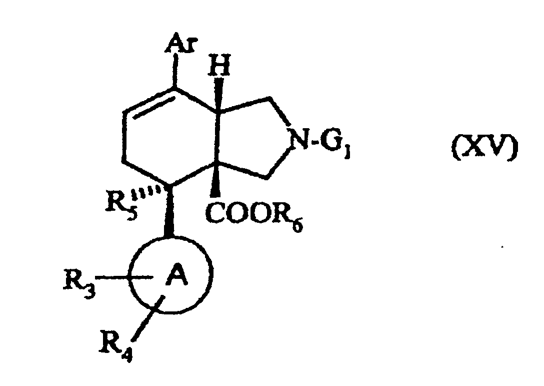 in the form of optical isomers, preferably the laevorotatory isomer, in which formula A, R₃, R₄, R₅, R₆, Ar and G₁ are as defined for the compound (IX),
and then, without distinction, by coupling with the side chain and optionally modification of the R substituent, in order to form the products of general formula (I), or
2. by coupling of the side chain, in order to form the product of general formula (VIII): 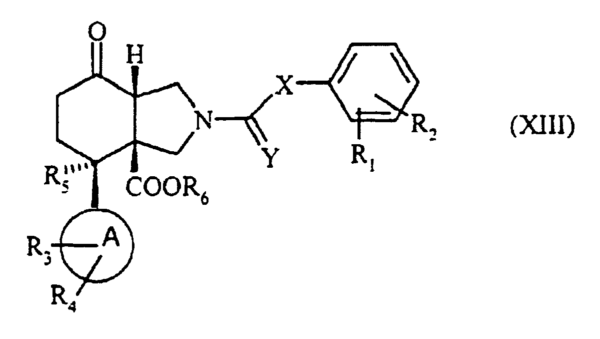 in the form of optical isomers, in which formula A, R₁, R₂, R₃, R₄, R₅, R₆, X and Y are defined as above, and then, without distinction, by cyclization of the cycloperhydroisoindole nucleus and optionally modification of the R substituent, in order to form the products of general formula (I) in the form of optical isomers.

2. Process for the preparation of the compounds of general formula (I) according to Claim 1, in which process, in the general formula (I):
A represents
• either a phenyl radical fused to the isoindole ring
• or a thienyl or indolyl radical;
and R₃ and R₄ represent a hydrogen atom,
Ar represents a 2,3-dihydro-1,4-benzodioxin-6-yl or 2,3-dihydrobenzofuran-5-yl or benzothien-2-yl radical or a phenyl radical optionally substituted at the 4 position, preferably by a methyl, trifluoromethyl or methoxy radical or a chlorine or bromine atom, or at the 2, 4 position by a chlorine atom;
R represents a carboxyl radical or a -COOMe radical or alternatively a -CON(R₇) (R₈) radical in which, when R₇ represents a hydrogen atom, R₈ represents a methyl radical substituted by the 3-pyridyl radical,
one of the R₁ or R₂ symbols represents a hydrogen atom and the other of the symbols represents a methoxy radical, more advantageously attached at the ortho position of the phenyl ring,
R₅ represents a hydrogen atom or a methyl radical,
X represents a methylene or vinyldiyl group,
Y represents an oxygen atom;
in the form of their optical isomers, as well as their salts.

3. Process for the preparation of the compounds of general formula (I) according to either one of the preceding claims, **characterized in that** the products of general formula (I) are individually chosen from:
methyl (3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-carboxylate
(3aRS,4SR,9SR,9aRS)-4,9-ethano-2-[2-(2-methoxyphenyl)-propenoyl]-9-(4-methylphenyl)-2,3,3a,4,9,9a-hexahydro-1H-benzo[f]isoindole-3a-carboxylic acid
(3aRS,4SR,9SR,9aRS)-4,9-ethano-2-[(2-methoxyphenyl)-acetyl]-9-(4-methylphenyl)-2,3,3a,4,9,9a-hexahydro-1H-benzo[f]isoindole-3a-carboxylic acid
(3aRS,4SR,9SR,9aRS)-4,9-ethano-9-(4-methoxyphenyl)-2-[(2-methoxyphenyl)acetyl]-2,3,3a,4,9,9a-hexahydro-1H-benzo[f]isoindole-3a-carboxylic acid
(3aRS,4SR,9SR,9aRS)-4,9-ethano-2-[(2-methoxyphenyl)-acetyl]-9-(4-methylsulphanylphenyl)-2,3,3a,4,9,9a-hexahydro-1H-benzo[f]isoindole-3a-carboxylic acid
(3aRS,4SR,9SR,9aRS)-4,9-ethano-9-(4-fluorophenyl)-2-[(2-methoxyphenyl)acetyl]-2,3,3a,4,9,9a-hexahydro-1H-benzo[f]isoindole-3a-carboxylic acid
(3aRS,4SR,9SR,9aRS)-4,9-ethano-2-[(2-methoxyphenyl)-acetyl]-9-(3-methylphenyl)-2,3,3a,4,9,9a-hexahydro-1H-benzo[f]isoindole-3a-carboxylic acid
(3aRS,4SR,9SR,9aRS)-4,9-ethano-9-(3-methoxyphenyl)-2-[(2-methoxyphenyl)acetyl]-2,3,3a,4,9,9a-hexahydro-1H-benzo[f]isoindole-3a-carboxylic acid
methyl (3aRS,4SR,9SR,9aRS)-9-(3,4-dimethylphenyl)-4,9-ethano-2-[(2-methoxyphenyl)acetyl]-2,3,3a,4,9,9a-hexahydro-1H-benzo[f]isoindole-3a-carboxylate
(3aRS,4SR,9SR,9aRS)-9-(3,4-dimethylphenyl)-4,9-ethano-2-[(2-methoxyphenyl)acetyl]-2,3,3a,4,9,9a-hexahydro-1H-benzo[f]isoindole-3a-carboxylic acid
(3aRS,4SR,9SR,9aRS)-3a-N-benzylcarbamoyl-4,9-ethano-2-[2-(2-methoxyphenyl)propenoyl]-9-(4-methylphenyl)-2,3,3a,4,9,9a-hexahydro-1H-benzo[f]isoindole
(3aRS,4SR,9SR,9aRS)-3a-carbamoyl-4,9-ethano-2-[2-(2-methoxyphenyl)propenoyl]-9-(4-methylphenyl)-2,3,3a,4,9,9a-hexahydro-1H-benzo[f]isoindole
benzyl (3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-hydroxamate
(3aRS,4SR,9SR,9aRS)-4,9-ethano-2-[2-(2-methoxyphenyl)-propenoyl]-9-(4-methylphenyl)-2,3,3a,4,9,9a-hexahydro-1H-benzo[f]isoindole-3a-hydroxamic acid
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N-(3-pyridylmethyl)-carboxamide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N-(4-pyridylmethyl)-carboxamide
methyl (3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-hydroxamate
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N',N'-dimethylcarbohydrazide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-phenylcarbohydrazide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N',N'-pentamethylenecarbohydrazide
(3aRS,4SR,9SR,9aRS)-4,9-ethano-2-[2-(methoxyphenyl)propenoyl]-9-(4-methylphenyl)-3a-phenylsulphonylaminocarbonyl-2,3,3a,4,9,9a-hexahydro-1H-benzo[f]isoindole
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N-(N-oxido-3-pyridyl)-methylcarboxamide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-(4-methoxyphenyl)-carbohydrazide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-methyl-N'-phenylcarbohydrazide
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-(2-methylphenyl)carbohydrazide
methyl (3aRS,4SR,9SR,9aRS)-9-(2,3-dihydro-1,4-benzodioxin-6-yl)-4,9-ethano-2-[2-(2-methoxyphenyl)-propenoyl]-2,3,3a,4,9,9a-hexahydro-1H-benzo[f]-isoindole-3a-carboxylate
(3aRS,4SR,9SR,9aRS)-9-(2,3-dihydro-1,4-benzodioxin-6-yl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(2,3-dihydro-1,4-benzodioxin-6-yl)-4,9-ethano-2-[2-(2-methoxyphenyl)propenoyl]-2,3,3a,4,9,9a-hexahydro-1H-benzo[f]isoindole-3a-N-(3-pyridylmethyl)carboxamide
(3aRS,4SR,9SR,9aRS)-9-(2,3-dihydro-1,4-benzodioxin-6-yl)-4,9-ethano-2-[2-(2-methoxyphenyl)propenoyl]-2,3,3a,4,9,9a-hexahydro-1H-benzo[f]isoindole-3a-N-(2-thienylmethyl)carboxamide
(3aRS,4SR,9SR,9aRS)-4,9-ethano-2-[(2-methoxyphenyl)-acetyl]-9-(3,4,5-trimethylphenyl)-2,3,3a,4,9,9a-hexahydro-1H-benzo[f]isoindole-3a-carboxylic acid
(3aRS,4SR,9SR,9aRS)-4,9-ethano-2-[2-(2-methoxyphenyl)-propenoyl]-9-(2-methylphenyl)-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(4-trifluoromethylphenyl)-2,3,3a,4,9,9a-hexahydro-1H-benzo[f]isoindole-3a-carboxylate
(3aRS,4SR,9SR,9aRS)-4,9-ethano-2-[2-(2-methoxyphenyl)-propenoyl]-9-(4-trifluoromethylphenyl)-2,3,3a,4,9,9a-hexahydro-1H-benzo[f]isoindole-3a-carboxylic acid
(3aRS,4SR,9SR,9aRS)-4,9-ethano-2-[2-(2-methoxyphenyl)-propenoyl]-9-(4-trifluoromethylphenyl)-2,3,3a,4,9,9a-hexahydro-1H-benzo[f]isoindole-3a-N-(4-pyridylmethyl)carboxamide
(3aRS,4SR,9SR,9aRS)-4,9-ethano-2-[2-(2-methoxyphenyl)-propenoyl]-9-(4-trifluoromethylphenyl)-2,3,3a,4,9,9a-hexahydro-1H-benzo[f]isoindole-3a-N'-benzoylcarbohydrazide
(3aRS,4SR,9SR,9aRS)-4,9-ethano-2-[2-(2-methoxyphenyl)-propenoyl]-9-(4-trifluoromethylphenyl)-2,3,3a,4,9,9a-hexahydro-1H-benzo[f]isoindole-3a-N'-phenylcarbohydrazide
methyl (3aRS,4SR,9SR,9aRS)-4,9-ethano-2-[2-(2-methoxyphenyl)propenoyl]-9-(2-naphthyl)-2,3,3a,4,9,9a-hexahydro-1H-benzo[f]isoindole-3a-carboxylate
(3aRS,4SR,9SR,9aRS)-4,9-ethano-2-[2-(2-methoxyphenyl)-propenoyl]-9-(2-naphthyl)-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(5-methyl-2-thienyl)-2,3,3a,4,9,9a-hexahydro-1H-benzo[f]isoindole-3a-carboxylate
(3aRS,4SR,9SR,9aRS)-4,9-ethano-2-[2-(2-methoxyphenyl)-propenoyl]-9-(5-methyl-2-thienyl)-2,3,3a,4,9,9a-hexahydro-1H-benzo[f]isoindole-3a-carboxylic acid
methyl (3aRS,4SR,9SR,9aRS)-9-(4-bromophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(4-bromophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(3,4-dichlorophenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(4-chlorophenyl)-2,3,3a,4,9,9a-hexahydro-1H-benzo[f]isoindole-3a-carboxylate
(3aRS,4SR,9SR,9aRS)-9-(4-chlorophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-4,9-ethano-2-[2-(2-methoxyphenyl)-propenoyl]-9-(4-methoxy-3-methylphenyl)-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(3-indolyl)-2,3,3a,4,9,9a-hexahydro-1H-benzo[f]isoindole-3a-carboxylate
(3aRS,4SR,9SR,9aRS)-4,9-ethano-9-(4-isopropylphenyl)-2-[(2-methoxyphenyl)acetyl]-2,3,3a,4,9,9a-hexahydro-1H-benzo[f]isoindole-3a-carboxylic acid
(3aRS,4SR,9SR,9aRS)-4,9-ethano-9-(4-isopropylphenyl)-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(3-thienyl)-2,3,3a,4,9,9a-hexahydro-1H-benzo[f]isoindole-3a-carboxylate
methyl (3aRS,4SR,9SR,9aRS)-4,9-ethano-9-(4-ethylphenyl)-2-[2-(2-methoxyphenyl)propenoyl]-2,3,3a,4,9,9a-hexahydro-1H-benzo[f]isoindole-3a-carboxylate
(3aRS,4SR,9SR,9aRS)-4,9-ethano-9-(4-ethylphenyl)-2-[2-(2-methoxyphenyl)propenoyl)-2,3,3a,4,9,9a-hexahydro-1H-benzo[f]isoindole-3a-carboxylic acid
methyl (3aRS,4SR,9SR,9aRS)-9-(2,3-dihydrobenzofuran-5-yl)-4,9-ethano-2-[2-(2-methoxyphenyl)propenoyl]-2,3,3a,4,9,9a-hexahydro-1H-benzo[f]isoindole-3a-carboxylate
methyl (3aRS,4SR,9SR,9aRS)-9-(2,3-dihydrobenzofuran-5-yl)-4,9-ethano-2-[2-(2-methoxyphenyl)propenoyl]-2,3,3a,4,9,9a-hexahydro-1H-benzo[f]isoindole-3a-carboxylate acid
(3aRS,4SR,9SR,9aRS)-9-(2,3-dihydrobenzofuran-5-yl)-4,9-ethano-2-[2-(2-methoxyphenyl)propenoyl)]-2,3,3a,4,9,9a-hexahydro-1H-benzo[f]isoindole-3a-N-(3-pyridylmethyl)-carboxamide
methyl (3aRS,4SR,9SR,9aRS)-4,9-ethano-9-(4-fluorophenyl)-2-[2-(2-methoxyphenyl)propenoyl]-2,3,3a,4,9,9a-hexahydro-1H-benzo[f]isoindole-3a-carboxylate
(3aRS,4SR,9SR,9aRS)-4,9-ethano-9-(4-fluorophenyl)-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-9-(4-chloro-3-fluorophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(4-chloro-3-fluorophenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-2-[2-(2-methoxyphenyl)propenoyl]-9-(3-methylphenyl)-2,3,3a,4,9,9a-hexahydro-1H-benzo[f]isoindole-3a-carboxylate
(3aRS,4SR,9SR,9aRS)-4,9-ethano-2-[2-(2-methoxyphenyl)propenoyl]-9-(3-methylphenyl)-2,3,3a,4,9,9a-hexahydro-1H-benzo[f]isoindole-3a-carboxylic acid
methyl (3aRS,4SR,9SR,9aRS)-9-(1,3-benzodioxol-5-yl)-4,9-ethano-2-[2-(2-methoxyphenyl)propenoyl]-2,3,3a,4,9,9a-hexahydro-1H-benzo[f]isoindole-3a-carboxylate
methyl (3aRS,4SR,9SR,9aRS)-9-(3,4-dimethylphenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(3,4-dimethylphenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-9-(4-methoxyphenyl)-2-[2-(2-methoxyphenyl)propenoyl]-2,3,3a,4,9,9a-hexahydro-2H-benzo[f]isoindole-3a-carboxylate
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N-(3-pyridylmethyl)carboxamide
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N'-phenylcarbohydrazide
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-hydroxamic acid
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N'-(3-pyridyl)carbohydrazide
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N-(3-thienylmethyl)carboxamide
(RS)- and (SR)-2-{(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-carbonylamino}phenylacetic acids
(3aRS,4SR,9SR,9aRS)-4,9-ethano-2-[2-(2-methoxyphenyl)propenoyl]-9-(4-trifluoromethoxyphenyl)-2,3,3a,4,9,9a-hexahydro-1H-benzo[f]isoindole-3a-carboxylic acid
methyl (3aRS,4SR,9SR,9aRS)-9-(3-bromophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(3-bromophenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-9-(3-fluorophenyl)-2-[2-(2-methoxyphenyl)propenoyl]-2,3,3a,4,9,9a-hexahydro-1H-benzo[f]isoindole-3a-carboxylate
(3aRS,4SR,9SR,9aRS)-4,9-ethano-9-(3-fluorophenyl)-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-4,9-ethano-9-(3-fluorophenyl)-2-[2-(2-methoxyphenyl)propenoyl]-2,3,3a,4,9,9a-hexahydro-1H-benzo[f]isoindole-3a-N-(3-pyridylmethyl)carboxamide
methyl (3aRS,4SR,9SR,9aRS)-9-(3-chlorophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(3-chlorophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(3-chlorophenyl)-4,9-ethano-2-[2-(2-methoxyphenyl)propenoyl]-2,3,3a,4,9,9a-hexahydro-1H-benzo[f]isoindole 3a-N-(3-pyridylmethyl)carboxamide
methyl (3aRS,4SR,9SR,9aRS)-9-(3-N,N-dimethylaminophenyl)-4,9-ethano-2-[2-(2-methoxyphenyl)-propenoyl]-2,3,3a,4,9,9a-hexahydro-1H-benzo[f]-isoindole-3a-carboxylate
(3aRS,4SR,9SR,9aRS)-9-(3-N,N-dimethylaminophenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-9-(3-aminophenyl)-4,9-ethano-2-[2-(2-methoxyphenyl)propenoyl]-2,3,3a,4,9,9a-hexahydro-1H-benzo[f]isoindole-3a-carboxylate hydrochloride
methyl (3aRS,4SR,9SR,9aRS)-9-(4-N,N-dimethylaminophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-9-(4-cyanophenyl)-4,9-ethano-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-9-(3-cyanophenyl)-4,9-ethano-2-[2-(2-methoxyphenyl)propenoyl]-2,3,3a,4,9,9a-hexahydro-1H-benzo[f]isoindole-3a-carboxylate
(3aRS,4SR,9SR,9aRS)-4,9-ethano-9-(3-hydroxy-4-methoxyphenyl)-2-[2-(2-methoxyphenyl)propenoyl]-2,3,3a,4,9,9a-hexahydro-1H-benzo[f]isoindole-3a-carboxylic acid
methyl (3aRS,4SR,9SR,9aRS)-4,9-ethano-5-methoxy-2-[2-(2-methoxyphenyl)propenoyl]-9-(4-methylphenyl)-2,3,3a,4,9,9a-hexahydro-1H-benzo[f]isoindole-3a-carboxylate
(3aRS,4SR,9SR,9aRS)-4,9-ethano-5-methoxy-2-[2-(2-methoxyphenyl)propenoyl]-9-(4-methylphenyl)-2,3,3a,4,9,9a-hexahydro-1H-benzo[f]isoindole-3a-carboxylic acid
(3aRS,4SR,9SR,9aRS)-4,9-ethano-2-[2-(2-methoxyphenyl)propenoyl]-4-methyl-9-(4-methoxyphenyl)-2,3,3a,4,9,9a-hexahydro-1H-benzo[f]isoindole-3a-carboxylic acid
(3aRS,4SR,9SR,9aRS)-9-(benzothien-2-yl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(2,4-dichlorophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,8SR,8aRS)-4,8-ethano-2-[2-(2-methoxyphenyl)propenoyl]-8-phenyl-2,3,3a,4,8,8a-hexahydro-1H-thieno[2,3-f]isoindole-3a-carboxylic acid
(3aRS,4SR,8SR,8aRS)-4,8-ethano-2-[2-(2-methoxyphenyl)propenoyl]-8-(4-methylphenyl)-2,3,3a,4,8,8a-hexahydro-1H-thieno[2,3-f]isoindole-3a-carboxylic acid
(3aRS,4SR,8SR,8aRS)-4,8-ethano-2-[2-(2-methoxyphenyl)propenoyl]-8-(4-trifluoromethylphenyl)-2,3,3a,4,8,8a-hexahydro-1H-thieno[2,3-f]isoindole-3a-carboxylic acid
(3aRS,4SR,8SR,8aRS)-N-benzyl-4,8-ethano-2-[2-(2-methoxyphenyl)propenoyl]-8-(4-trifluoromethylphenyl)-2,3,3a,4,8,8a-hexahydro-1H-thieno[2,3-f]isoindole-3a-carboxamide
(3aRS,4SR,10SR,10aRS)-4,9-ethano-2-[2-(2-methoxyphenyl)propenoyl]-10-(4-methylphenyl)-2,3,3a,4,10,10a-hexahydro-1H-indolo[2,3-f]isoindole-3a-carboxylic acid
(3aRS,4SR,8SR,8aRS)-N-(3-pyridyl)methyl-4,8-ethano-2-[2-(2-methoxyphenyl)propenoyl]-8-(4-methylphenyl)-2,3,3a,4,8,8a-hexahydro-1H-thieno[2,3-f]isoindole-3a-carboxamide
methyl (3aRS,4SR,8SR,8aRS)-4,8-ethano-8-(4-methoxyphenyl)-2-[2-(2-methoxyphenyl)propenoyl]-2,3,3a,4,8,8a-hexahydro-1H-thieno[2,3-f]isoindole-3a-carboxylate
(3aRS,4SR,8SR,8aRS)-4,8-ethano-8-(4-methoxyphenyl)-2-[2-(2-methoxyphenyl)propenoyl]-2,3,3a,4,8,8a-hexahydro-1H-thieno[2,3-f]isoindole-3a-carboxylic acid
(3aRS,4SR,8SR,8aRS)-N-(3-pyridyl)methyl-8-(benzo-1,4-dioxan-6-yl)-4,8-ethano-2-[2-(2-methoxyphenyl)propenoyl]-2,3,3a,4,8,8a-hexahydro-1H-thieno[2,3-f]isoindole-3a-carboxamide,
in the form of their optical isomers, as well as their salts.

4. Process for the preparation of the compounds of general formula (I) according to any one of the preceding claims, **characterized in that** the compounds of general formula (I) are individually chosen from:
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-carboxylic acid
(3aRS,4SR,9SR,9aRS-9-(benzothien-2-yl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(2,4-dichlorophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(3,4-dichlorophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-9-(4-bromophenyl)-4,9-ethano-2-[2-(2-methoxyphenyl)propenoyl]-2,3,3a,4,9,9a-hexahydro-1H-benzo[f]isoindole-3a-carboxylic acid
(3aRS,4SR,9SR,9aRS)-4,9-ethano-2-[2-(2-methoxyphenyl)propenoyl]-9-(4-chlorophenyl)-2,3,3a,4,9,9a-hexahydro-1H-benzo[f]isoindole-3a-carboxylic acid
methyl (3aRS,4SR,9SR,9aRS)-9-(2,3-dihydrobenzofuran-5-yl)-4,9-ethano-2-[2-(2-methoxyphenyl)propenoyl]-2,3,3a,4,9,9a-hexahydro-1H-benzo[f]isoindole-3a-carboxylate acid
(3aRS,4SR,8SR,8aRS)-4,8-ethano-2-[2-(2-methoxyphenyl)propenoyl]-8-(4-methylphenyl)-2,3,3a,4,8,8a-hexahydro-1H-thieno[2,3-f]isoindole-3a-carboxylic acid,
in the form of their optical isomers, as well as their salts.

5. Process for the preparation of the compounds of general formula (I) according to Claim 1, **characterized in that** the products of general formula (I) are obtained in the form of their dextrorotatory enantiomer.

6. Process for the preparation of the compounds of general formula (X'), in the form of optical isomers: 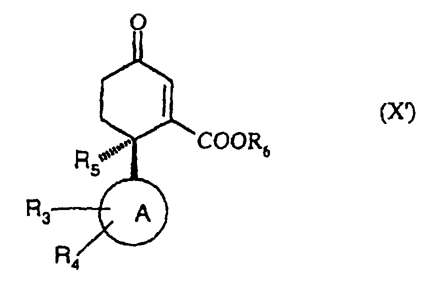 in which formula
A, R₃, R₄, R₅ and R₆ are as defined in Claim 1, **characterized in that** the procedure is carried out starting from the compounds of general formula (X), in the form of a racemic mixture: 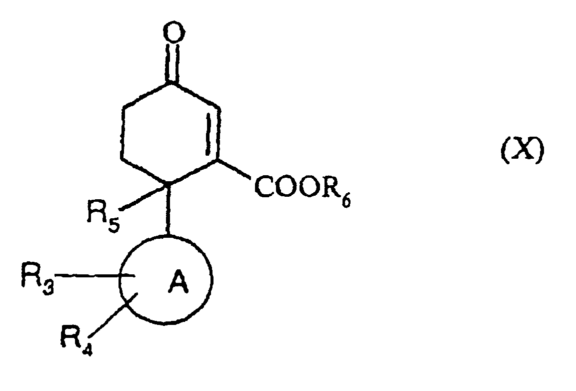 in a mixture of a hydrocarbon-comprising solvent and of an aqueous solution, at a pH of between 6 and 8, by the action of an enzyme chosen from lipases, esterases or proteases.

7. Process according to Claim 6, **characterized in that** the enzyme is chosen from lipases or esterases.

8. Process according to Claim 7, **characterized in that** the lipases and esterases are chosen from: EC 3.1.1.3 *Candida antartica fraction B* (Chirazyme® L2, Roche Diagnostic, formerly Boehringer Mannheim), EC #3.1.1.3 *Pseudomonas cepacia* (Lipase PS-C, Amano Enzymes), EC 3.1.1.1 (ESL 01, Diversa, formerly Recombinant Biocatalyst), EC 3.1.1.3 *Candida antartica fraction B* (SP 525, Novozyme, formerly Novo Nordisk), EC 3.1.1.3 *Pseudomonas sp.* (Chirazyme® L6, Roche Diagnostic, formerly Boehringer Mannheim), EC 3.1.1.3 *Rhizomucor miehei* (Esterase 30000, DSM, formerly Gist Brocades).

9. Process according to Claim 8, **characterized in that** the enzyme is EC 3.1.1.3 *Candida antartica fraction B* (Chirazyme® L2, Roche Diagnostic, formerly Boehringer Mannheim).

10. Process according to Claim 7, **characterized in that** the proteases and lipases are chosen from: EC 3.4.21.80 *Streptomyces griseus* (Pronase®, Roche Diagnostic, formerly Boehringer Mannheim), EC 3.4.21.62 *Bacillus licheniformis* (Subtilisin, Novozyme, formerly Novo Nordisk), EC 3.4.21.62 *Bacillus licheniformis* (Alcalase 2,4 L, Novozyme, formerly Novo Nordisk), EC 3.4.21.14 *Bacillus subtilis* (Protease Nagarse, Sigma Aldrich), EC 3.4.21.62 *Bacillus licheniformis* (EH 16, Altus Biologics), EC 3.4.21.62 *Bacillus sp.* (EH 15, Altus Biologics), EC 3.1.1.3 *Candida antartica fraction A* (EH 11, Altus Biologics), EC 3.1.1.3 *Mucor javanicus* (Lipase M, Amano Enzymes).

11. Process according to Claim 6, **characterized in that** the organic solvent is cyclohexane.

12. Process according to Claim 6, **characterized in that** the aqueous solution is a dipotassium hydrogenphosphate solution.
